# EUROPEAN PATENT SPECIFICATION

(11) **EP 1 660 465 B1**
(45) Date of publication and mention of the grant of the patent: **17.12.2014**
(21) Application number: 04779405.2
(22) Date of filing: 28.07.2004
(51) Int. Cl.: C07D 263/20, C07D 413/10, C07D 413/12, C07D 413/14, C07D 417/10, C07D 417/12, C07D 417/14

(54) **PROCESS FOR THE SYNTHESIS OF BIARYL OXAZOLIDINONES**
VERFAHREN ZUR SYNTHESE VON BIARYLOXAZOLIDINONEN
PROCEDE DE SYNTHESE D'OXAZOLIDINONES DE BIARYLE

(30) Priority: 29.07.2003 US 490855 P; 15.12.2003 US 529731 P; 17.12.2003 US 530371 P; 19.12.2003 US 531584 P; 02.06.2004 US 859476; 02.06.2004 US 576163 P
(43) Date of publication of application: 31.05.2006
(73) Proprietor: Melinta Therapeutics, Inc., New Haven, CT 06511 (US)
(72) Inventor: WU, Yusheng, Lebanon, NJ 08833 (US); CHEN, Shili, Cheshire, CT 06410 (US); CHEN, Yi, Cheshire, CT 06410 (US); HANSELMANN, Roger, Branford, CT 06405 (US); LOU, Rongliang, Cheshire CT 06410 (US); ZHOU, Jiacheng, Hockessin, DE 19707 (US)
(74) Representative: Mintz Levin Cohn Ferris Glovsky and Popeo LLP
(86) International application number: PCT/US2004/024339
(87) International publication number: WO 2005/012271

(56) References cited:
- EP-A- 0 352 781
- EP-A- 0 694 543
- WO-A-03/022824
- WO-A-2004/029066
- WO-A-2004/048392
- WO-A-2004/056817
- WO-A-2004/078753
- WO-A-2005/012270
- WO-A-2005/019211
- GLEAVE D M ET AL.: "Synthesis And Antibacterial Activity of [6,5,5] and [6,6,5] Tricyclic Fused Oxazolidinones" BIOORGANIC & MEDICINAL CHEMISTRY LETTERS, vol. 8, no. 10, 19 May 1998 (1998-05-19), pages 1231-1236, XP004137053
- MOLANDER G A ET AL: "PALLADIUM-CATALYZED SUZUKI-MIYAURA CROSS-COUPLING REACTIONS OF POTASSIUM ARYL- AND HETEROARYLTRIFLUOROBORATES" JOURNAL OF ORGANIC CHEMISTRY, AMERICAN CHEMICAL SOCIETY. EASTON, US, vol. 68, no. 11, 30 May 2003 (2003-05-30), pages 4302-4314, XP001160394 ISSN: 0022-3263

## Description

### FIELD OF THE INVENTION

The present invention relates generally to a process for synthesizing anti-infective, anti-proliferative, anti-inflammatory, and prokinetic agents. More particularly, the invention relates to a process for synthesizing biaryl oxazolidinone compounds that are useful as therapeutic agents.

### BACKGROUND

Since the discovery of penicillin in the 1920s and streptomycin in the 1940s, many new compounds have been discovered or specifically designed for use as antibiotic agents. It was once believed that infectious diseases could be completely controlled or eradicated with the use of such therapeutic agents. However, such beliefs have been shaken by the fact that strains of cells or microorganisms resistant to currently effective therapeutic agents continue to evolve. In fact, virtually every antibiotic agent developed for clinical use has ultimately encountered problems with the emergence of resistant bacteria. For example, resistant strains of Gram-positive bacteria such as methicillin-resistant staphylocci, penicillin-resistant streptococci, and vancomycin-resistant enterococci have developed, which can cause serious and even fatal results for patients infected with such resistant bacteria. Bacteria that are resistant to macrolide antibiotics, i.e., antibiotics based on a 14- to 16-membered lactone ring, have developed. Also, resistant strains of Gram-negative bacteria such as *H. influenzae* and *M. catarrhalis* have been identified. *See, e.g.,* F.D. Lowry, "Antimicrobial Resistance: The Example of Staphylococcus aureus," J. Clin. Invest., 2003, 111(9), 1265-1273; and Gold, H.S. and Moellering, R.C., Jr., "Antimicrobial-Drug Resistance," N. Engl. J. Med., 1996, 335, 1445-53.

The problem of resistance is not limited to the area of anti-infective agents, because resistance has also been encountered with anti-proliferative agents used in cancer chemotherapy. Therefore, there exists a need for new anti-infective and anti-proliferative agents that are both effective against resistant bacteria and resistant strains of cancer cells.

In the antibiotic area, despite the problem of increasing antibiotic resistance, no new major classes of antibiotics have been developed for clinical use since the approval in the United States in 2000 of the oxazolidinone ring-containing antibiotic, N-[[(5S)-3-[3-fluoro-4-(4-morpholinyl)phenyl]-2-oxo-5-oxazolidinyl]methyl acetamide, which is known as linezolid and is sold under the tradename Zyvox® (*see* compound A). *See,* R.C. Moellering, Jr., "Linezolid: The First Oxazolidinone Antimicrobial," Annals of Internal Medicine, 2003, 138(2), 135-142.

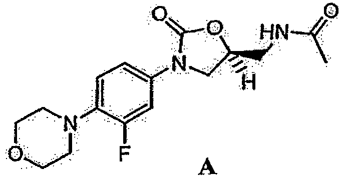

Linezolid was approved for use as an anti-bacterial agent active against Gram-positive organisms. Unfortunately, linezolid-resistant strains of organisms are already being reported. *See,* Tsiodras et al., Lancet, 2001, 358, 207; Gonzales et al., Lancet, 2001, 357, 1179; Zurenko et al., Proceedings Of The 39th Annual Interscience Conference On Antibacterial Agents And Chemotherapy (ICAAC); San Francisco, CA, USA, (September 26-29, 1999). Because linezolid is both a clinically effective and commercially significant anti-microbial agent, investigators have been working to develop other effective linezolid derivatives.

Notwithstanding the foregoing, there is an ongoing need for new anti-infective and anti-proliferative agents. Furthermore, because many anti-infective and anti-proliferative agents have utility as anti-inflammatory agents and prokinetic agents, there is also an ongoing need for new compounds useful as anti-inflammatory and prokinetic agents, as well as methods for making such compounds.

### SUMMARY OF THE INVENTION

The present invention provides processes for preparing biaryl oxazolidinone compounds as defined in the claims.

The processes generally provide the desired biaryl oxazolidinone compound at or near the end of the overall process, and further synthetic manipulation of the biaryl system or its substituents is generally not necessary.

The foregoing and other aspects and embodiments of the invention can be more fully understood by reference to the following detailed description and claims.

### DETAILED DESCRIPTION OF THE INVENTION

The present invention relates to processes for preparing biaryl oxazolidinone compounds useful as anti-proliferative agents and/or anti-infective agents. The compounds may be used without limitation, for example, as anti-cancer, anti-microbial, anti-bacterial, anti-fungal, antiparasitic and/or anti-viral agents. Further, compounds produced by the processes of the invention can be used without limitation as anti-inflammatory agents, for example, for use in treating chronic inflammatory airway diseases, and/or as prokinetic agents, for example, for use in treating gastrointestinal motility disorders such as gastroesophageal reflux disease, gastroparesis (diabetic and post surgical), irritable bowel syndrome, and constipation.

Compounds synthesized according to the methods of the invention may be used to treat a disorder in a mammal by administering to the mammal an effective amount of one or more compounds of the invention thereby to ameliorate a symptom of a particular disorder. Such a disorder can be selected from the group consisting of a skin infection, nosocomial pneumonia, post-viral pneumonia, an abdominal infection, a urinary tract infection, bacteremia, septicemia, endocarditis, an atrio-ventricular shunt infection, a vascular access infection, meningitis, surgical prophylaxis, a peritoneal infection, a bone infection, a joint infection, a methicillin-resistant *Staphylococcus aureus* infection, a vancomycin-resistant *Enterococci* infection, a linezolid-resistant organism infection, and tuberculosis.

### 1. Definitions

The term "substituted," as used herein, means that any one or more hydrogens on the designated atom is replaced with a selection from the indicated group, provided that the designated atom's normal valency is not exceeded, and that the substitution results in a stable compound. When a substituent is keto (i.e., =O), then 2 hydrogens on the atom are replaced. Keto substituents are not present on aromatic moieties. Ring double bonds, as used herein, are double bonds that are formed between two adjacent ring atoms (e.g., C=C, C=N, or N=N).

The present invention is intended to include all isotopes of atoms occurring in the present compounds. Isotopes include those atoms having the same atomic number but different mass numbers. By way of general example and without limitation, isotopes of hydrogen include tritium and deuterium, and isotopes of carbon include C-13 and C-14.

The compounds described herein may have asymmetric centers. Compounds of the present invention containing an asymmetrically substituted atom may be isolated in optically active or racemic forms. It is well known in the art how to prepare optically active forms, such as by resolution of racemic forms or by synthesis from optically active starting materials. Many geometric isomers of olefins, C=N double bonds, and the like can also be present in the compounds described herein, and all such stable isomers are contemplated in the present invention. Cis and trans geometric isomers of the compounds of the present invention are described and may be isolated as a mixture of isomers or as separated isomeric forms. All chiral, diastereomeric, racemic, and geometric isomeric forms of a structure are intended, unless the specific stereochemistry or isomeric form is specifically indicated. All tautomers of shown or described compounds are also considered to be part of the present invention.

When any variable (e.g., R¹) occurs more than one time in any constituent or formula for a compound, its definition at each occurrence is independent of its definition at every other occurrence. Thus, for example, if a group is shown to be substituted with 0-2 R¹ moieties, then the group may optionally be substituted with up to two R¹ moieties and R¹ at each occurrence is selected independently from the definition of R¹. Also, combinations of substituents and/or variables are permissible, but only if such combinations result in stable compounds.

When a bond to a substituent is shown to cross a bond connecting two atoms in a ring, then such substituent may be bonded to any atom in the ring. When a substituent is listed without indicating the atom via which such substituent is bonded to the rest of the compound of a given formula, then such substituent may be bonded via any atom in such substituent. Combinations of substituents and/or variables are permissible, but only if such combinations result in stable compounds.

Compounds of the present invention that contain nitrogens can be converted to N-oxides by treatment with an oxidizing agent (e.g., 3-chloroperoxybenzoic acid (*m*-CPBA) and/or hydrogen peroxides) to afford other compounds of the present invention. Thus, all shown and claimed nitrogen-containing compounds are considered, when allowed by valency and structure, to include both the compound as shown and its N-oxide derivative (which can be designated as N→O or N⁺-O⁻). Furthermore, in other instances, the nitrogens in the compounds of the present invention can be converted to N-hydroxy or N-alkoxy compounds. For example, N-hydroxy compounds can be prepared by oxidation of the parent amine by an oxidizing agent such as *m*-CPBA. All shown and claimed nitrogen-containing compounds are also considered, when allowed by valency and structure, to cover both the compound as shown and its N-hydroxy (i.e., N-OH) and N-alkoxy (i.e., N-OR, wherein R is substituted or unsubstituted C₁₋₆ alkyl, C₁₋₆ alkenyl, C₁₋₆ alkynyl, C₃₋₁₄ carbocycle, or 3-14-membered heterocycle) derivatives.

When an atom or chemical moiety is followed by a subscripted numeric range (e.g., C₁₋₆), the invention is meant to encompass each number within the range as well as all intermediate ranges. For example, "C₁₋₆ alkyl," is meant to include alkyl groups with 1, 2, 3, 4, 5, 6, 1-6, 1-5, 1-4, 1-3, 1-2, 2-6, 2-5, 2-4, 2-3, 3-6, 3-5, 3-4, 4-6, 4-5, and 5-6 carbons.

As used herein, "alkyl" is intended to include both branched and straight-chain saturated aliphatic hydrocarbon groups having the specified number of carbon atoms. For example, C₁₋₆ alkyl is intended to include C₁, C₂, C₃, C₄, C₅, and C₆ alkyl groups. Examples of alkyl include, but are not limited to, methyl, ethyl, n-propyl, i-propyl, n-butyl, s-butyl, t-butyl, n-pentyl, s-pentyl, and n-hexyl.

As used herein, "alkenyl" is intended to include hydrocarbon chains of either straight or branched configuration having one or more carbon-carbon double bonds occurring at any stable point along the chain. For example, C₂₋₆ alkenyl is intended to include C₂, C₃, C₄, C₅, and C₆ alkenyl groups. Examples of alkenyl include, but are not limited to, ethenyl and propenyl.

As used herein, "alkynyl" is intended to include hydrocarbon chains of either straight or branched configuration having one or more carbon-carbon triple bonds occurring at any stable point along the chain. For example, C₂₋₆ alkynyl is intended to include C₂, C₃, C₄, C₅, and C₆ alkynyl groups. Examples of alkynyl include, but are not limited to, ethynyl and propynyl.

As used herein, "halo" or "halogen" refers to fluoro, chloro, bromo, and iodo. "Counterion" is used to represent a small, negatively charged species such as chloride, bromide, hydroxide, acetate, and sulfate.

As used herein, "carbocycle" or "carbocyclic ring" is intended to mean any stable monocyclic, bicyclic, or tricyclic ring having the specified number of carbons, any of which may be saturated, unsaturated, or aromatic. For example a C₃₋₁₄ carbocycle is intended to mean a mono-, bi-, or tricyclic ring having 3, 4, 5, 6, 7, 8, 9, 10, 11, 12, 13, or 14 carbon atoms. Examples of carbocycles include, but are not limited to, cyclopropyl, cyclobutyl, cyclobutenyl, cyclopentyl, cyclopentenyl, cyclohexyl, cycloheptenyl, cycloheptyl, cycloheptenyl, adamantyl, cyclooctyl, cyclooctenyl, cyclooctadienyl, fluorenyl, phenyl, naphthyl, indanyl, adamantyl, and tetrahydronaphthyl. Bridged rings are also included in the definition of carbocycle, including, for example, [3.3.0]bicyclooctane, [4.3.0]bicyclononane, [4.4.0]bicyclodecane, and [2.2.2]bicyclooctane. A bridged ring occurs when one or more carbon atoms link two non-adjacent carbon atoms. Preferred bridges are one or two carbon atoms. It is noted that a bridge always converts a monocyclic ring into a tricyclic ring. When a ring is bridged, the substituents recited for the ring may also be present on the bridge. Fused (e.g., naphthyl and tetrahydronaphthyl) and spiro rings are also included.

As used herein, the term "heterocycle" or "heterocyclic" is intended to mean any stable monocyclic, bicyclic, or tricyclic ring which is saturated, unsaturated, or aromatic and comprises carbon atoms and one or more ring heteroatoms, e.g., 1 or 1-2 or 1-3 or 1-4 or 1-5 or 1-6 heteroatoms, independently selected from the group consisting of nitrogen, oxygen, and sulfur. A bicyclic or tricyclic heterocycle may have one or more heteroatoms located in one ring, or the heteroatoms may be located in more than one ring. The nitrogen and sulfur heteroatoms may optionally be oxidized (i.e., N→O and S(O)ₚ, where p = 1 or 2). When a nitrogen atom is included in the ring it is either N or NH, depending on whether or not it is attached to a double bond in the ring (i.e., a hydrogen is present if needed to maintain the tri-valency of the nitrogen atom). The nitrogen atom may be substituted or unsubstituted (i.e., N or NR wherein R is H or another substituent, as defined). The heterocyclic ring may be attached to its pendant group at any heteroatom or carbon atom that results in a stable structure. The heterocyclic rings described herein may be substituted on carbon or on a nitrogen atom if the resulting compound is stable. A nitrogen in the heterocycle may optionally be quaternized. It is preferred that when the total number of S and O atoms in the heterocycle exceeds 1, then these heteroatoms are not adjacent to one another. Bridged rings are also included in the definition of heterocycle. A bridged ring occurs when one or more atoms (i.e., C, O, N, or S) link two non-adjacent carbon or nitrogen atoms. Preferred bridges include, but are not limited to, one carbon atom, two carbon atoms, one nitrogen atom, two nitrogen atoms, and a carbon-nitrogen group. It is noted that a bridge always converts a monocyclic ring into a tricyclic ring. When a ring is bridged, the substituents recited for the ring may also be present on the bridge. Spiro and fused rings are also included.

As used herein, the term "aromatic heterocycle" or "heteroaryl" is intended to mean a stable 5, 6, or 7-membered monocyclic or bicyclic aromatic heterocyclic ring or 7, 8, 9, 10, 11, or 12-membered bicyclic aromatic heterocyclic ring which consists of carbon atoms and one or more heteroatoms, e.g., 1 or 1-2 or 1-3 or 1-4 or 1-5 or 1-6 heteroatoms, independently selected from the group consisting of nitrogen, oxygen, and sulfur. In the case of bicyclic heterocyclic aromatic rings, only one of the two rings needs to be aromatic (e.g., 2,3-dihydroindole), though both may be (e.g., quinoline). The second ring can also be fused or bridged as defined above for heterocycles. The nitrogen atom may be substituted or unsubstituted (i.e., N or NR wherein R is H or another substituent, as defined). The nitrogen and sulfur heteroatoms may optionally be oxidized (i.e., N→O and S(O)ₚ, where p = 1 or 2). It is to be noted that total number of S and O atoms in the aromatic heterocycle is not more than 1.

Examples of heterocycles include, but are not limited to, acridinyl, azocinyl, benzimidazolyl, benzofuranyl, benzothiofuranyl, benzothiophenyl, benzoxazolyl, benzoxazolinyl, benzthiazolyl, benztriazolyl, benztetrazolyl, benzisoxazolyl, benzisothiazolyl, benzimidazolinyl, carbazolyl, 4a*H*-carbazolyl, carbolinyl, chromanyl, chromenyl, cinnolinyl, decahydroquinolinyl, 2*H*,6*H*-1,5,2-dithiazinyl, dihydrofuro[2,3-*b*]tetrahydrofuran, furanyl, furazanyl, imidazolidinyl, imidazolinyl, imidazolyl, 1*H*-indazolyl, indolenyl, indolinyl, indolizinyl, indolyl, 3H-indolyl, isatinoyl, isobenzofuranyl, isochromanyl, isoindazolyl, isoindolinyl, isoindolyl, isoquinolinyl, isothiazolyl, isoxazolyl, methylenedioxyphenyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxindolyl, pyrimidinyl, phenanthridinyl, phenanthrolinyl, phenazinyl, phenothiazinyl, phenoxathinyl, phenoxazinyl, phthalazinyl, piperazinyl, piperidinyl, piperidonyl, 4-piperidonyl, piperonyl, pteridinyl, purinyl, pyranyl, pyrazinyl, pyrazolidinyl, pyrazolinyl, pyrazolyl, pyridazinyl, pyridooxazole, pyridoimidazole, pyridothiazole, pyridinyl, pyridyl, pyrimidinyl, pyrrolidinyl, pyrrolinyl, 2H-pyrrolyl, pyrrolyl, quinazolinyl, quinolinyl, 4*H*-quinolizinyl, quinoxalinyl, quinuclidinyl, tetrahydrofuranyl, tetrahydroisoquinolinyl, tetrahydroquinolinyl, tetrazolyl, 6*H*-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl, and xanthenyl.

As used herein, the term "amine protecting group" is intended to mean a functional group that converts an amine, amide, or other nitrogen-containing moiety into a different chemical group that is substantially inert to the conditions of a particular chemical reaction. Amine protecting groups are preferably removed easily and selectively in good yield under conditions that do not affect other functional groups of the molecule. Examples of amine protecting groups include, but are not limited to, benzyl, *t*-butyldimethylsilyl, *t*-butdyldiphenylsilyl, *t-*butyloxycarbonyl, *p*-methoxybenzyl, methoxymethyl, tosyl, trifluoroacetyl, trimethylsilyl, fluorenyl-methyloxycarbonyl, 2-trimethylsilyl-ethyoxycarbonyl, 1-methyl-1-(4-biphenylyl) ethoxycarbonyl, allyloxycarbonyl, and benzyloxycarbonyl. Other suitable amine protecting groups are straightforwardly identified by those of skill in the art, e.g., by reference to Green & Wuts, Protective Groups in Organic Synthesis, 3d Ed. (1999, John Wiley & Sons, Inc.).

"Stable compound" and "stable structure" are meant to indicate a compound that is sufficiently robust to survive isolation to a useful degree of purity from a reaction mixture, and formulation into an efficacious therapeutic agent.

All percentages and ratios used herein, unless otherwise indicated, are by weight.

Throughout the description, where compositions are described as having, including, or comprising specific components, it is contemplated that compositions also consist essentially of, or consist of, the recited components. Similarly, where processes are described as having, including, or comprising specific process steps, the processes also consist essentially of, or consist of, the recited processing steps. Further, it should be understood that the order of steps or order for performing certain actions are immaterial so long as the invention remains operable. Moreover, two or more steps or actions may be conducted simultaneously.

### 2. Processes of the Invention

The processes of the invention involve a Suzuki-type coupling reaction between an aryl borane compound (e.g., an aryl boronic acid, aryl boronic ester, aryl boronic halide, or organoborane) and an aryl compound having an electronegative substituent (e.g., an aryl halide or aryl sulfonate) in a solvent in the presence of a base and a palladium catalyst. *See, e.g.,* Miyaura et al., Tetrahedron Letters, 3437 (1979), and Miyaura & Suzuki, Chem. Comm., 866 (1979).

Disclosed herein are processes for synthesizing compounds having the formula:

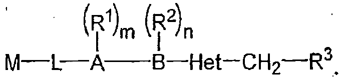

In one approach, the process includes the step of combining a compound of formula (I): 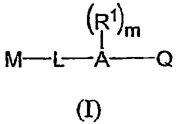 with a compound of formula (II): 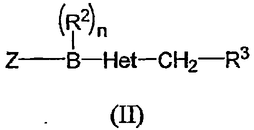 in a solvent in the presence of a base and a palladium catalyst, wherein
A is selected from the group consisting of:
   phenyl, pyridyl, pyrazinyl, pyrimidinyl, and pyridazinyl;
B is selected from the group consisting of:
   phenyl, pyridyl, pyrazinyl, pyrimidinyl, and pyridazinyl;
Het-CH₂-R³ is selected from the group consisting of: 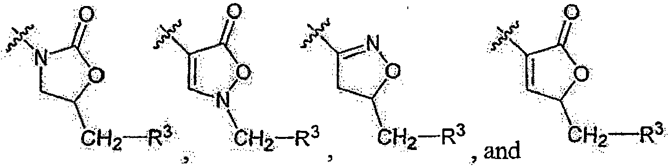
M-L is selected from the group consisting of:
   a) M-X, b) M-L¹, c) M-L¹-X, d) M-X-L², e) M-L¹-X-L², f) M-X-L¹-X-L²,
   g) M-L¹-X-L²-X, h) M-X-X-, i) M-L¹-X-X-, j) M-X-X-L², and
   k) M-L¹-X-X-L², wherein
X, at each occurrence, independently is selected from the group consisting of:
   a) -O-, b) -NR⁴-, c) -N(O)-, d) N(OR⁴)-, e) -S(O)ₚ-, f) -SO₂NR⁴-, g) -NR⁴SO₂-, h) -NR⁴-N=, i) =N-NR⁴-, j) -O-N=, k) =N-O-, l) -N=, m) =N-, n) -NR⁴-NR⁴-, o) -NR⁴C(O)O-, p) -OC(O)NR⁴-, q) -NR⁴C(O)NR⁴- r) NR⁴C(NR⁴)NR⁴-, and s) 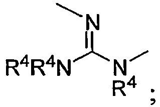
L¹ is selected from the group consisting of:
   a) C₁₋₆ alkyl, b) C₂₋₆ alkenyl, and c) C₂₋₆ alkynyl,
      wherein any of a) - c) optionally is substituted with one or more R⁵ groups; and
L² is selected from the group consisting of:
   a) C₁₋₆ alkyl, b) C₂₋₆ alkenyl, and c) C₂₋₆ alkynyl,
      wherein any of a) - c) optionally is substituted with one or more R⁵ groups;
   alternatively, L in M-L is a bond;
M is selected from the group consisting of:
   a) C₃₋₁₄ saturated, unsaturated, or aromatic carbocycle, b) 3-14 membered saturated, unsaturated, or aromatic heterocycle containing one or more heteroatoms selected from the group consisting of nitrogen, oxygen, and sulfur, c) C₁₋₆ alkyl, d) C₂₋₆ alkenyl, e) C₂₋₆ alkynyl, and f) -CN,
      wherein any of a) - e) optionally is substituted with one or more R⁵ groups;
Q is a borane having the formula -BY₂, wherein
   Y, at each occurrence, independently is selected from the group consisting of:
   a) -OH, b) -OC₁₋₆ alkyl, c) -OC₂₋₆ alkenyl, d) -OC₂₋₆ alkynyl, e) -OC₁₋₁₄ saturated, unsaturated, or aromatic carbocycle, f) C₁₋₆ alkyl, g) C₂₋₆ alkenyl, h) C₂₋₆ alkynyl, and i) C₁₋₁₄ saturated, unsaturated, or aromatic carbocycle,
      wherein any of b) - i) optionally is substituted with one or more halogens;
   alternatively, two Y groups taken together comprise a chemical moiety selected from the group consisting of:
   a) -OC(R⁴)(R⁴)C(R⁴)(R⁴)O-, and b) -OC(R⁴)(R⁴)CH₂C(R⁴)(R⁴)O-;
   alternatively, Q is a BF₃ alkali metal salt or 9-borabicyclo[3.3.1]nonane;
Z is selected from the group consisting of:
   a) I, b) Br, c) Cl, and d) R⁹OSO₃-;
R¹, at each occurrence, independently is selected from the group consisting of:
   a) F, b) Cl, c) Br, d) I, e) -CF₃, f) -OR⁴, g) -CN, h) -NO₂, i) -NR⁴R⁴, j) -C(O)R⁴, k) -C(O)OR⁴, l) -OC(O)R⁴, m) -C(O)NR⁴R⁴, n) -NR⁴C(O)R⁴, o) -OC(O)NR⁴R⁴, p) NR⁴C(O)OR⁴, q) NR⁴C(O)NR⁴R⁴, r) -C(S)R⁴, s) -C(S)OR⁴, t) -OC(S)R⁴, u) -C(S)NR⁴R⁴, v) -NR⁴C(S)R⁴, w) -OC(S)NR⁴R⁴, x) NR⁴C(S)OR⁴, y) -NR⁴C(S)NR⁴R⁴, z) -C(NR⁴)R⁴, aa) -C(NR⁴)OR⁴, bb) -OC(NR⁴)R⁴, cc) -C(NR⁴)NR⁴R⁴, dd) -NR⁴C(NR⁴)R⁴, ee) -OC(NR⁴)NR⁴R⁴, ff) -NR⁴C(NR⁴)OR⁴, gg) -NR⁴C(NR⁴)NR⁴R⁴, hh) -S(O)ₚR⁴, ii) -SO₂NR⁴R⁴, and jj) R⁴;
R², at each occurrence, independently is selected from the group consisting of:
   a) F, b) Cl, c) Br, d) I, e) -CF₃, f) -OR⁴, g) -CN, h) -NO₂, i) NR⁴R⁴,j) -C(O)R⁴, k) -C(O)OR⁴,l) -OC(O)R⁴, m) -C(O)NR⁴R⁴, n) -NR⁴C(O)R⁴, o) -OC(O)NR⁴R⁴, p) NR⁴C(O)OR⁴, q) NR⁴C(O)NR⁴R⁴, r) -C(S)R⁴, s) -C(S)OR⁴, t) -OC(S)R⁴, u) -C(S)NR⁴R⁴, v) NR⁴C(S)R⁴, w) -OC(S)NR⁴R⁴, x) NR⁴C(S)OR⁴, y) -NR⁴C(S)NR⁴R⁴, z) -C(NR⁴)R⁴, aa) -C(NR⁴)OR⁴, bb) -OC(NR⁴)R⁴, cc) -C(NR⁴)NR⁴R⁴, dd) NR⁴C(NR⁴)R⁴, ee) -OC(NR⁴)NR⁴R⁴, ff) -NR⁴C(NR⁴)OR⁴, gg) -NR⁴C(NR⁴)NR⁴R⁴, hh) -S(O)ₚR⁴, ii) -SO₂NR⁴R⁴, and jj) R⁴;
R³ is selected from the group consisting of:
   a) -OR⁴, b) NR⁴R⁴, c) -C(O)R⁴, d) -C(O)OR⁴, e) -OC(O)R⁴, f) -C(O)NR⁴R⁴, g) NR⁴C(O)R⁴, h) -OC(O)NR⁴R⁴, i) NR⁴C(O)OR⁴, j) NR⁴C(O)NR⁴R⁴, k) -C(S)R⁴, l) -C(S)OR⁴, m) -OC(S)R⁴, n) -C(S)NR⁴R⁴, o) -NR⁴C(S)R⁴, p) -OC(S)NR⁴R⁴, q) NR⁴C(S)OR⁴, r) NR⁴C(S)NR⁴R⁴, s) -C(NR⁴)R⁴, t) -C(NR⁴)OR⁴, u) -OC(NR⁴)R⁴, v) -C(NR⁴)NR⁴R⁴, w) -NR⁴C(NR⁴)R⁴, x) -OC(NR⁴)NR⁴R⁴, y) -NR⁴C(NR⁴)OR⁴, z) -NR⁴C(NR⁴)NR⁴R⁴, aa) -S(O)ₚR⁴, bb) -SO₂NR⁴R⁴, and cc) R⁴;
R⁴, at each occurrence, independently is selected from the group consisting of:
   a) H, b) -OR⁶, c) an amine protecting group, d) C₁₋₆ alkyl, e) C₂₋₆ alkenyl, f) C₂₋₆ alkynyl, g) C₃₋₁₄ saturated, unsaturated, or aromatic carbocycle, h) 3-14 membered saturated, unsaturated, or aromatic heterocycle comprising one or more heteroatoms selected from the group consisting of nitrogen, oxygen, and sulfur, i) -C(O)-C₁₋₆ alkyl, j) -C(O)-C₂₋₆ alkenyl, k) -C(O)-C₂₋₆ alkynyl, l) -C(O)-C₃₋₁₄ saturated, unsaturated, or aromatic carbocycle, m) -C(O)-3-14 membered saturated, unsaturated, or aromatic heterocycle comprising one or more heteroatoms selected from the group consisting of nitrogen, oxygen, and sulfur, n) -C(O)O-C₁₋₆ alkyl, o) -C(O)O-C₂₋₆ alkenyl, p) -C(O)O-C₂₋₆ alkynyl, q) -C(O)O-C₃₋₁₄ saturated, unsaturated, or aromatic carbocycle, and r) -C(O)O-3-14 membered saturated, unsaturated, or aromatic heterocycle comprising one or more heteroatoms selected from the group consisting of nitrogen, oxygen, and sulfur,
      wherein any of d) - r) optionally is substituted with one or more R⁵ groups;
R⁵, at each occurrence, is independently selected from the group consisting of:
   a) F, b) Cl, c) Br, d) I, e) =O, f) =S, g) NR⁶, h) NOR⁶, i) =N-NR⁶R⁶, j) -CF₃, k) - OR⁶, l) -CN, m) -NO₂, n) NR⁶R⁶, o) -C(O)R⁶, p) -C(O)OR⁶, q) -OC(O)R⁶, r) -C(O)NR⁶R⁶, s) NR⁶C(O)R⁶, t) -OC(O)NR⁶R⁶, u) -NR⁶C(O)OR⁶, v) -NR⁶C(O)NR⁶R⁶, w) -C(S)R⁶, x) -C(S)OR⁶, y) -OC(S)R⁶, z) -C(S)NR⁶R⁶, aa) -NR⁶C(S)R⁶, bb) -OC(S)NR⁶R⁶, cc) -NR⁶C(S)OR⁶, dd) NR⁶C(S)NR⁶R⁶, ee) -C(NR⁶)R⁶, ff) -C(NR⁶)OR⁶, gg) -OC(NR⁶)R⁶, hh) -C(NR⁶)NR⁶R⁶, ii) -NR⁶C(NR⁶)R⁶, jj) -OC(NR⁶)NR⁶R⁶, kk) -NR⁶C(NR⁶)OR⁶, ll) -NR⁶C(NR⁶)NR⁶R⁶, mm) -S(O)ₚR⁶, nn) -SO₂NR⁶R⁶, and oo) R⁶;
R⁶, at each occurrence, independently is selected from the group consisting of:
   a) H, b) -OR⁸, c) an amine protecting group, d) C₁₋₆ alkyl, e) C₂₋₆ alkenyl, f) C₂₋₆ alkynyl, g) C₃₋₁₄ saturated, unsaturated, or aromatic carbocycle, h) 3-14 membered saturated, unsaturated, or aromatic heterocycle comprising one or more heteroatoms selected from the group consisting of nitrogen, oxygen, and sulfur, i) -C(O)-C₁₋₆ alkyl, j) -C(O)-C₂₋₆ alkenyl, k) -C(O)-C₂₋₆ alkynyl, l) -C(O)-C₃₋₁₄ saturated, unsaturated, or aromatic carbocycle, m) -C(O)-3-14 membered saturated, unsaturated, or aromatic heterocycle comprising one or more heteroatoms selected from the group consisting of nitrogen, oxygen, and sulfur, n) -C(O)O-C₁₋₆ alkyl, o) -C(O)O-C₂₋₆ alkenyl, p) -C(O)O-C₂₋₆ alkynyl, q) -C(O)O-C₃₋₁₄ saturated, unsaturated; or aromatic carbocycle, and r) -C(O)O-3-14 membered saturated, unsaturated, or aromatic heterocycle comprising one or more heteroatoms selected from the group consisting of nitrogen, oxygen, and sulfur,
      wherein any of d) - r) optionally is substituted with one or more R⁷ groups;
R⁷, at each occurrence, independently is selected from the group consisting of:
   a) F, b) Cl, c) Br, d) I, e) =O, f) =S, g) =NR⁸, h) NOR⁸, i) N-NR⁸R⁸, j) -CF₃, k) - OR⁸, l) -CN, m) -NO₂, n) NR⁸R⁸, o) -C(O)R⁸, p) -C(O)OR⁸, q) -OC(O)R⁸, r) -C(O)NR⁸R⁸, s) -NR⁸C(O)R⁸, t) -OC(O)NR⁸R⁸, u) -NR⁸C(O)OR⁸, v) -NR⁸C(O)NR⁸R⁸, w) -C(S)R⁸, x) -C(S)OR⁸, y) -OC(S)R⁸, z) -C(S)NR⁸R⁸, aa) -NR⁸C(S)R⁸, bb) -OC(S)NR⁸R⁸, cc) -NR⁸C(S)OR⁸, dd) -NR⁸C(S)NR⁸R⁸, ee) -C(NR⁸)R⁸, ff) -C(NR⁸)OR⁸, gg) -OC(NR⁸)R⁸, hh) -C(NR⁸)NR⁸R⁸, ii) NR⁸C(NR⁸)R⁸, jj) -OC(NR⁸)NR⁸R⁸, kk) NR⁸C(NR⁸)OR⁸, ll) -NR⁸C(NR⁸)NR⁸R⁸, mm) -S(O)ₚR⁸, nn) -SO₂NR⁸R⁸, oo) C₁₋₆ alkyl, pp) C₂₋₆ alkenyl, qq) C₂₋₆ alkynyl, rr) C₃₋₁₄ saturated, unsaturated, or aromatic carbocycle, and ss) 3-14 membered saturated, unsaturated, or aromatic heterocycle comprising one or more heteroatoms selected from the group consisting of nitrogen, oxygen, and sulfur,
      wherein any of oo) - ss) optionally is substituted with one or more moieties selected from the group consisting of R⁸, F, Cl, Br, I, -CF₃, -OR⁸, -SR⁸, -CN, -NO₂, -NR⁸R⁸, -C(O)R⁸, -C(O)OR⁸, -OC(O)R⁸, -C(O)NR⁸R⁸, -NR⁸C(O)R⁸, -OC(O)NR⁸R⁸, NR⁸C(O)OR⁸, -NR⁸C(O)NR⁸R⁸, -C(S)R⁸, -C(S)OR⁸, -OC(S)R⁸, -C(S)NR⁸R⁸, -NR⁸C(S)R⁸, -OC(S)NR⁸R⁸, -NR⁸C(S)OR⁸, NR⁸C(S)NR⁸R⁸, -C(NR⁸)R⁸, -C(NR⁸)OR⁸, -OC(NR⁸)R⁸, -C(NR⁸)NR⁸R⁸, -NR⁸C(NR⁸)R⁸, -OC(NR⁸)NR⁸R⁸, NR⁸C(NR⁸)OR⁸, -NR⁸C(NR⁸)NR⁸R⁸, -SO2NR⁸R⁸, and -S(O)ₚR⁸;
R⁸, at each occurrence, independently is selected from the group consisting of:
   a) H, b) an amine protecting group, c) C₁₋₆ alkyl, d) C₂₋₆ alkenyl, e) C₂₋₆ alkynyl, f) C₃₋₁₄ saturated, unsaturated, or aromatic carbocycle, g) 3-14 membered saturated, unsaturated, or aromatic heterocycle comprising one or more heteroatoms selected from the group consisting of nitrogen, oxygen, and sulfur, h) -C(O)-C₁₋₆ alkyl, i) -C(O)-C₂₋₆ alkenyl, j) -C(O)-C₂₋₆ alkynyl, k) -C(O)-C₃₋₁₄ saturated, unsaturated, or aromatic carbocycle, l) -C(O)-3-14 membered saturated, unsaturated, or aromatic heterocycle comprising one or more heteroatoms selected from the group consisting of nitrogen, oxygen, and sulfur, m) -C(O)O-C₁₋₆ alkyl, n) -C(O)O-C₂₋₆ alkenyl, o) -C(O)O-C₂₋₆ alkynyl, p) -C(O)O-C₃₋₁₄ saturated, unsaturated, or aromatic carbocycle, and q) -C(O)O-3-14 membered saturated, unsaturated, or aromatic heterocycle comprising one or more heteroatoms selected from the group consisting of nitrogen, oxygen, and sulfur,
      wherein any of c) -q) optionally is substituted with one or more moieties selected from the group consisting of F, Cl, Br, I, -CF₃, -OH, -OC₁₋₆ alkyl, -SH, -SC₁₋₆ alkyl, -CN, NO₂, NH₂, -NHC₁₋₆ alkyl, -N(C₁₋₆ alkyl)₂, -C(O)C₁₋₆ alkyl, -C(O)OC₁₋₆ alkyl, -C(O)NH₂, -C(O)NHC₁₋₆ alkyl, -C(O)N(C₁₋₆ alkyl)₂, -NHC(O)C₁₋₆ alkyl, -SO₂NH₂-, -SO₂NHC₁₋₆ alkyl, -SO₂N(C₁₋₆ alkyl)₂, and -S(O)ₚC₁₋₆ alkyl;
R⁹ is selected from the group consisting of:
   a) C₁₋₆ alkyl, b) phenyl, and c) toluyl;
      wherein any of a) - c) optionally is substituted with one or more moieties selected from the group consisting of F, Cl, Br, and I;
m is 0, 1, 2, 3, or 4;
n is 0, 1, 2, 3, or 4; and
p, at each occurrence, independently is 0, 1, or 2.

In an alternative approach, the method includes the step of combining a compound of formula (III): 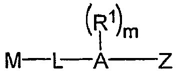 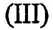 with a compound of formula (IV): 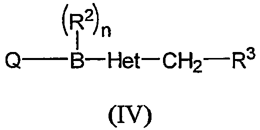 in a solvent in the presence of a base and a palladium catalyst, wherein A, B, Het, L, M, R¹, R², R³, R⁴, Q, Z, m, and n are defined as described above.

In another aspect, the invention provides processes for preparing a compound having the formula:

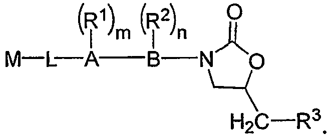

In one approach, the process includes the step of combining a compound of formula (V): 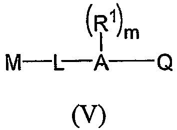 with a compound of formula (VI): 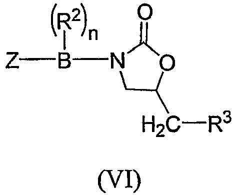 in a solvent in the presence of a base and a palladium catalyst. In an alternative approach, the method includes the step of combining a compound of formula (VII): 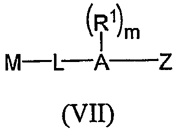 with a compound of formula (VIII): 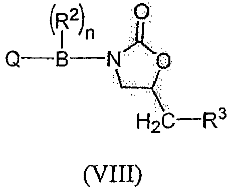 in a solvent in the presence of a base and a palladium catalyst. In either approach, A, B, L, M, R¹, R², R³, Q, Z, m, and n are defined as described above.

In yet another aspect, the disclosure provides processes for preparing a compound having the formula:

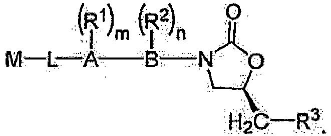

In one approach, the process includes the step of combining a compound of formula (IX): 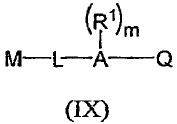 with a compound of formula (X): 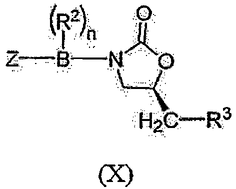 in a solvent in the presence of a base and a palladium catalyst. Alternatively, the compound can be prepared by combining a compound of formula (XI): 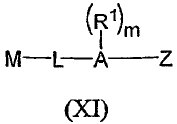 with a compound of formula (XII): 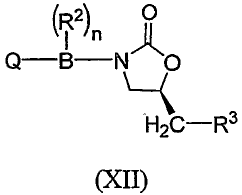 in a solvent in the presence of a base and a palladium catalyst. In either approach, A, B, L, M, R¹, R², R³, Q, Z, m, and n are defined as described above.

Embodiments of any of the above processes can include the following:

In preferred compounds, A and B independently are selected from the group consisting of phenyl and pyridyl, and m and n independently are 0, 1, or 2. R³ can be triazole, tetrazole, oxazole, or isoxazole, and particularly [1,2,3]triazol-1-yl. Alternatively, R³ can be NHC(O)R⁴, and particularly -NHC(O)CH₃.

In various embodiments, compounds (II), (VI), or (X) can have a formula selected from: 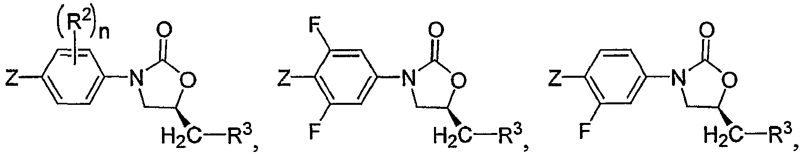 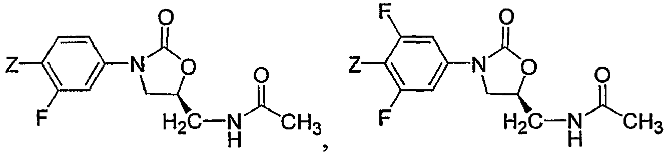 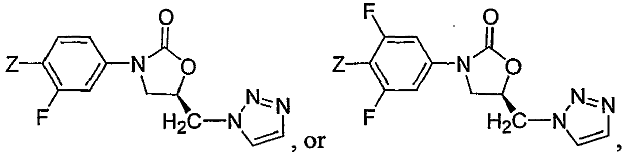 wherein R², R³, Z, and n are defined as described above.

In addition, compounds (I), (V), or (IX) can have a formula selected from: 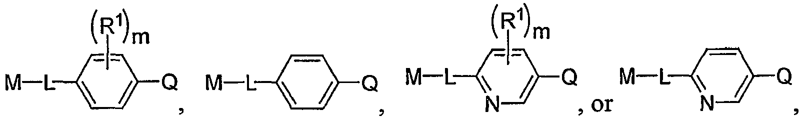 wherein L, M, Q, R¹, and m are defined as described above.

In other embodiments, compounds (IV), (VIII), or (XII) can have a formula selected from: 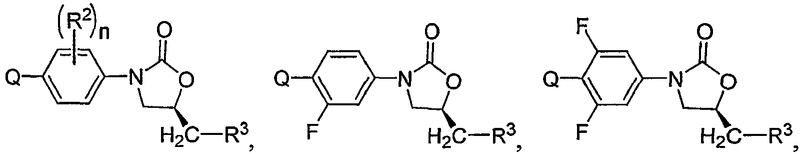 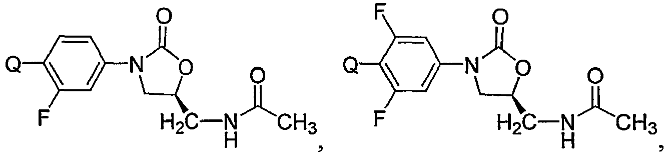 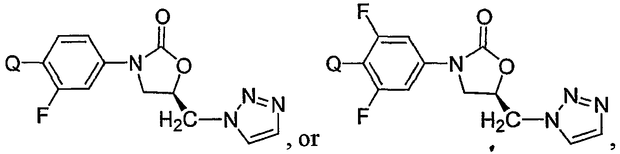 wherein R², R³, Q, and n are defined as described above.

Additionally, compounds (III), (VII), or (XI) can have one of the following formulas: 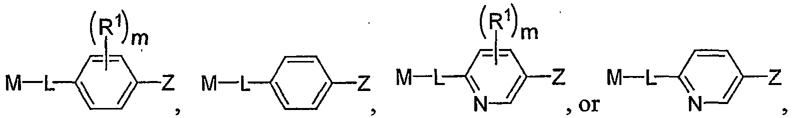 wherein L, M, R¹, Z, and m are defined as described above.

In any of these embodiments, M-L can be M-CH₂-X-CH₂-. In some embodiments, X is -NR⁴-, where R⁴ can be, for example, H or an amine protecting group. Examples of suitable amine protecting groups include benzyl, *t*-butyldimethylsilyl, *t*-butdyldiphenylsilyl, *t*-butyloxycarbonyl, *p*-methoxybenzyl, methoxymethyl, tosyl, trifluoroacetyl, trimethylsilyl, fluorenyl-methyloxycarbonyl, 2-trimethylsilyl-ethyoxycarbonyl, 1-methyl-1-(4-biphenylyl)ethoxycarbonyl, allyloxycarbonyl, and benzyloxycarbonyl. In embodiments where R⁴ is an amine protecting group, the processes can include the step of removing the amine protecting group.

In alternative embodiments, M-L is M-S-L¹-NR⁴- L², wherein L¹ and L² are C₁₋₆ alkyl. Particularly, M-L can be M-S-CH₂CH₂-NH-CH₂-. In still other embodiments, L is C₁₋₆ alkyl, and particularly -CH₂-.

In the above embodiments, M can be a 5-6 membered saturated, unsaturated, or aromatic heterocycle comprising one or more heteroatoms selected from the group consisting of nitrogen, oxygen, and sulfur. Examples of suitable heterocycles include triazole, tetrazole, oxazole, and isoxazole, and particularly isoxazol-4-yl, [1,2,3]triazol-1-yl, and [1,2,3]triazol-4-yl.

Alternatively, M-L can be M-X-CH₂-. In some embodiments, X is -NR⁴-, where R⁴ can be, for example, H or an amine protecting group, as described above. In embodiments where R⁴ is an amine protecting group, the processes can include the step of removing the amine protecting group. In other embodiments, M-L is 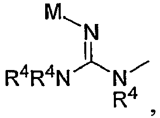 or, more particularly,

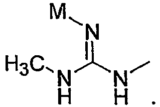

In the above embodiments, M can be a halogenated C₁₋₆ alkyl, C₂₋₆ alkenyl, or C₂₋₆ alkynyl group, optionally substituted with one or more R⁵ groups, as defined above. Alternatively, M can be a-CN group. In particular embodiments, M is a C₁₋₆ alkyl substituted with one or more atoms selected from the group consisting of F, Cl, Br, and I, for example, -CH₂CH₂CH₂F. M can also be a C₁₋₆ alkyl substituted with one or more -CN groups, for example, -CH₂CH₂CN. Other examples of suitable M groups include, but are not limited to, -CH₂CH(OH)CH₂F and -CH₂C(O)NH₂.

In alternative embodiments, M is C₁₋₆ alkyl, C₂₋₆ alkenyl, or C₂₋₆ alkynyl, wherein one or more carbons is replaced with S(O)ₚ and one or more of the remaining carbons optionally is substituted with one or more R⁵ groups.

Other embodiments include compounds wherein M has the formula: 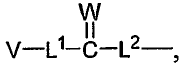 wherein V is H, -NR⁴R⁴, -OR⁴, or C₁₋₆ alkyl optionally substituted with one or more R⁵ groups; W is O or S; and L¹ and L² are defined as described above.

The Z group can be a halogen or a sulfonate. Examples of suitable sulfonates include, but are not limited to, methanesulfonate ("mesylate"), trifluoromethanesulfonate ("triflate"), and *p*-toluenesulfonate ("tosylate"). In preferred embodiments, the Z group is I. Preferred Q groups include -B(OH)₂, -BF₂·KF, and

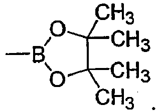

In any of the above processes, the base can be selected from the group consisting of alkali metal hydroxides, alkali metal carbonates, alkali metal fluorides, trialkyl amines, and mixtures thereof. Examples of suitable bases include potassium carbonate, sodium carbonate, sodium methoxide, sodium ethoxide, potassium fluoride, triethylamine, diisopropylethylamine, and mixtures thereof. In certain embodiments, the ratio of equivalents of base to equivalents of compound (I), (IV), (V), (VIII), (IX), or (XII) is about 3:1.

The catalyst can be a palladium catalyst, for example, a ligand coordinated palladium(0) catalyst (e.g., a tetrakis(trialkylphosphine)palladium(0) or a tetrakis(triarylphosphine) palladium(0) catalyst) or a ligand coordinated palladium(II) catalyst. Suitable catalysts include, for example, tetrakis(triphenylphosphine)palladium(0), dichloro[1,1'-bis(diphenylphosphino) ferrocene]palladium(II), dichlorobis(triphenylphosphine)palladium(II), palladium(II) acetate, and palladium(II) chloride. In particular embodiments, the catalyst is tetrakis(triphenylphosphine) palladium(0), and the ratio of the equivalents of the catalyst to the equivalents of compound (I), (IV), (V), (VIII), (IX), or (XII) is about 1:20.

The solvent can be an aqueous solvent, or a mixture of water and an organic solvent, wherein the organic solvent is selected from the group consisting of methanol, ethanol, propanol, isopropanol, butanol, isobutanol, secondary butanol, tertiary butanol, benzene, toluene, tetrahydrofuran, dimethylformamide, 1,2-diethyl ether, dimethoxyethane, diisopropyl ether, methyltertiarybutyl ether, methoxymethyl ether, 2-methoxyethyl ether, 1,4-dioxane, 1,3-dioxolane, and mixtures thereof. In particular embodiments, the solvent is a mixture of water, toluene, and ethanol, for example, in a ratio of about 1:3:1 by volume.

The process can be carried out at a temperature of about 20 °C to about 100 °C. In some embodiments, the process is carried out at the reflux temperature of the solvent.

Other reaction conditions for the Suzuki-type coupling reactions described herein are straightforwardly identified by those of skill in the art, e.g., by reference to Suzuki & Brown, Organic Synthesis Via Boranes Volume 3: Suzuki Coupling, (Aldrich, 2003).

Table 1 includes exemplary compounds that can be synthesized according to the processes described herein.

**Table 1**

| **Compound Number** | **Structure** |
|---|---|
| **1** | 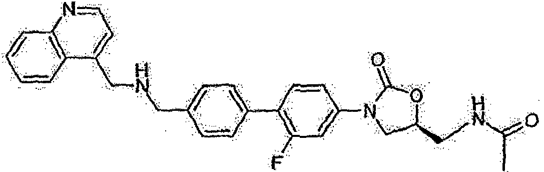 |
| | (5S)N-[3-(2-Fluro-4'-{[(quinolin-4-ylmethyl)-amino]-methyl}-biphenyl-4-yl)-2-oxo-oxazolidin-5-ylmethyl]-acetamide |
| **2** | 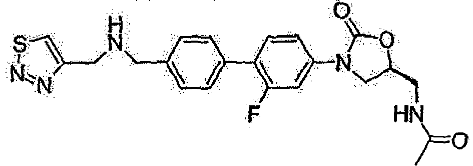 |
| | (5S)N-[3-(2-Fluloro-4'-{[([1,2,3]thiadiazol-4-ylmethyl)-amino]-methyl}-biphenyl-4-yl)-2-oxo-oxazolidin-5-ylmethyl]-acetamide |
| **3** | 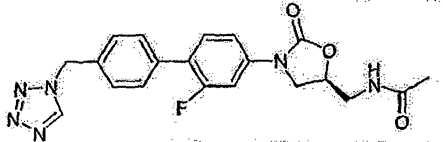 |
| | (5S)N-{3-[3-Fluoro-4-(6-tetrazol-1-ylmethyl-pyridin-3-yl)-phenyl]-2-oxo-oxazolidin-5-ylmethyl}-acetamide |
| **4** | 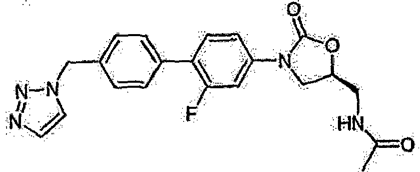 |
| | (5S)N-[3-(2-Fluoro-4'-[1,2,3]triazol-1-ylmethyl-biphenyl-4-yl)-2-oxo-oxazolidin-5-ylmethyl]-acetamide |
| **5** | 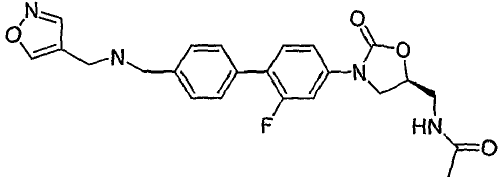 |
| | (5S)N-[3-(2-Fluoro-4'-{[(isoxazol-4-ylmethyl)-amino]-methyl}-biphenyl-4-yl)-2-oxo-oxazolidin-5-ylmethyl]-acetamide |
| **6** | 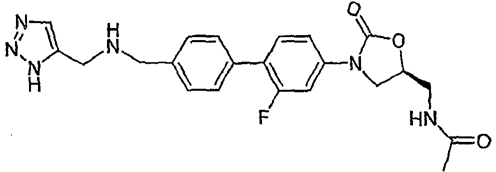 |
| | (5S)N-[3-(2-Fluoro-4'-{[(3H-[1,2,3]triazol-4-ylmethyl)-amino]-methyl}-biphenyl-4-yl)-2-oxo-oxazolidin-5-ylmethyl]-acetamide |
| **7** | 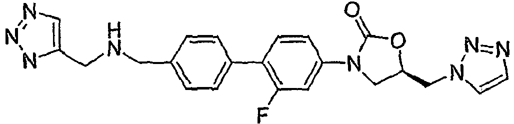 |
| | (5R)3-(2-Fluoro-4'-{[(3H-[1,2,3]triazol-4-ylmethyl)-amino]-methyl}-biphenyl-4-yl)-5-[1,2,3]triazol-1-ylmethyl-oxazolidin-2-one |
| **8** | 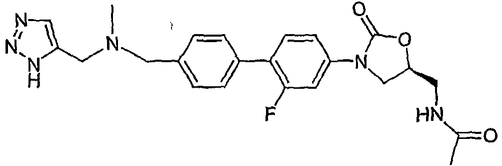 |
| | (5S)N-[3-(2-Fluoro-4'-{[methyl-(3H-[1,2,3]triazol-4-ylmethyl)-amino]-methyl}-biphenyl-4-yl)-2-oxo-oxazolidin-5-ylmethyl]-acetamide |
| **9** | 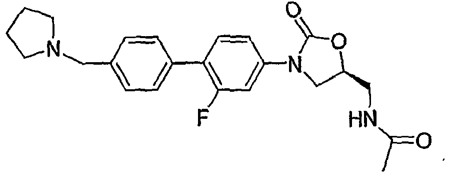 |
| | (5S)N-[3-(2-Fluoro-4'-pyrrolidin-1-ylmethyl-biphenyl-4-yl)-2-oxo-oxazolidin-5-ylmethyl]-acetamide |
| **10** | 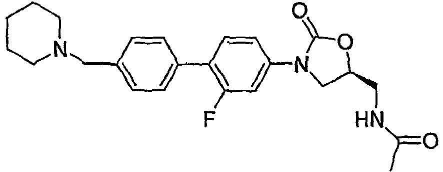 |
| | (5S)N-[3-(2-Fluoro-4'-piperidin-1-ylmethyl-biphenyl-4-yl)-2-oxo-oxazolidin-5-ylmethyl]-acetamide |
| **11** | 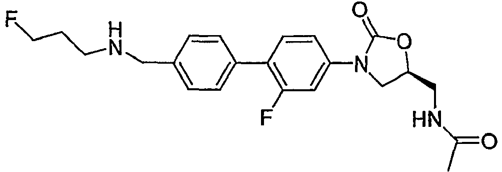 |
| | (5S)N-(3-{2-Fluoro-4'-[(3-fluoro-propylamino)-methyl]-biphenyl-4-yl}-2-oxo-oxazolidin-5-ylmethyl)-acetamide |
| **12** | 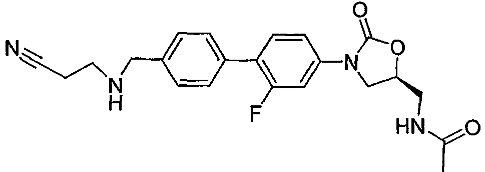 |
| | (5S)N-(3-{4'-[(2-Cyano-ethylamino)-methyl]-2-fluoro-biphenyl-4-yl}-2-oxo-oxazolidin-5-ylmethyl)-acetamide |
| **13** | 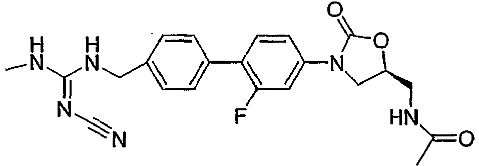 |
| | (5S)N-{3-[4-(4-N-(N-methyl-N'-cyano) guanylaminomethyl-phenyl-4-yl)-3-fluorophenyl]-2-oxo-oxazolidin-5-ylmethyl}-acetamide |
| **14** | 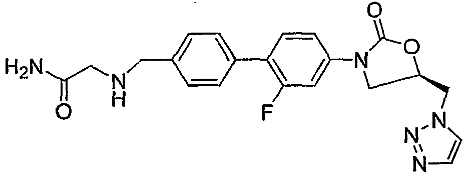 |
| | (5R)2-{[2'-Fluoro-4'-(2-oxo-5-[1,2,3]triazol-1-ylmethyl-oxazolidin-3-yl)-biphenyl-4-ylmethyl]-amino}-acetamide |
| **15** | 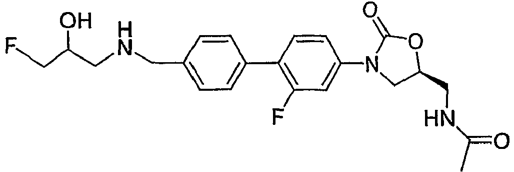 |
| | (5S)N-(3-{2-Fluoro-4'-[(3-fluoro-2-hydroxy-propylamino)-methyl]-biphenyl-4-yl}-2-oxo-oxazolidin-5-ylmethyl)-acetamide |
| **16** | 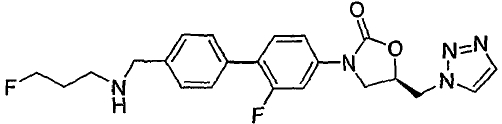 |
| | (5R)3-{2-Fluoro-4'-[(3-fluoro-propylamino)-methyl]-biphenyl-4-yl}-5-[1,2,3]triazol-1-ylmethyl-oxazolidin-2-one |
| **17** | 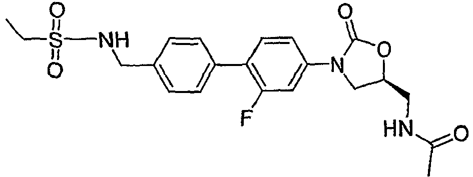 |
| | (5S)N-{3-[4'-(Ethanesulfonylamino-methyl)-2-fluoro-biphenyl-4-yl]-2-oxo-oxazolidin-5-ylmethyl}-acetamide |
| **18** | 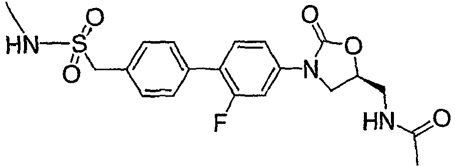 |
| | (5S)N-[3-(2-Fluoro-4'-methylsulfamoylmethyl-biphenyl-4-yl)-2-oxo-oxazolidin-5-ylmethyl]-acetamide |
| **19** | 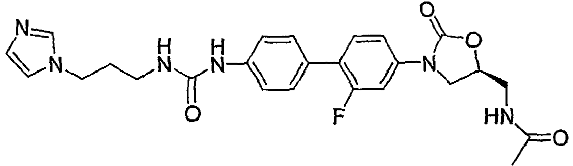 |
| | (5S)N-(3-{2-Fluoro-4'-[3-(3-imidazol-1-yl-propyl)-ureido]-biphenyl-4-yl}-2-oxo-oxazolidin-5-ylmethyl)-acetamide |
| **20** | 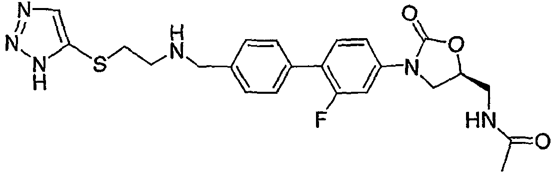 |
| | (5S)N-[3-(2-Fluoro-4'-{[2-(3H-[1,2,3]triazol-4-ylsulfanyl)-ethylamino]-methyl}-biphenyl-4-yl)-2-oxo-oxazolidin-5-ylmethyl]-acetamide |
| **21** | 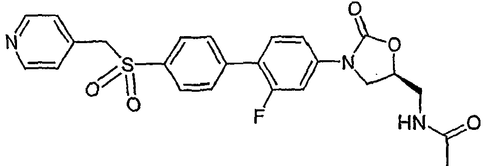 |
| | (5S)N-{3-[2-Fluoro-4'-(pyridin-4-ylmethanesulfonyl)-biphenyl-4-yl]-2-oxo-oxazolidin-5-ylmethyl}-acetamide |
| **22** | 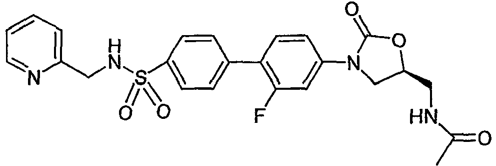 |
| | (5S)N-(3-{2-Fluoro-4'-[(pyridin-2-ylmethyl)-sulfamoyl]-biphenyl-4-yl}-2-oxo-oxazolidin-5-ylmethyl)-acetamide |
| **23** | 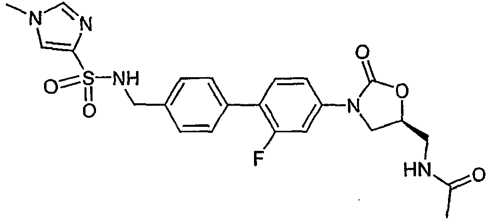 |
| | (5S)N-(3-{2-Fluoro-4'-[(1-methyl-1H-imidazole-4-sulfonylamino)-methyl]-biphenyl-4-yl}-2-oxo-oxazolidin-5-ylmethyl)-acetamide |
| **24** | 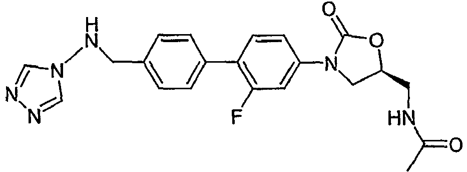 |
| | (5S)N-{3-[2-Fluoro-4'-([1,2,4]triazol-4-ylaminomethyl)-biphenyl-4-yl]-2-oxo-oxazolidin-5-ylmethyl}-acetamide |
| **25** | 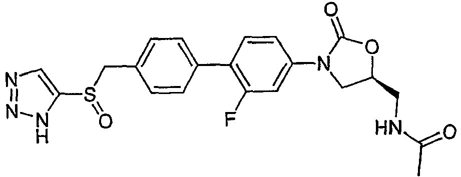 |
| | (5S)N-{3-[2-Fluoro-4'-(3H-[1,2,3]triazole-4-sulfonylmethyl)-biphenyl-4-yl]-2-oxo-oxazolidin-5-ylmethyl}-acetamide |
| **26** | 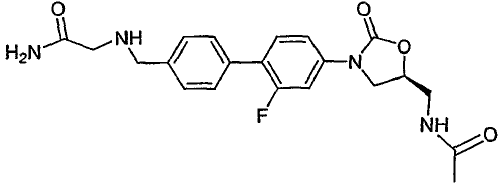 |
| | (5S)2-({4'-[5-(Acetylamino-methyl)-2-oxo-oxazolidin-3-yl]-2'-fluoro-biphenyl-4-ylmethyl}-amino)-acetamide |
| **27** | 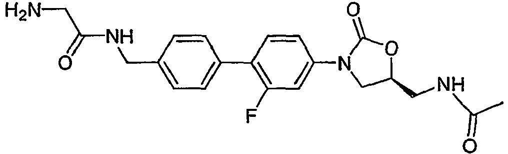 |
| | (5S)N-{4-[5-(Acetylamino-methyl)-2-oxo-oxazolidin-3-yl]-2'-fluoro-biphenyl-4-ylmethyl}-2-amino-acetamide |
| **28** | 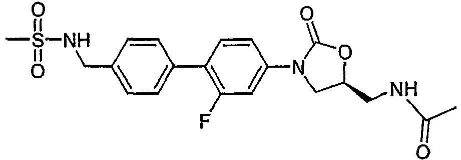 |
| | (5S)N-{3-[2-Fluoro-4'-(methanesulfonylamino-methyl)-biphenyl-4-yl]-2-oxo-oxazolidin-5-ylmethyl}-acetamide |
| **29** | 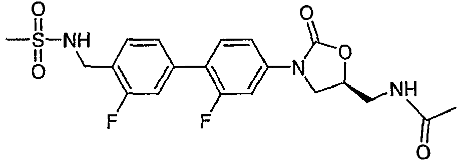 |
| | (5S)N-{3-[2,3'-Difluoro-4'-(methanesulfonylamino-methyl)-biphenyl-4-yl]-2-oxo-oxazolidin-5-ylmethyl}-acetamide |
| **30** | 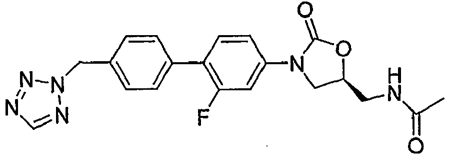 |
| | (5S)N-[3-(2-Fluoro-4'-tetrazol-2-ylmethyl-biphenyl-4-yl)-2-oxo-oxazolidin-5-ylmethyl]-acetamide |
| **31** | 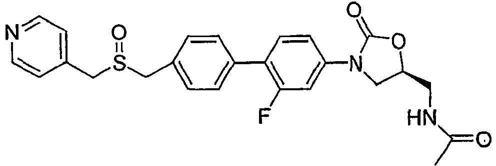 |
| | (5S)N-{3-[2-Fluoro-4'-(pyridin-4-ylmethanesulfinylmethyl)-biphenyl-4-yl]-2-oxo-oxazolidin-5-ylmethyl}-acetamide |
| **32** | 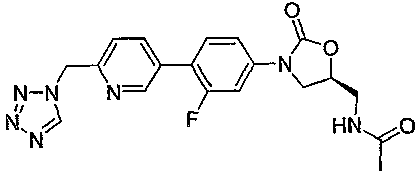 |
| | (5S)N-{3-[3-Fluoro-4-(6-tetrazol-1-ylmethyl-pyridin-3-yl)-phenyl]-2-oxo-oxazolidin-5-ylmethyl}-acetamide |
| **33** | 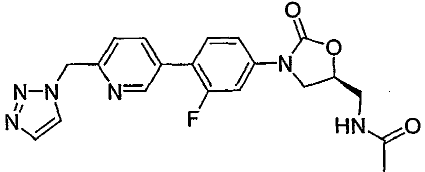 |
| | (5S)N-{3-[3-Fluoro-4-(6-[1,2,3]triazol-1-ylmethyl-pyridin-3-yl)-phenyl]-2-oxo-oxazolidin-5-ylmethyl}-acetamide |
| **34** | 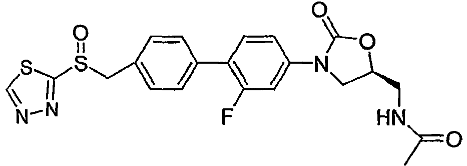 |
| | (5S)N-{3-[2-Fluoro-4'([1,3,4]thiadiazole-2-sulfinylmethyl)-biphenyl-4-yl]-2-oxo-oxazolidin-5-ylmethyl -acetamide |
| **35** | 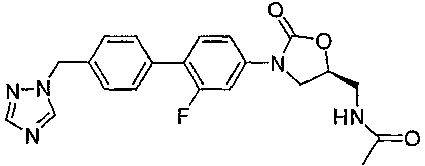 |
| | (5S)N-[3-(2-Fluoro-4'-[1,2,4]triazol-1-ylmethyl-biphenyl-4-yl)-2-oxo-oxazolidin-5-ylmethyl]-acetamide |
| **36** | 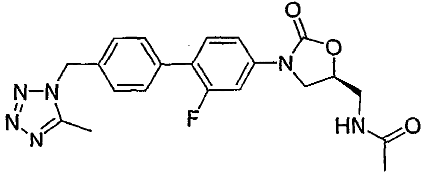 |
| | (5S)N-{3-[2-Fluoro-4'-(5-methyl-tetrazol-1-ylmethyl)-biphenyl-4-yl]-2-oxo-oxazolidin-5-ylmethyl}-acetamide |
| **37** | 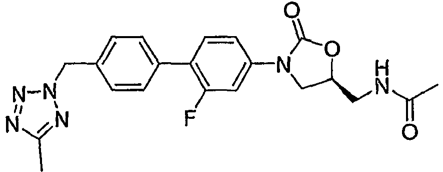 |
| | (5S)N-{3-[2-Fluoro-4'-(5-methyl-tetrazol-2-ylmethyl)-biphenyl-4-yl]-2-oxo-oxazolidin-5-ylmethyl}-acetamide |
| **38** | 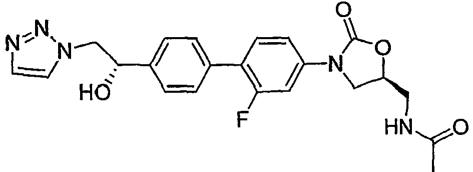 |
| | (S,S)N-{3-[2-Fluoro-4'-(1-hydroxy-2-[1,2,3]triazol-1-yl-ethyl)-biphenyl-4-yl]-2-oxo-oxazolidin-5-ylmethyl}-acetamide |
| **39** | 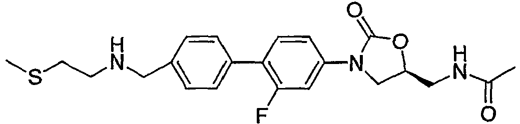 |
| | (5S)N-(3-{2-Fluoro-4'-[(2-methylsulfanyl-ethylamino)-methyl]-biphenyl-4-yl}-2-oxo-oxazolidin-5-ylmethyl)-acetamide |
| **40** | 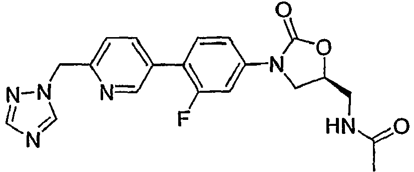 |
| | (5S)N-{3-[3-Fluoro-4-(6-[1,2,4]triazol-1-ylmethyl-pyridin-3-yl)-phenyl]-2-oxo-oxazolidin-5-ylmethyl}-acetamide |
| **41** | 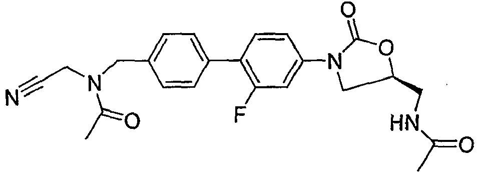 |
| | (5S)N-(3-{4'-[(Acetyl-cyanomethyl-amino)-methyl]-2-fluoro-biphenyl-4-yl}-2-oxo-oxazolidin-5-ylmethyl)-acetamide |
| **42** | 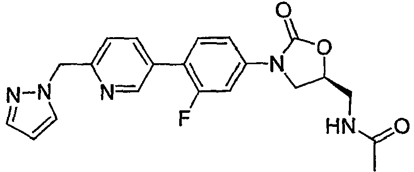 |
| | (5S)N-{3-[3-Fluoro-4-(6-pyrazol-1-ylmethyl-pyridin-3-yl)-phenyl]-2-oxo-oxazolidin-5-ylmethyl}-acetamide |
| **43** | 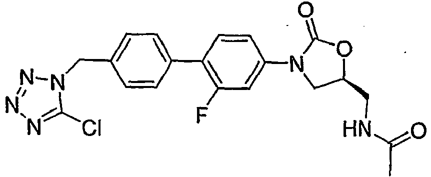 |
| | (5S)N-{3-[4'-(5-Chloro-tetrazol-1-ylmethyl)-2-fluoro-biphenyl-4-yl]-2-oxo-oxazolidin-5-ylmethyl}-acetamide |
| **44** | 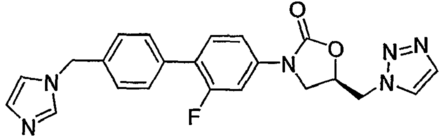 |
| | (5R)3-(2-Fluoro-4'-imidazol-1-ylmethyl-biphenyl-4-yl)-5-[1,2,3]triazol-1-ylmethyl-oxazolidin-2-one |
| **45** | 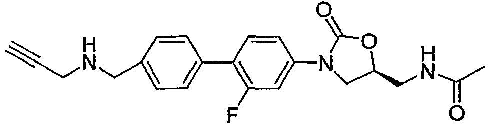 |
| | (5S)N-[3-(2-Fluoro-4'-prop-2-ynylaminomethyl-biphenyl-4-yl)-2-oxo-oxazolidin-5-ylmethyl]-acetamide |
| **46** | 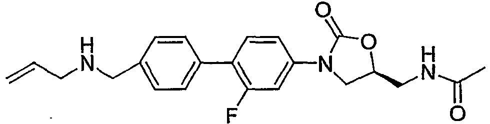 |
| | (5S)N-[3-(4'-Allylaminomethyl-2-fluoro-biphenyl-4-yl)-2-oxo-oxazolidin-5-ylmethyl]-acetamide |
| **47** | 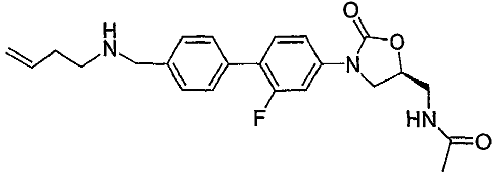 |
| | (5S)N-[3-(4'-But-3-enylaminomethyl-2-fluoro-biphenyl-4-yl)-2-oxo-oxazolidin-5-ylmethyl]-acetamide |
| **48** | 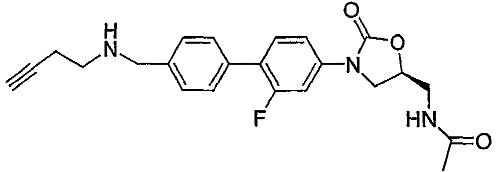 |
| | (5S)N-[3-(4'-But-3-ynylaminomethyl-2-fluoro-biphenyl-4-yl)-2-oxo-oxazolidin-5-ylmethyl]-acetamide |
| **49** | 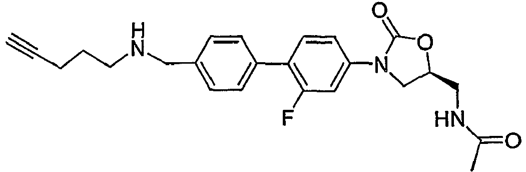 |
| | (5S)N-[3-(2-Fluoro-4'-pent-4-ynylaminomethyl-biphenyl-4-yl)-2-oxo-oxazolidin-5-ylmethyl]-acetamide |
| **50** | 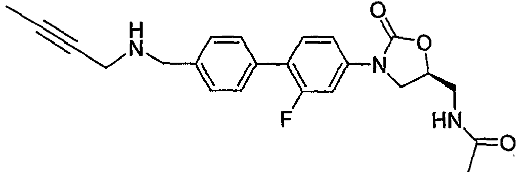 |
| | (5S)N-[3-(4'-But-2-ynylaminomethyl-2-fluoro-biphenyl-4-yl)-2-oxo-oxazolidin-5-ylmethyl]-acetamide |
| **51** | 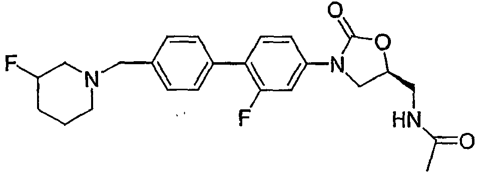 |
| | (5S)N-{3-[2-Fluoro-4'-(3-fluoro-piperidin-1-ylmethyl)-biphenyl-4-yl]-2-oxo-oxazolidin-5-ylmethyl}-acetamide |
| **52** | 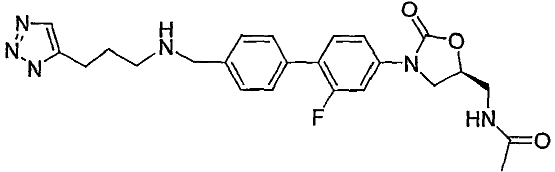 |
| | (5S)N-[3-(2-Fluoro-4'-{[3-(3H-[1,2,3]triazol-4-yl)-propylamino]-methyl}-biphenyl-4-yl)-2-oxo-oxazolidin-5-ylmethyl]-acetamide |
| **53** | 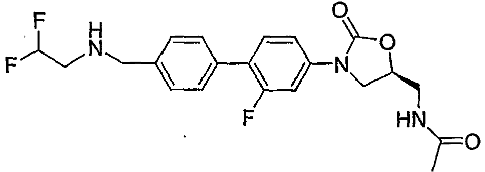 |
| | (5S)N-(3-{4'-[(2,2-Difluoro-ethylamino)-methyl]-2-fluoro-biphenyl-4-yl}-2-oxo-oxazolidin-5-ylmethyl)-acetamide |
| **54** | 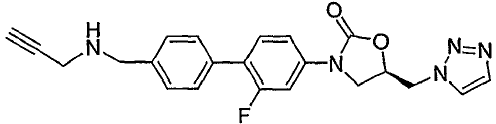 |
| | (5R)3-(2-Fluoro-4'-prop-2-ynylaminomethyl-biphenyl-4-yl)-5-[1,2,3]triazol-1-ylmethyl-oxazolidin-2-one |
| **55** | 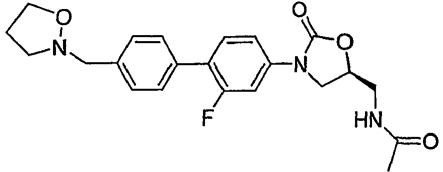 |
| | (5S)N-[3-(2-Fluoro-4'-isoxazolidin-2-ylmethyl-biphenyl-4-yl)-2-oxo-oxazolidin-5-ylmethyl]-acetamide |
| **56** | 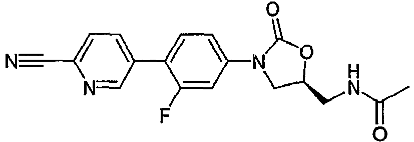 |
| | (5S)N-{3-[4-(6-Cyano-pyridin-3-yl)-3-fluoro-phenyl]-2-oxo-oxazolidin-5-ylmethyl}-acetamide |
| **57** | 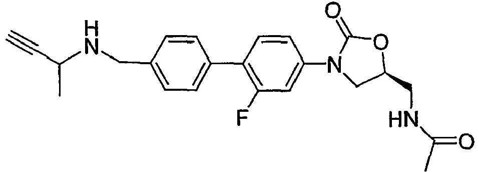 |
| | (5S)N-(3-{2-Fluoro-4'-[(1-methyl-prop-2-ynylamino)-methyl]-biphenyl-4-yl}-2-oxo-oxazolidin-5-ylmethyl)-acetamide |
| **58** | 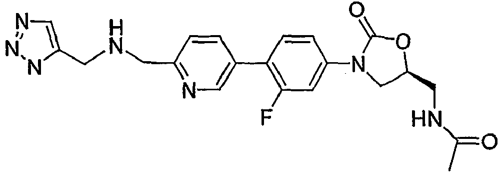 |
| | (5S)N-{3-[3-Fluoro-4-(6-{[(3H-[1,2,3]triazol-4-ylmethyl)-amino]-methyl}-pyridin-3-yl)-phenyl]-2-oxo-oxazolidin-5-ylmethyl}-acetamide |
| **59** | 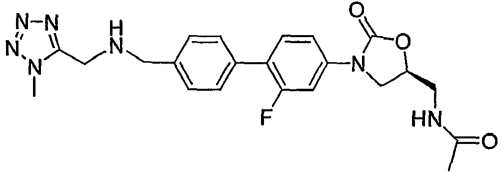 |
| | (5S)N-[3-(2-Fluoro-4'-{[(1-methyl-1H-tetrazol-5-ylmethyl)-amino]-methyl}-biphenyl-4-yl)-2-oxo-oxazolidin-5-ylmethyl]-acetamide |
| **60** | 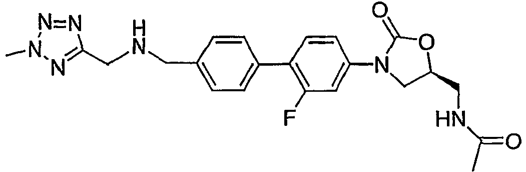 |
| | (5S)N-[3-(2-Fluoro-4'-{[(2-methyl-2H-tetrazol-5-ylmethyl)-amino]-methyl}-biphenyl-4-yl)-2-oxo-oxazolidin-5-ylmethyl]-acetamide |
| **61** | 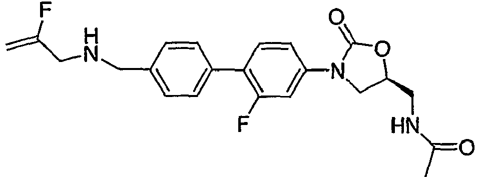 |
| | (5S)N-(3-{2-Fluoro-4'-[(2-fluoro-allylamino)-methyl]-biphenyl-4-yl}-2-oxo-oxazolidin-5-ylmethyl)-acetamide |
| **62** | 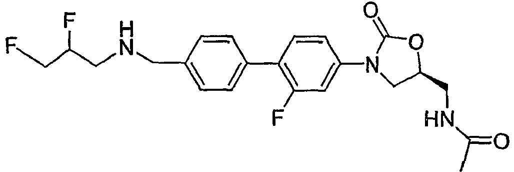 |
| | (5S)N-(3-{4'-[(2,3Difluoropropylamino)-methyl]-2-fluoro-biphenyl-4-yl}-2-oxo-oxazolidin-5-ylmethyl)-acetamide |
| **63** | 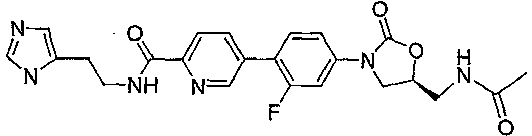 |
| | (5S)5-{4-[5-(Acetylamino-methyl)-2-oxo-oxazolidin-3-yl]-2-fluorophenyl}-pyridine-2-carboxylic acid [2-(3H-imidazol-4-yl)-ethyl]-amide |
| **64** | 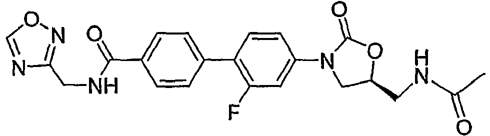 |
| | (5S)4'-[5-(Acetylamino-methyl)-2-oxo-oxazolidin-3-yl]-2'-fluorobiphenyl-4-carboxylic acid ([1,2,4]oxadiazol-3-ylmethyl)-amide |
| **65** | 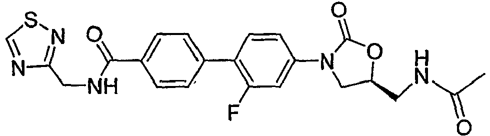 |
| | (5S)4'-[5-(Acetylamino-methyl)-2-oxo-oxazolidin-3-yl]-2'-fluorobiphenyl-4-carboxylic acid ([1,2,4]thiadiazol-3-ylmethyl)-amide |
| **66** | 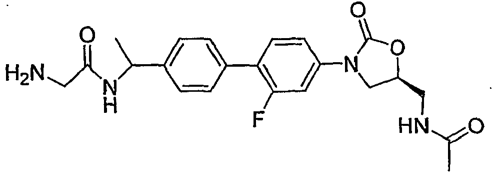 |
| | (5S)N-(1-{4'-[5-(Acetylamino-methyl)-2-oxo-oxazolidin-3-yl]-2'-fluoro-biphenyl-4-yl}-ethyl)-2-amino-acetamide |
| **67** | 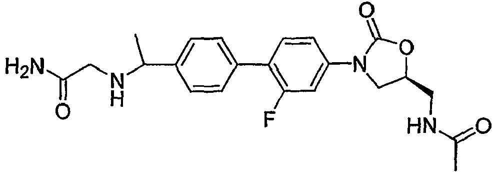 |
| | (5S)2-(1-{4'-[5-(Acetylamino-methyl)-2-oxo-oxazolidin-3-yl]-2'-fluorobiphenyl-4-yl}-ethylamino)-acetamide |
| **68** | 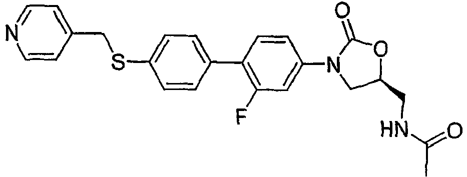 |
| | (5S)N-{3-[2-Fluoro-4'-(pyridin-4-ylmethylsulfanyl)-biphenyl-4-yl]-2-oxo-oxazolidin-5-ylmethyl}-acetamide |
| **69** | 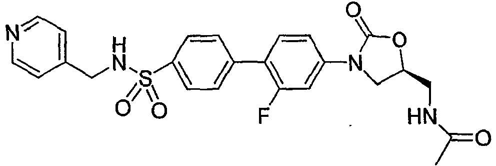 |
| | (5S)N-(3-{2-Fluoro-4'-[(pyridin-4-ylmethyl)-sulfamoyl]-biphenyl-4-yl}-2-oxo-oxazolidin-5-ylmethyl)-acetamide |
| **70** | 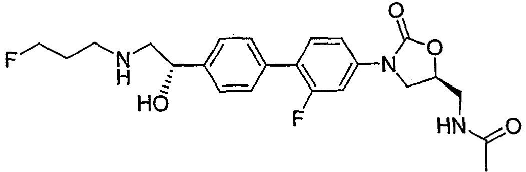 |
| | (S,S)N-(3-{2-Fluoro-4'-[2-(3-fluoro-propylamino)-1-hydroxy-ethyl]-biphenyl-4-yl}-2-oxo-oxazolidin-5-ylmethyl)-acetamide |
| **71** | 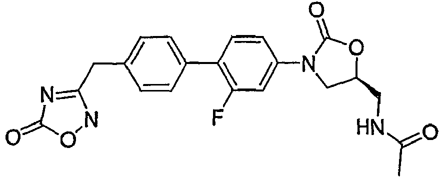 |
| | (5S)N-{3-[2-Fluoro-4'-(5-oxo-2,5-dihydro-[1,2,4]oxadiazol-3-ylmethyl)-biphenyl-4-yl]-2-oxo-oxazolidin-5-ylmethyl}-acetamide |
| **72** | 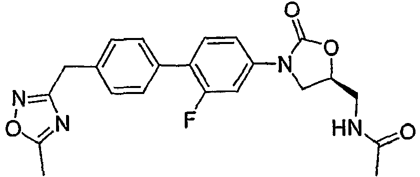 |
| | (5S)N-{3-[2-Fluoro-4'-(5-methyl-[1,2,4]oxadiazol-3-ylmethyl)-biphenyl-4-yl]-2-oxo-oxazolidin-5-ylmethyl}-acetamide |
| **73** | 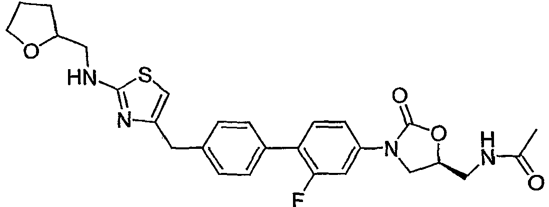 |
| | (5S)N-[3-(2-Fluoro-4'-{2-[(tetrahydro-furan-2-ylmethyl)-amino]-thiazol-4-ylmethyl}-biphenyl-4-yl)-2-oxo-oxazolidin-5-ylmethyl]-acetamide |
| **74** | 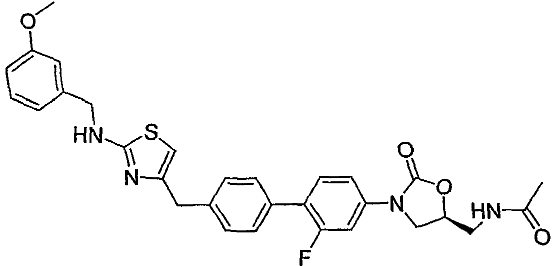 |
| | (5S)N-(3-{2-Fluoro-4'-[2-(3-methoxy-benzylamino)-thiazol-4-ylmethyl]-biphenyl-4-yl}-2-oxo-oxazolidin-5-ylmethyl)-acetamide |
| **75** | 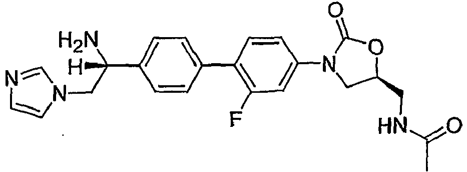 |
| | (S,S)N-{3-[4'-(1-Amino-2-imidazol-1-yl-ethyl)-2-fluoro-biphenyl-4-yl]-2-oxo-oxazolidin-5-ylmethyl}-acetamide |
| **76** | 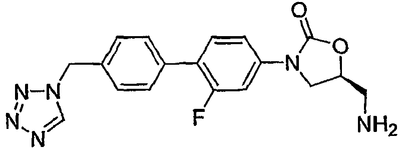 |
| | (5S)5-Aminomethyl-3-(2-fluoro-4'-tetrazol-1-ylmethyl-biphenyl-4-yl)-oxazolidin-2-one |
| **77** | 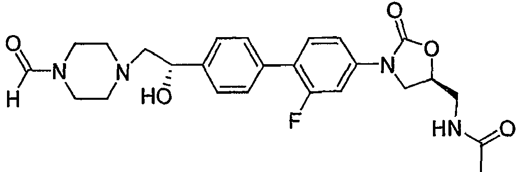 |
| | (S,S)N-(3-{2-Fluoro-4'-[2-(4-formyl-piperazin-1-yl)-1-hydroxy-ethyl]-biphenyl-4-yl}-2-oxo-oxazolidin-5-ylmethyl)-acetamide |
| **78** | 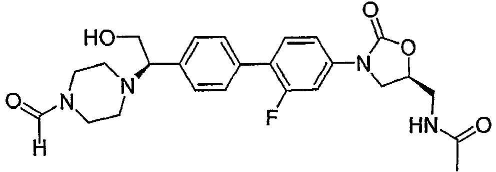 |
| | (R,S)N-(3-{2-Fluoro-4'-[1-(4-formyl-piperazin-1-yl)-2-hydroxy-ethyl]-biphenyl-4-yl}-2-oxo-oxazolidin-5-ylmethyl)-acetamide |
| **79** | 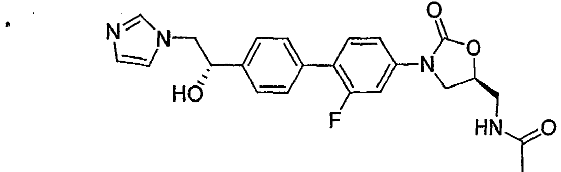 |
| | (S,S)N-{3-[2-Fluoro-4'-(1-hydroxy-2-imidazol-1-yl-ethyl)-biphenyl-4-yl]-2-oxo-oxazolidin-5-ylmethyl]-acetamide |
| **80** | 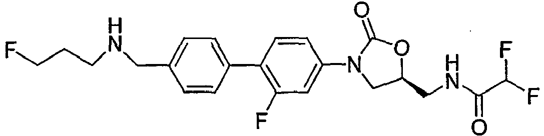 |
| | (5S)2,2-Difluoro-N-(3-{2-fluoro-4'-[(3-fluoro-propylamino)-methyl]-biphenyl-4-yl}-2-oxo-oxazolidin-5-ylmethyl)-acetamide |
| **81** | 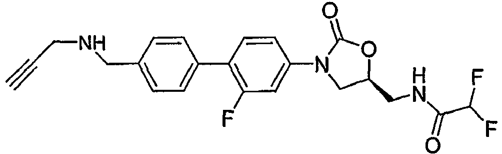 |
| | (5S)2,2-Difluoro-N-[3-(2-fluoro-4'-prop-2-ynylaminomethyl-biphenyl-4-yl)-2-oxo-oxazolidin-5-ylmethyl]-acetamide |

The exemplary compounds in Table 1 can be synthesized by the process depicted in Scheme A using, for example, the aryl boronic acids and aryl iodides listed in Table 2, below.

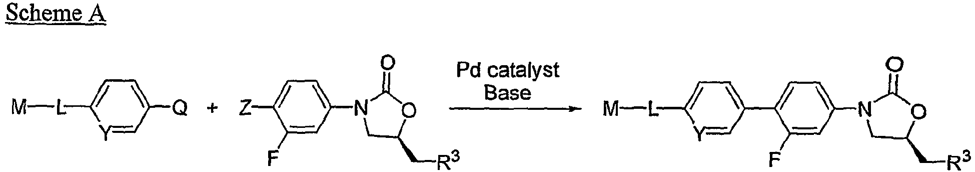

**Table 2**

| **M** | **L** | **Y** | **Q** | **Z** | **R³** | **Product** |
|---|---|---|---|---|---|---|
| 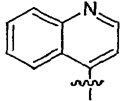 | -CH₂NHCH₂- * | CH | B(OH)₂ | I | -NHAc | **1** |
| 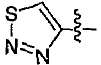 | -CH₂NHCH₂- * | CH | B(OH)₂ | I | -NHAc | **2** |
| 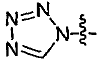 | -CH₂- | CH | B(OH)₂ | I | -NHAc | **3** |
| 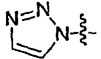 | -CH₂- | CH | B(OH)₂ | I | -NHAc | **4** |
| 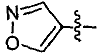 | -CH₂NHCH₂- * | CH | B(OH)₂ | I | -NHAc | **5** |
| 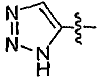 | -CH₂NHCH₂- * | CH | B(OH)₂ | I | -NHAc | **6** |
| 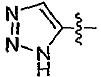 | -CH₂NHCH₂- * | CH | B(OH)₂ | I | 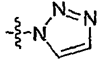 | **7** |
| 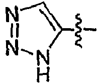 | -CH₂N(CH₃)CH₂- | CH | B(OH)₂ | I | -NHAc | **8** |
| 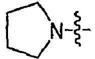 | -CH₂- | CH | B(OH)₂ | I | -NHAc | **9** |
| 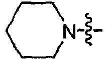 | -CH₂- | CH | B(OH)₂ | I | -NHAc | **10** |
| FCH₂CH₂CH₂- | -NHCH₂- * | CH | B(OH)₂ | I | -NHAc | **11** |
| NCCH₂CH₂- | -NHCH₂- * | CH | B(OH)₂ | I | -NHAc | **12** |
| 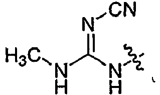 | -CH₂- | CH | B(OH)₂ | I | -NHAc | **13** |
| H₂NC(O)CH₂- | -NHCH₂- * | CH | B(OH)₂ | I | 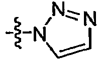 | **14** |
| FCH₂CH(OH)CH₂- | -NHCH₂- * | CH | B(OH)₂ | I | -NHAc | **15** |
| FCH₂CH₂CH₂- | -NHCH₂- * | CH | B(OH)₂ | I | 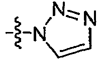 | **16** |
| CH₃CH₂- | -SO₂NHCH₂- * | CH | B(OH)₂ | I | -NHAc | **17** |
| CH₃- | -NHSO₂CH₂- * | CH | B(OH)₂ | I | -NHAc | **18** |
| 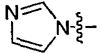 | -(CH₂)₃NHC(O)NH-* | CH | B(OH)₂ | I | -NHAc | **19** |
| 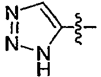 | -SCH₂CH₂NHCH₂- * | CH | B(OH)₂ | I | -NHAc | **20** |
| 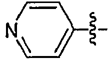 | -CH₂SO₂- | CH | B(OH)₂ | I | -NHAc | **21** |
| 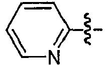 | -CH₂NHSO₂- * | CH | B(OH)₂ | I | -NHAc | **22** |
| 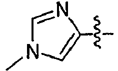 | -SO₂NHCH₂- * | CH | B(OH)₂ | I | -NHAc | **23** |
| 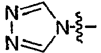 | -NHCH₂- * | CH | B(OH)₂ | I | -NHAc | **24** |
| 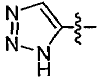 | -S(O)CH₂- | CH | B(OH)₂ | I | -NHAc | **25** |
| H₂NC(O)CH₂- | -NHCH₂- * | CH | B(OH)₂ | I | -NHAc | **26** |
| H₂NCH₂C(O)- | -NHCH₂- * | CH | B(OH)₂ | I | -NHAc | **27** |
| CH₃- | -SO₂N-HCH₂- * | CH | B(OH)₂ | I | -NHAc | **28** |
| CH₃- | -SO₂NHCH₂- * | CF | B(OH)₂ | I | -NHAc | **29** |
| 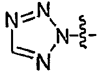 | -CH₂- | CH | B(OH)₂ | I | -NHAc | **30** |
| 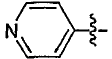 | -CH₂S(O)CH₂- | CH | B(OH)₂ | I | -NHAc | **31** |
| 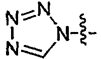 | -CH₂- | N | B(OH)₂ | I | -NHAc | **32** |
| 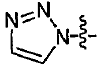 | -CH₂- | N | B(OH)₂ | I | -NHAc | **33** |
| 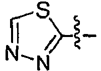 | -S(O)CH₂- | CH | B(OH)₂ | I | -NHAc | **34** |
| 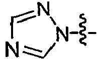 | -CH₂- | CH | B(OH)₂ | I | -NHAc | **35** |
| 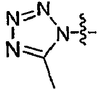 | -CH₂- | CH | B(OH)₂ | I | -NHAc | **36** |
| 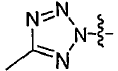 | -CH₂- | CH | B(OH)₂ | I | -NHAc | **37** |
| 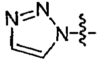 | -CH₂CH(OH)- | CH | B(OH)₂ | I | -NHAc | **38** |
| CH₃- | -SCH₂CH₂NHCH₂- * | CH | B(OH)₂ | I | -NHAc | **39** |
| 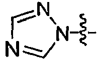 | -CH₂- | N | B(OH)₂ | I | -NHAc | **40** |
| NCCH₂- | 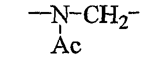 | CH | B(OH)₂ | I | -NHAc | **41** |
| 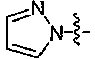 | -CH₂- | N | B(OH)₂ | I | NHAc | **42** |
| 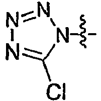 | -CH₂- | CH | B(OH)₂ | I | -NHAc | **43** |
| 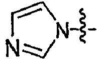 | -CH₂- | CH | B(OH)₂ | I | 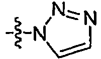 | **44** |
| HC≡CCH₂- | NHCH₂- * | CH | B(OH)₂ | I | -NHAc | **45** |
| H₂C=CHCH₂- | -NHCH₂- * | CH | B(OH)₂ | I | -NHAc | **46** |
| H₂C=CHCH₂CH₂- | -NHCH₂- * | CH | B(OH)₂ | I | -NHAc | **47** |
| HC≡CCH₂CH₂- | -NHCH₂- * | CH | B(OH)₂ | I | -NHAc | **48** |
| HC≡C(CH₂)₃- | -NHCH₂- * | CH | B(OH)₂ | I | -NHAc | **49** |
| CH₃C≡CCH₂- | -NHCH₂- * | CH | B(OH)₂ | I | -NHAc | **50** |
| 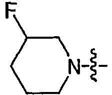 | -CH₂- | CH | B(OH)₂ | I | -NHAc | **51** |
| 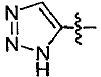 | -(CH₂)₃NHCH₂- * | CH | B(OH)₂ | I | -NHAc | **52** |
| F₂HCCH₂- | -NHCH₂- * | CH | B(OH)₂ | I | -NHAc | **53** |
| HC≡CCH₂- | -NHCH₂- * | CH | B(OH)₂ | I | 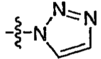 | **54** |
| 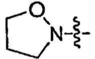 | -CH₂- | CH | B(OH)₂ | I | -NHAc | **55** |
| NC- | (bond) | N | B(OH)₂ | I | -NHAc | **56** |
| 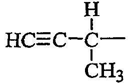 | -NHCH₂- * | CH | B(OH)₂ | I | -NHAc | **57** |
| 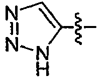 | -CH₂NHCH₂- * | N | B(OH)₂ | I | -NHAc | **58** |
| 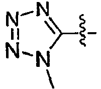 | -CH₂NHCH₂- * | CH | B(OH)₂ | I | -NHAc | **59** |
| 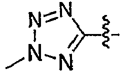 | -CH₂NHCH₂- * | CH | B(OH)₂ | I | -NHAc | **60** |
| HC=CFCH₂- | -NHCH₂- * | CH | B(OH)₂ | I | -NHAc | **61** |
| FH₂CCHFCH₂- | -NHCH₂- * | CH | B(OH)₂ | I | NHAc | **62** |
| 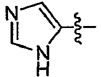 | -CH₂CH₂NHC(O)- * | N | B(OH)₂ | I | NHAc | **63** |
| 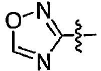 | -CH₂NHC(O)- * | CH | B(OH)₂ | I | -NHAc | **64** |
| 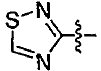 | -CH₂NHC(O)- * | CH | B(OH)₂ | I | -NHAc | **65** |
| H₂NCH₂C(O)- | 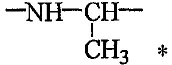 | CH | B(OH)₂ | I | -NHAc | **66** |
| H₂NC(O)CH₂- | 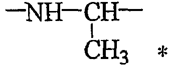 | CH | B(OH)₂ | I | -NHAc | **67** |
| 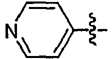 | -CH₂S- | CH | B(OH)₂ | I | -NHAc | **68** |
| 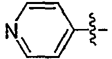 | -CH₂NHSO₂- * | CH | B(OH)₂ | I | -NHAc | **69** |
| FCH₂CH₂CH₂- | -NHCH₂CH(OH)- * | CH | B(OH)₂ | I | -NHAc | **70** |
| 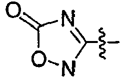 | -CH₂- | CH | B(OH)₂ | I | -NHAc | **71** |
| 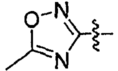 | -CH₂- | CH | B(OH)₂ | I | -NHAc | **72** |
| 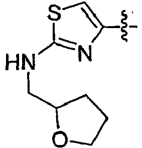 | -CH₂- | CH | B(OH)₂ | I | -NHAc | **73** |
| 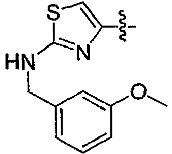 | -CH₂- | CH | B(OH)₂ | I | -NHAc | **74** |
| 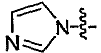 | 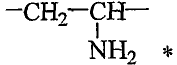 | CH | B(OH)₂ | I | -NHAc | **75** |
| 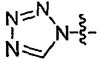 | -CH₂- | CH | B(OH)₂ | I | -NH₂ | **76** |
| 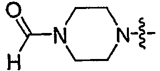 | -CH₂CH(OH)- | CH | B(OH)₂ | I | -NHAc | **77** |
| 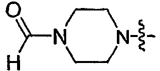 | 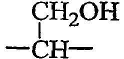 | CH | B(OH)₂ | I | -NHAc | **78** |
| 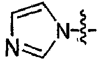 | -CH₂CH(OH)- | CH | B(OH)₂ | I | -NHAc | **79** |
| FCH₂CH₂CH₂- | -NHCH₂- * | CH | B(OH)₂ | I | -NHC(O)CHF₂ | **80** |
| HC≡CCH₂- | -NHCH₂- * | CH | B(OH)₂ | I | -NHC(O)CHF₂ | **81** |

Compounds containing L groups having free amines (e.g., compounds derived from the reagents having L groups marked with * in Table 2) alternatively may be synthesized from the corresponding protected amine (e.g., -CH₂N(BOC)CH₂- or -N(BOC)CH₂-) followed by amine deprotection.

Alternatively, the exemplary compounds in Table 1 can be synthesized by the process depicted in Scheme B using, for example, the aryl boronic acids and aryl iodides listed in Table 3, below.

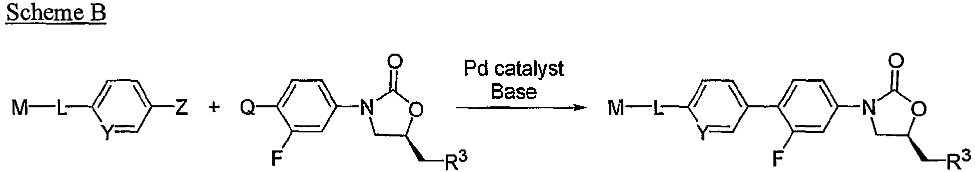

**Table 3**

| **M** | **L** | **Y** | **Z** | **Q** | **R³** | **Product** |
|---|---|---|---|---|---|---|
| 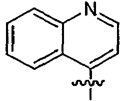 | -CH₂NHCH₂- * | CH | I | B(OH)₂ | -NHAc | **1** |
| 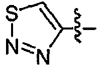 | -CH₂NHCH₂- * | CH | I | B(OH)₂ | -NHAc | **2** |
| 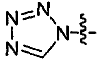 | -CH₂- | CH | I | B(OH)₂ | -NHAc | **3** |
| 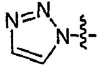 | -CH₂- | CH | I | B(OH)₂ | -NHAc | **4** |
| 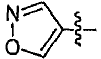 | -CH₂NHCH₂- * | CH | I | B(OH)₂ | -NHAc | **5** |
| 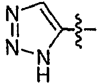 | -CH₂NHCH₂- * | CH | I | B(OH)₂ | -NHAc | **6** |
| 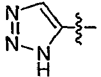 | -CH₂NHCH₂- * | CH | I | B(OH)₂ | 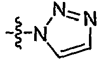 | **7** |
| 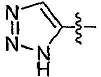 | -CH₂N(CH₃)CH₂- | CH | I | B(OH)₂ | -NHAc | **8** |
| 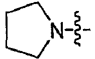 | -CH₂- | CH | I | B(OH)₂ | -NHAc | **9** |
| 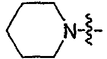 | -CH₂- | CH | I | B(OH)₂ | -NHAc | **10** |
| FCH₂CH₂CH₂- | -NHCH₂- * | CH | I | B(OH)₂ | -NHAc | **11** |
| NCCH₂CH₂- | -NHCH₂- * | CH | I | B(OH)₂ | -NHAc | **12** |
| 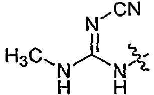 | -CH₂- | CH | I | B(OH)₂ | -NHAc | **13** |
| H₂NC(O)CH₂- | -NHCH₂- * | CH | I | B(OH)₂ | 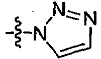 | **14** |
| FCH₂CH(OH)CH₂- | -NHCH₂- * | CH | I | B(OH)₂ | -NHAc | **15** |
| FCH₂CH₂CH₂- | -NHCH₂- * | CH | I | B(OH)₂ | 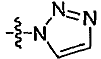 | **16** |
| CH₃CH₂- | -SO₂NHCH₂- * | CH | I | B(OH)₂ | -NHAc | **17** |
| CH₃- | -NHSO₂CH₂- | CH | I | B(OH)₂ | -NHAc | **18** |
| 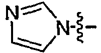 | -(CH₂)₃NHC(O)NH- * | CH | I | B(OH)₂ | -NHAc | **19** |
| 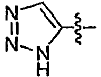 | -SCH₂CH₂NHCH₂- * | CH | I | B(OH)₂ | -NHAc | **20** |
| 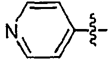 | -CH₂SO₂- | CH | I | B(OH)₂ | -NHAc | **21** |
| 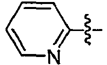 | -CH₂NHSO₂- * | CH | I | B(OH)₂ | -NHAc | **22** |
| 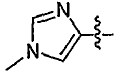 | -SO₂NHCH₂- * | CH | I | B(OH)₂ | -NHAc | **23** |
| 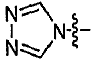 | -NHCH₂- * | CH | I | B(OH)₂ | -NHAc | **24** |
| 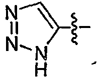 | -S(O)CH₂- | CH | I | B(OH)₂ | -NHAc | **25** |
| H₂NC(O)CH₂- | -NHCH₂- * | CH | I | B(OH)₂ | -NHAc | **26** |
| H₂NCH₂C(O)- | -NHCH₂- * | CH | I | B(OH)₂ | -NHAc | **27** |
| CH₃- | -SO₂NHCH₂- * | CH | I | B(OH)₂ | -NHAc | **28** |
| CH₃- | -SO₂NHCH₂- * | CF | I | B(OH)₂ | -NHAc | **29** |
| 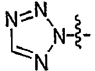 | -CH₂- | CH | I | B(OH)₂ | NHAc | **30** |
| 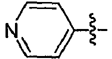 | -CH₂S(O)CH₂- | CH | I | B(OH)₂ | -NHAc | **31** |
| 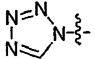 | -CH₂- | N | I | B(OH)₂ | -NHAc | **32** |
| 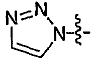 | -CH₂- | N | I | B(OH)₂ | -NHAc | **33** |
| 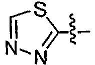 | -S(O)CH₂- | CH | I | B(OH)₂ | -NHAc | **34** |
| 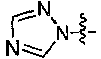 | -CH₂- | CH | I | B(OH)₂ | -NHAc | **35** |
| 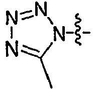 | -CH₂- | CH | I | B(OH)₂ | -NHAc | **36** |
| 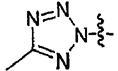 | -CH₂- | CH | I | B(OH)₂ | -NHAc | **37** |
| 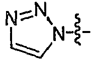 | -CH₂CH(OH)- | CH | I | B(OH)₂ | -NHAc | **38** |
| CH₃- | -SCH₂CH₂NHCH₂- * | CH | I | B(OH)₂ | -NHAc | **39** |
| 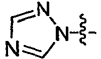 | -CH₂- | N | I | B(OH)₂ | -NHAc | **40** |
| NCCH₂- | 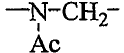 | CH | I | B(OH)₂ | -NHAc | **41** |
| 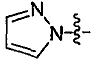 | -CH₂- | N | I | B(OH)₂ | -NHAc | **42** |
| 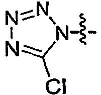 | -CH₂- | CH | I | B(OH)₂ | -NHAc | **43** |
| 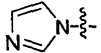 | -CH₂- | CH | I | B(OH)₂ | 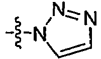 | **44** |
| HC≡CCH₂- | -NHCH₂- * | CH | I | B(OH)₂ | -NHAc | **45** |
| H₂C=CHCH₂- | -NHCH₂- * | CH | I | B(OH)₂ | -NHAc | **46** |
| H₂C=CHCH₂CH₂- | -NHCH₂- * | CH | I | B(OH)₂ | -NHAc | **47** |
| HC≡-CCH₂CH₂- | -NHCH₂- * | CH | I | B(OH)₂ | -NHAc | **48** |
| HC≡C(CH₂)₃- | -NHCH₂- * | CH | I | B(OH)₂ | -NHAc | **49** |
| CH₃C≡CCH₂- | -NHCH₂- * | CH | I | B(OH)₂ | -NHAc | **50** |
| 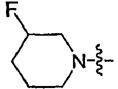 | -CH₂- | CH | I | B(OH)₂ | -NHAc | **51** |
| 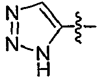 | -(CH₂)₃NHCH₂- * | CH | I | B(OH)₂ | -NHAc | **52** |
| F₂HCCH₂- | -NHCH₂- * | CH | I | B(OH)₂ | -NHAc | **53** |
| HC≡CCH₂- | -NHCH₂- * | CH | I | B(OH)₂ | 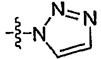 | **54** |
| 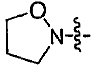 | -CH₂- | CH | I | B(OH)₂ | -NHAc | **55** |
| NC- | (bond) | N | I | B(OH)₂ | -NHAc | **56** |
| 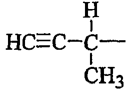 | -NHCH₂- * | CH | I | B(OH)₂ | -NHAc | **57** |
| 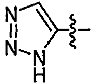 | -CH₂NHCH₂- * | N | I | B(OH)₂ | -NHAc | **58** |
| 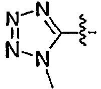 | -CH₂NHCH₂- * | CH | I | B(OH)₂ | -NHAc | **59** |
| 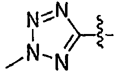 | -CH₂NHCH₂- * | CH | I | B(OH)₂ | -NHAc | **60** |
| HC=CFCH₂- | -NHCH₂- * | CH | I | B(OH)₂ | -NHAc | **61** |
| FH₂CCHFCH₂- | -NHCH₂- * | CH | I | B(OH)₂ | -NHAc | **62** |
| 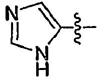 | -CH₂CH₂NHC(O)- * | N | I | B(OH)₂ | NHAc | **63** |
| 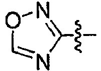 | -CH₂NHC(O)- * | CH | I | B(OH)₂ | -NHAc | **64** |
| 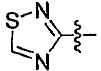 | -CH₂NHC(O)- * | CH | I | B(OH)₂ | -NHAc | **65** |
| H₂NCH₂C(O)- | 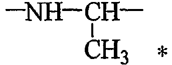 | CH | I | B(OH)₂ | -NHAc | **66** |
| H₂NC(O)CH₂- | 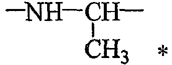 | CH | I | B(OH)₂ | -NHAc | **67** |
| 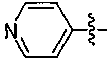 | -CH₂S- | CH | I | B(OH)₂ | -NHAc | **68** |
| 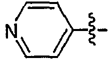 | -CH₂NHSO₂- * | CH | I | B(OH)₂ | -NHAc | **69** |
| FCH₂CH₂CH₂- | -NHCH₂CH(OH)- * | CH | I | B(OH)₂ | -NHAc | **70** |
| 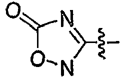 | -CH₂- | CH | I | B(OH)₂ | -NHAc | **71** |
| 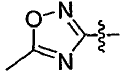 | -CH₂- | CH | I | B(OH)₂ | -NHAc | **72** |
| 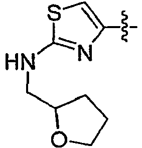 | -CH₂- | CH | I | B(OH)₂ | -NHAc | **73** |
| 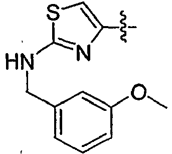 | -CH₂- | CH | I | B(OH)₂ | -NHAc | **74** |
| 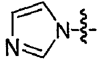 | 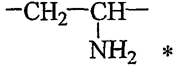 | CH | I | B(OH)₂ | -NHAc | **75** |
| 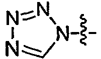 | -CH₂- | CH | I | B(OH)₂ | -NH₂ | **76** |
| 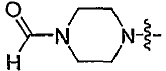 | -CH₂CH(OH)- | CH | I | B(OH)₂ | -NHAc | **77** |
| 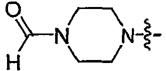 | 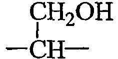 | CH | I | B(OH)₂ | -NHAc | **78** |
| 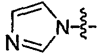 | -CH₂CH(OH)- | CH | I | B(OH)₂ | -NHAc | **79** |
| FCH₂CH₂CH₂- | -NHCH₂- * | CH | I | B(OH)₂ | -NHC(O)CHF₂ | **80** |
| HC≡CCH₂- | -NHCH₂- * | CH | I | B(OH)₂ | -NHC(O)CHF₂ | **81** |

As discussed above, compounds containing L groups having free amines (e.g., compounds derived from the reagents having L groups marked with * in Table 3) alternatively may be synthesized from the corresponding protected amine (e.g., -CH₂N(BOC)CH₂- or -N(BOC)CH₂-) followed by amine deprotection.

In addition to the reagents listed in Tables 2 and 3, reagents containing other Q groups (e.g., boronic esters, boronic halides, or organoboranes) and/or reagents containing other Z groups (e.g., other halogens or sulfonates) may be used to synthesize the exemplary compounds in Table 1 according to the processes of the invention.

### 3. Examples

Embodiments of the present disclosure are described in the following examples.

Nuclear magnetic resonance (NMR) spectra were obtained on a Bruker Avance 300 or Avance 500 spectrometer, or in some cases a GE-Nicolet 300 spectrometer. Common reaction solvents were either high performance liquid chromatography (HPLC) grade or American Chemical Society (ACS) grade, and anhydrous as obtained from the manufacturer unless otherwise noted. "Chromatography" or "purified by silica gel" refers to flash column chromatography using silica gel (EM Merck, Silica Gel 60, 230-400 mesh) unless otherwise noted.

### Example 1

Scheme 1 depicts the synthesis of compound **11** from aryl iodide **108** and aryl boronic acid **120.**

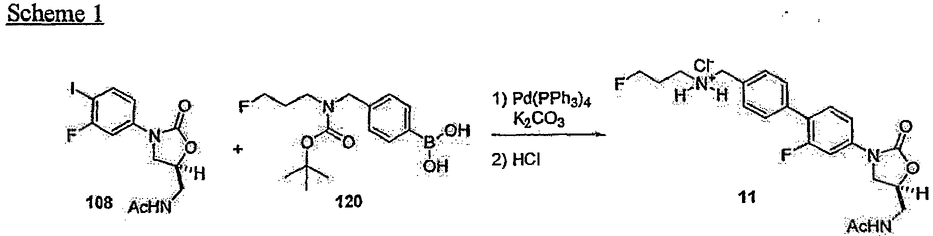

### a. Synthesis of aryl iodide 108 - Method A

Scheme 2 depicts the synthesis of aryl iodide **108** from 3-fluoroanaline **101**.

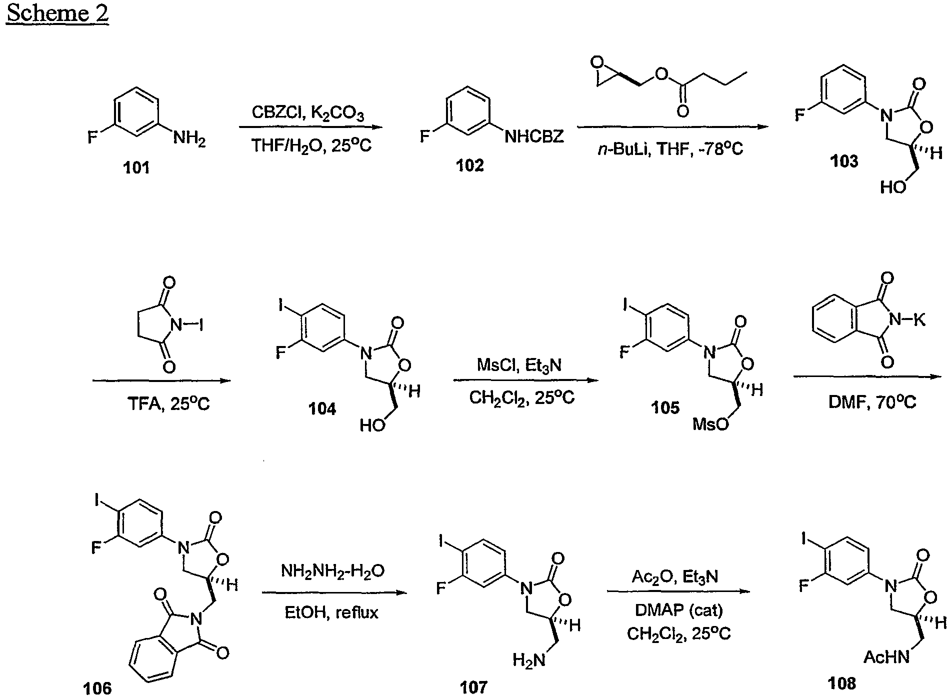

A solution of 3-fluoroanaline **101** (18.7 g, 168.3 mmol) in tetrahydrofuran (THF, 150 mL) was treated with potassium carbonate (K₂CO₃, 46.45 g, 336.6 mmol, 2.0 equiv) and H₂O (150 mL) before a solution of benzyl chloroformate (CBZCl, 31.58 g, 185.1 mmol, 26.1 mL, 1.1 equiv) in THF (50 mL) was dropwise added into the reaction mixture at room temperature under N₂. The resulting reaction mixture was stirred at room temperature for 2 h. When TLC showed the reaction was complete, the reaction mixture was treated with H₂O (100 mL) and ethyl acetate (EtOAc, 100 mL). The two layers were separated, and the aqueous layer was extracted with EtOAc (2 x 100 mL). The combined organic extracts were washed with H₂O (2 x100 mL) and saturated aqueous sodium chloride (NaCl, 100 mL), dried over magnesium sulfate (MgSO₄), and concentrated *in vacuo*. The residue was further dried *in vacuo* to afford the desired (3-fluorophenyl)-carbamic acid benzyl ester **102** (39.2 g, 95% yield) as pale-yellow oil. This product was directly used in subsequent reactions without further purification. ¹H NMR (300 MHz, CDCl₃) δ 5.23 (s, 2H, OC*H*₂Ph), 6.75-6.82 (m, 2H), 7.05 (dd, 1H, *J*= 1.4, 8.2 Hz), 7.22-7.45 (m, 6H). C₁₄H₁₂FNO₂, LCMS (EI) *m*/*e* 246 (M⁺ + H).

A solution of amine **102** (39.2 g, 160.0 mmol) in anhydrous THF (300 mL) was cooled to -78 °C in a dry-ice/acetone bath before a solution of *n*-butyl lithium (*n*-BuLi, 2.5 M solution in hexane, 70.4 mL, 176 mmol, 1.1 equiv) was dropwise added under N₂. The resulting reaction mixture was subsequently stirred at -78 °C for 1 h before a solution of (R)-(-)-glycidyl butyrate (25.37 g, 24.6 mL, 176 mmol, 1.1 equiv) in anhydrous THF (100 mL) was dropwise added into the reaction mixture at -78 °C under N₂. The resulting reaction mixture was stirred at -78 °C for 30 min before being gradually warmed to room temperature for 12 h under N₂. When TLC and HPLC/MS showed the reaction was complete, the reaction mixture was quenched with H₂O (200 mL), and the resulting mixture was stirred at room temperature for 1 h before EtOAc (200 mL) was added. The two layers were separated, and the aqueous layer was extracted with EtOAc (2 x 100 mL). The combined organic extracts were washed with H₂O (2 x 100 mL) and saturated aqueous NaCl (100 mL), dried over MgSO₄, and concentrated *in vacuo.* White crystals precipitated from the concentrated solution when most of the solvent was evaporated. The residue was then treated with 20% EtOAc/hexane (100 mL) and the resulting slurry was stirred at room temperature for 30 min. The solids were collected by filtration and washed with 20% EtOAc/hexane (2 x 50 mL) to afford the desired (5*R*)-(3-(3-fluoro-phenyl)-5-hydroxymethyloxazolidin-2-one **103** (24.4 g, 72.3% yield) as white crystals. This product was directly used in subsequent reactions without further purification. ¹H NMR (300 MHz, DMSO-*d*₆) δ 3.34-3.72 (m, 2H), 3.83 (dd, 1H, *J*= 6.2,9.0 Hz), 4.09 (t, 1H, *J* = 12.0 Hz), 4.68-4.75 (m, 1H), 5.23 (t, 1H, J = 5.6 Hz, O*H*), 6.96 (m, 1H), 7.32-7.56 (m, 3H). C₁₀H₁₀FNO₃, LCMS (EI) *m*/*e* 212 (M⁺ + H).

A solution of alcohol **103** (10.74 g, 50.9 mmol) in trifluoroacetic acid (TFA, 50 mL) was treated with *N*-iodosuccinimide (12.03 g, 53.45 mmol, 1.05 equiv) at 25 °C and stirred for 2 h. When TLC and HPLC/MS showed the reaction was complete, the reaction mixture was concentrated *in vacuo.* The residue was then treated with H₂O (100 mL) and 20% EtOAc/hexane (100 mL) at 25 °C, and the resulting mixture was stirred at 25 °C for 30 min before being cooled to 0-5 °C for 2 h. The white solids were collected by filtration, washed with H₂O (2 x 25 mL) and 20% EtOAc/hexane (2 x 25 mL), and dried *in vacuo* to afford the desired (5*R*)-3-(3-fluoro-4-iodo-phenyl)-5-hydroxymethyl-oxazolidin-2-one **104** (15.1 g, 88% yield) as an off-white powder. This product was directly used in subsequent reactions without further purification. ¹H NMR (300 MHz, DMSO-*d₆*) δ 3.58 (dd, 1H, *J*= 4.2, 12.6 Hz), 3.67 (dd, 1H, *J*= 3.0,12.6 Hz), 3.67 (dd, 1H, *J*= 6.3, 9.0 Hz), 4.07 (t, 1H, *J* = 9.0 Hz), 4.72 (m, 1H), 5.21 (br. s, 1H, O*H*), 7.22 (dd, 1H, *J* = 2.4, 8.4 Hz), 7.58 (dd, 1H, *J* = 2.4,11.1 Hz), 7.81 (dd, 1H, *J* = 7.8, 8.7 Hz). C₁₀H₉FINO₃, LCMS (EI) *m*/*e* 338 (M⁺ + H).

A solution of iodo-alcohol **104** (25.2 g, 74.8 mmol) in methylene chloride (CH₂Cl₂, 150 mL) was treated with triethylamine (TEA, 15.15 g, 20.9 mL, 150 mmol, 2.0 equiv) at 25 °C, and the resulting mixture was cooled to 0-5 °C before methanesulfonyl chloride (MsCl, 10.28 g, 6.95 mL, 89.7 mmol, 1.2 equiv) was dropwise introduced into the reaction mixture at 0-5 °C under N₂. The resulting reaction mixture was subsequently stirred at 0-5 °C for 1 h under N₂. When TLC and HPLC/MS showed the reaction was complete, the reaction mixture was quenched with H₂O (100 mL) and CH₂Cl₂ (100 mL). The two layers were separated, and the aqueous layer was extracted with CH₂Cl₂ (100 mL). The combined organic extracts were washed with H₂O (2 x 100 mL) and saturated aqueous NaCl (100 mL), dried over MgSO₄, and concentrated *in vacuo.* The residue was further dried *in vacuo* to afford the desired (5*R*)-methanesulfonic acid 3-(3-fluoro-4-iodo-phenyl)-2-oxo-oxazolidin-5-ylmethyl ester **105** (30.71 g, 98.9% yield) as an off-white powder. This product was directly used in subsequent reactions without further purification. C₁₁H₁₁FINO₅S, LCMS (EI) *m*/*e* 416 (M⁺ + H).

A solution of mesylate **105** (26.38 g, 63.57 mmol) in anhydrous *N,N*-dimethylformamide (DMF, 120 mL) was treated with solid potassium phthalimide (12.95 g, 70.0 mmol, 1.1 equiv) at 25 °C, and the resulting reaction mixture was warmed to 70 °C for 2 h. When TLC and HPLC showed the reaction was complete, the reaction mixture was cooled to room temperature before being quenched with H₂O (400 mL). The resulting mixture was stirred at room temperature for 10 min before being cooled to 0-5 °C for 1 h. The white precipitate was collected by filtration, washed with water (3 x 100 mL), and dried *in vacuo* to afford the desired (5*R*)-2-[3-(3-fluoro-4-iodo-phenyl)-2-oxo-oxazolidin-5-ylmethyl]-isoindole-1,3-dione **106** (27.85 g, 94%) as an off-white powder. This product was directly used in subsequent reactions without further purification. C₁₈H₁₂FIN₂O₄, LCMS (EI) *m*/*e* 467 (M⁺ + H).

A solution of phthalimide **106** (23.3 g, 50.0 mmol) in ethanol (EtOH,150 mL) was treated with hydrazine monohydrate (12.52 g, 12.1 mL, 250 mmol, 5.0 equiv) at 25 °C, and the resulting reaction mixture was warmed to reflux for 2 h. A white precipitate formed as the reaction mixture refluxed. When TLC and HPLC showed that the reaction was complete, the reaction mixture was cooled to room temperature before being quenched with H₂O (100 mL). The aqueous solution was then extracted with CH₂Cl₂ (3 x 200 mL), and the combined organic extracts were washed with H₂O (2 x 100 mL) and saturated aqueous NaCl (100 mL), dried over MgSO₄, and concentrated *in vacuo.* The residue was further dried *in vacuo* to afford the desired (5*S*)-5-aminomethyl-3-(3-fluoro-4-iodo-phenyl)-oxazolidin-2-one **107** (16.0 g, 95.2% yield) as a white powder. This product was directly used in the subsequent reactions without further purification. C₁₀H₁₀FIN₂O₂, LCMS (EI) *m*/*e* 337 (M⁺+H).

A suspension of amine **107** (16.0 g, 47.6 mmol) in CH₂Cl₂ (150 mL) was treated with TEA (9.62 g, 13.2 mL, 95.2 mmol, 2.0 equiv) at 25 °C, and the resulting reaction mixture was cooled to 0-5 °C before being treated with acetic anhydride (Ac₂O, 7.29 g, 6.75 mL, 71.4 mmol, 1.5 equiv) and 4-*N,N*-dimethylaminopyridine (DMAP, 58 mg, 0.5 mmol, 0.01 equiv) at 0-5 °C under N₂. The resulting reaction mixture was subsequently stirred at 0-5 °C for 2 h. When TLC and HPLC showed the reaction was complete, the reaction mixture was quenched with H₂O (100 mL). The two layers were separated, and the aqueous layer was extracted with CH₂Cl₂ (2 x 50 mL). The combined organic extracts were washed with H₂O (2 x 100 mL) and saturated aqueous NaCl (100 mL), dried over MgSO₄, and concentrated *in vacuo.* The residue was further dried *in vacuo* to afford the desired (5*S*)-*N-*[3-(3-fluoro-4-iodo-phenyl)-2-oxo-oxazolidin-5-ylmethyl]-acetamide **108** (17.36 g, 96.5% yield) as a white powder. This product was directly used in subsequent reactions without further purification. ¹H NMR (300 MHz, DMSO-*d₆*) δ 1.63 (s, 3H, NHCOC*H*₃), 3.25 (t, 2H, *J* = 5.4 Hz), 3.56 (dd, 1H, *J=* 6.4, 9.2 Hz), 3.95 (t, 1H, *J=* 9.1 Hz), 4.58 (m, 1H), 5.16 (t, 1H, *J=* 5.7 Hz, O*H*), 7.02 (dd, 1H, *J=* 2.4, 8.2 Hz), 7.38 (dd, 1H, *J=* 2.4, 10.8 Hz), 7.66 (t, 1H, *J=* 7.5, 8.4 Hz), 8.08 (t, 1H, *J=* 5.8 Hz, N*H*COCH₃). C₁₂H₁₂FIN₂O₃, LCMS (EI) *m*/*e* 379 (M⁺ + H).

### b. Synthesis of aryl iodide 108 - Method B

Scheme 3 depicts an alternate synthesis of aryl iodide **108** from alcohol **103.**

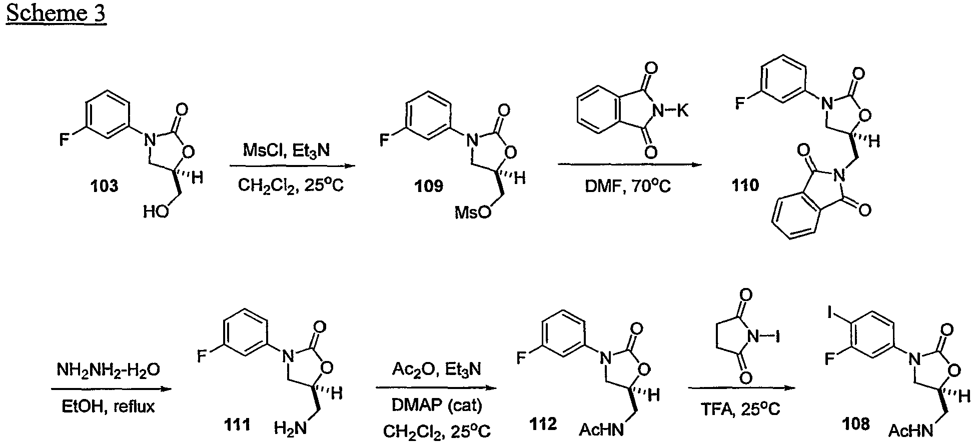

A solution of alcohol **103** (6.33 g, 30.0 mmol) in CH₂Cl₂ (60 mL) was treated with TEA (6.07 g, 8.36 mL, 60 mmol, 2.0 equiv) at 25 °C, and the resulting mixture was cooled to 0-5 °C before MsCl (3.78 g, 2.55 mL, 33.0 mmol, 1.1 equiv) was dropwise introduced into the reaction mixture at 0-5 °C under N₂. The resulting reaction mixture was subsequently stirred at 0-5 °C for 1 h under N₂. When TLC and HPLC/MS showed the reaction was complete, the reaction mixture was quenched with H₂O (40 mL) and CH₂Cl₂ (40 mL). The two layers were separated, and the aqueous layer was extracted with CH₂Cl₂ (40 mL). The combined organic extracts were washed with H₂O (2 x 40 mL) and saturated aqueous NaCl (40 mL), dried over MgSO₄, and concentrated *in vacuo.* The residue was further dried *in vacuo* to afford the desired (5R)-methanesulfonic acid 3-(3-fluoro-phenyl)-2-oxo-oxazolidin-5-ylmethyl ester **109** (7.69 g, 88.7% yield) as an off-white powder. This product was directly used in subsequent reactions without further purification. C₁₁H₁₂FNO₅S, LCMS (EI) *mle* 290 (M⁺ + H).

A solution of mesylate **109** (2.89 g, 10.0 mmol) in anhydrous DMF (20 mL) was treated with solid potassium phthalimide (2.22 g, 70.0 mmol, 1.2 equiv) at 25 °C, and the resulting reaction mixture was warmed to 70 °C for 4 h. When TLC and HPLC showed the reaction was complete, the reaction mixture was cooled to room temperature before being quenched with H₂O (60 mL). The resulting mixture was stirred at room temperature for 10 min before being cooled to 0-5 °C for 1 h. The white precipitate was collected by filtration, washed with water (2 x 40 mL), and dried *in vacuo* to afford the desired (6*R*)-2-[3-(3-fluoro-phenyl)-2-oxo-oxazolidin-5-yhnethyl]-isoindole-1,3-dione **110** (3.12 g, 91.8% yield) as an off-white powder. This product was directly used in subsequent reactions without further purification. C₁₈H₁₃FN₂O₄, LCMS (EI) *m*/*e* 341 (M⁺ + H).

A solution of phthalimide **110** (3.0 g, 8.82 mmol) in ethanol (EtOH, 30 mL) was treated with hydrazine monohydrate (2.20 g, 2.2 mL, 44.12 mmol, 5.0 equiv) at 25 °C, and the resulting reaction mixture was warmed to reflux for 2 h. White precipitates formed as the reaction mixture was refluxed. When TLC and HPLC showed the reaction was complete, the reaction mixture was cooled to room temperature before being quenched with H₂O (20 mL). The aqueous solution was then extracted with CH₂Cl₂ (3 x 40 mL), and the combined organic extracts were washed with H₂O (2 x 20 mL) and saturated aqueous NaCl (20 mL), dried over MgSO₄, and concentrated *in vacuo.* The residue was further dried *in vacuo* to afford the desired (5*S*)-5-aminomethyl-3-(3-fluoro-phenyl)-oxazolidin-2-one **111** (1.79 g, 96.6% yield) as a white powder. This product was directly used in the subsequent reactions without further purification. C₁₀H₁₁FN₂O₂, LCMS (EI) *mle* 211 (M⁺ + H).

A suspension of amine **111** (2.60 g, 12.38 mmol) in CH₂Cl₂ (40 mL) was treated with TEA (2.50 g, 3.4 mL, 24.76 mmol, 2.0 equiv) at 25 °C, and the resulting reaction mixture was cooled to 0-5 °C before being treated with acetic anhydride (Ac₂O, 1.90 g, 1.75 mL, 18.75 mmol, 1.5 equiv) and DMAP (15 mg, 0.12 mmol, 0.01 equiv) at 0-5 °C under N₂. The resulting reaction mixture was subsequently stirred at 0-5 °C for 2 h. When TLC and HPLC showed the reaction was complete, the reaction mixture was quenched with H₂O (20 mL). The two layers were separated, and the aqueous layer was then extracted with CH₂Cl₂ (2 x 20 mL). The combined organic extracts were washed with H₂O (2 x 20 mL) and saturated aqueous NaCl (20 mL), dried over MgSO₄, and concentrated *in vacuo.* The residue was further dried *in vacuo* to afford the desired (5*S*)-*N*-[3-(3-fluoro-4-phenyl)-2-oxo-oxazolidin-5-ylmethyl]-acetamide **112** (2.93 g, 94% yield) as a white powder. This product was directly used in the subsequent reactions without further purification. C₁₂H₁₃FN₂O₃, LCMS (EI) *mle* 253 (M⁺ + H).

A solution of acetamide **112** (2.3 g, 9.1 mmol) in TFA (20 mL) was treated with *N-*iodosuccinimide (2.3 g, 10.0 mmol, 1.1 equiv) at 25 °C, and the resulting reaction mixture was stirred at 25 °C for 2 h. When TLC and HPLC/MS showed the reaction was complete, the reaction mixture was concentrated *in vacuo.* The residue was treated with H₂O (20 mL) and 20% EtOAc/hexane (20 mL) at 25 °C, and the resulting mixture was stirred at 25 °C for 30 min before being cooled to 0-5 °C for 2 h. The white solids were collected by filtration, washed with H₂O (2 x 20 mL) and 20% EtOAc/hexane (2 x 20 mL), and *dried in vacuo* to afford the desired (5*S*)-*N*-[3-(3-fluoro-4-iodo-phenyl)-2-oxo-oxazolidin-5-ylmethyl]-acetamide **108** (3.34 g, 96.8% yield) as an off-white powder. This product was found to be identical with the material obtained from Method A and was directly used in subsequent reactions without further purification.

### c. Synthesis of aryl boronic acid 120

Scheme 4 depicts three synthetic routes to aryl boronic acid **115**.

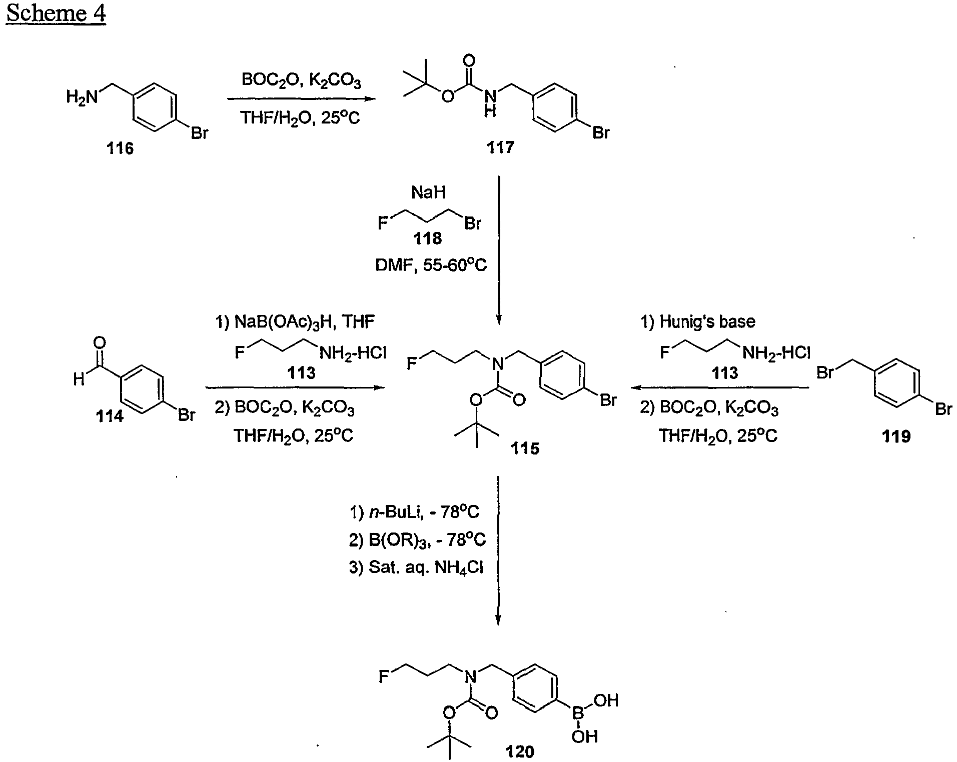

### i. Synthesis of amine 113

A solution of 3-fluoro-propan-1-ol (31.2 g, 400 mmol) in 300 mL of CH₂Cl₂ was treated with methanesulfonyl chloride (55 g, 38 mL, 480 mmol, 1.2 equiv) at 0 °C. The resulting reaction mixture was gradually warmed to room temperature and stirred for 1-2 hours. When ¹H NMR showed the reaction was complete, the reaction mixture was treated with H₂O (100 mL), and the two layers were separated. The aqueous layer was extracted with CH₂Cl₂ (2 x 100 mL). The combined organic layers were washed with H₂O (3 x 100 mL) and dried over MgSO₄. The solvent was *removed in vacuo* to afford the desired methanesulfonic acid 3-fluoro-propyl ester (57.2 g, 91% yield) as a yellow oil.

A solution of methanesulfonic acid 3-fluoro-propyl ester (34.5 g, 221 mmol) in 250 mL of anhydrous DMF was treated with solid potassium phthalimide (49 g, 265 mmol, 1.2 equiv) at 25 °C. The resulting suspension was warmed to 70-80 °C for 2 hours. When ¹H NMR showed that the reaction was complete, the reaction mixture was treated with H₂O (200 mL). The aqueous solution was extracted with EtOAc (3 x 100 mL). The combined organic layers were washed with H₂O (3 x 100 mL) and dried over MgSO₄. The solvent was removed *in vacuo* to afford the desired 2-(3-fluoro-propyl)-isoindole-1,3-dione (45.4 g, 45.5 g theoretical, 99.7% yield) as a white powder.

A suspension of 2-(3-fluoro-propyl)-isoindole-1,3-dione (45.4 g, 221 mmol) in 400 mL of 95% aqueous ethanol was treated with hydrazine monohydrate (11.3 g, 11.1 mL, 223 mmol, 1.0 equiv). The solution was refluxed for three hours. When ¹H NMR showed the reaction was complete, the reaction mixture was cooled to room temperature before being treated with concentrated aqueous HCl (250 mL) to pH 1-2. The white phthalhydrazide precipitate was collected by filtration and washed with 95 % aqueous ethanol (4 x 100 mL). The combined filtrates were then concentrated to about 100 mL before 250 mL of H₂O was added. The insoluble material was removed by filtration and the filtrates were concentrated to dryness *in vacuo.* The filtrates were recrystallized from ethanol/diethyl ether and *dried in vacuo* to afford the desired 3-fluoro-propylamine monohydrochloride salt **113** (20.83 g, 83.8 % yield) as white crystals. This product was used directly in subsequent reactions without further purification. ¹H NMR (DMSO-*d*₆,300 MHz) δ 1.89 - 2.07 (m, 2H), 2.52 - 2.90 (m, 2H), 4.47(t, 1H, *J=* 5.8Hz), 4.63(t, 1H, *J*= 5.8Hz), 8.19 (s, 3H).

### ii. Synthesis of bromide 115

### Method A

To a solution of amine **113** (6.0 g, 52.8 mmol, 1.16 equiv) in DMF (200 mL) was added 4-bromobenzalaldehyde **114** (8.50 g, 45.5mmol) at room temperature. The resulting reaction mixture was then treated with sodium triacetoxyborohydride (NaB(OAc)₃H, 16.10g, 72.0 mmol, 1.6 equiv) at room temperature and stirred for 2 h. When TLC and HPLC/MS showed the reaction was complete, the reaction mixture was quenched with water (100 mL). The resulting aqueous mixture was treated with solid sodium carbonate (Na₂CO₃, 99.64 g, 91.0 mmol, 2.0 equiv) and di-*tert*-butyl dicarbonate (BOC₂O, 12.9 g, 59.1 mmol, 1.3 equiv) at room temperature. The mixture was then stirred at room temperature for 1.5 h before being quenched with water (100 mL). The reaction mixture was then extracted with EtOAc (3 x 60 mL). The combined organic extracts were washed with 0.5 M aqueous HCl (100 mL) and water (3 x 100 mL), dried over anhydrous sodium sulfate (Na₂SO₄) and concentrated *in vacuo.* The residue was then purified by flash column chromatography (3-4% EtOAc/hexane) to afford the desired (4-bromo-benzyl)-(3-fluoro-propyl)-carbamic acid *tert*-butyl ester **115** (11.38 g, 72% yield) as a colorless oil. C₁₅H₂₁BrFNO₂, HPLC/MS (ESI) *m*/*e* 347 (N⁺+H).

### Method B

A solution of 4-bromobenzylamine hydrochloride **116** (2.225g, 10.0 mmol) and potassium carbonate (2.07 g, 15.0 mmol, 1.5 equiv) in THF (20 mL) and water (5 mL) was treated with BOC₂O (2.40 g, 11.0 mmol, 1.1 equiv) at room temperature and stirred for 12 h. When TLC and HPLC/MS showed the reaction was complete, the reaction mixture was treated with water (10 mL) and EtOAc (40 mL). The two layers were separated, and the aqueous layer was extracted with EtOAc (20 mL). The combined organic extracts were washed with water (2 x 20 mL) and saturated aqueous NaCl (20 mL), dried over MgSO₄, and concentrated *in vacuo.* The residue was further dried *in vacuo* to afford the desired (4-bromo-benzyl)-carbamic acid *tert*-butyl ester **117** (2.60 g, 90.9% yield) as a colorless oil.

To a solution **of 117** (286 mg, 1.0 mmol) in anhydrous DMF (3.0 mL) was added sodium hydride (NaH, 60% oil dispersion, 48.0 mg, 1.2 mmol, 1.2 equiv) at 0 °C. The resulting mixture was stirred at 0 °C for 30 min before 1-bromo-3-fluoropropane **118** (170 mg, 1.2 mmol, 1.2 equiv) was added. The reaction mixture was subsequently warmed to 50-60 °C and stirred for 24 hours. The reaction mixture was then quenched with water (10 mL), and the resulting aqueous solution was extracted with EtOAc (2 x 20 mL). The combined organic extracts were washed with water (10 mL) and saturated aqueous NaCl (10 mL), dried over MgSO₄ and concentrated *in vacuo.* The residue was purified by column chromatography (10-15% EtOAc/hexane gradient elution) to afford the desired (4-bromo-benzyl)-(3-fluoro-propyl)-carbamic acid *tert*-butyl ester **115** (158 mg, 46% yield) as a colorless oil.

### Method C

A solution of 4-bromobenzylbromide **119** (0.30 g, 1.20 mmol) and amine **113** (0.272 g, 2.40 mmol, 2.0 equiv) in anhydrous DMF (8.0 mL) was treated with diisopropylethylamine (Hunig's base, 2.0 mL) at room temperature. The resulting reaction mixture was warmed to 60 °C for 24 h. When TLC and HPLC showed the reaction was complete, the reaction mixture was cooled to 25 °C before being treated with water (8.0 mL). The resulting aqueous solution was then treated with solid sodium bicarbonate (NaHCO₃, 0.30 g, 3.60 mmol, 3.0 equiv) and BOC₂O (0.524 g, 2.40 mmol, 2.0 equiv) at 25 °C and stirred for 24 h. When TLC and HPLC/MS showed the reaction was complete, the reaction mixture was treated with water (20 mL) and EtOAc, 20 mL. The two layers were separated, and the aqueous layer was extracted with EtOAc (2 x 30 mL). The combined organic extracts were washed with H₂O (4 x 10 mL) and saturated aqueous NaCl (10 mL), dried over MgSO₄, and concentrated *in vacuo.* The residue was purified by column chromatography (3% EtOAc/hexanes) to afford the desired (4-bromo-benzyl)-(3-fluoro-propyl)-carbamic acid *tert*-butyl ester **115** (0.24 g, 57.8% yield) as a colorless oil.

### iii. Synthesis of boronic acid 120

To a solution of bromide **115** (3.0 g, 8.7 mmol) in anhydrous THF (30 mL) at -78 °C was added a 2.5 M solution of *n*-BuLi in hexane (3.64 mL, 9.1 mmol, 1.05 equiv). The resulting reaction mixture was stirred at -78 °C for 1 h before trimethyl borate (B(OMe)₃, 1.2 mL, 10.4 mmol, 1.2 equiv) was added dropwise. The resulting reaction mixture was stirred at -78 °C for 0.5 h before being gradually warmed to room temperature overnight. The reaction mixture was poured into water (60 mL), and the aqueous solution was treated with 1.0 N aqueous HCl to pH 4.0. The aqueous mixture was then extracted with EtOAc (4 x 30 mL). The combined organic extracts were washed with saturated aqueous NaCl (30 mL), dried over anhydrous Na₂SO₄, and concentrated *in vacuo* to obtain the desired 4-(*N*-*tert*-butylcarbonyl-3-fluoropropylaminomethyl) phenyl boronic acid **120** (2.5 g). This product was directly used in subsequent reactions without further purification. C₁₅H₂₃BFNO₄, HPLC/MS (ESI) *m*/*e* 312 (M⁺+H).

### d. Synthesis of Compound 11

Scheme 5 depicts the synthesis of compound **11** from aryl iodide **108** and aryl boronic acid **120.**

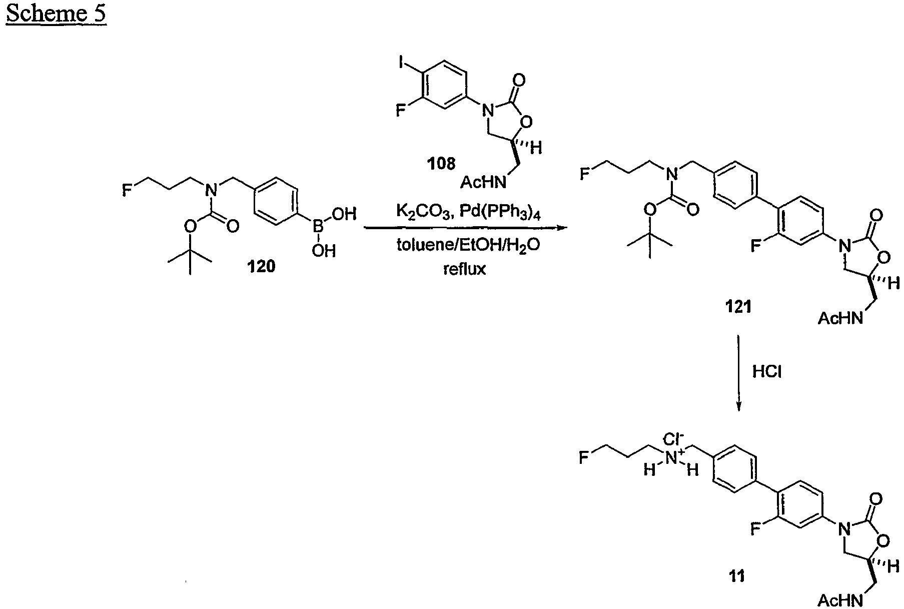

A suspension of aryl boronic acid **120** (2.50 g, 8.03 mmol) in a mixture of toluene (24 mL), EtOH (8 mL), and water (8 mL) was treated with aryl iodide **108** (2.53 g, 6.7 mmol, 0.83 equiv) and solid K₂CO₃ (2.80 g, 20.1 mmol, 3.0 equiv) at room temperature. The resulting reaction mixture was degassed three times under a steady stream of argon before tetrakis(triphenylphosphine)palladium (0) (Pd(PPh₃)₄, 387 mg, 0.335mmol, 0.05 equiv) was added. The resulting reaction mixture was degassed three times under a steady stream of argon before being warmed to reflux for 8 h. When TLC and HPLC/MS showed the reaction was complete, the reaction mixture was cooled to room temperature before being poured into water (60 mL) and EtOAc (60 mL). The two layers were separated, and the organic phase was washed with water (30 mL) and saturated aqueous NaCl (2 x 30 mL), dried over anhydrous Na₂SO₄, and concentrated *in vacuo.* The product was then recrystallized from EtOAc/hexanes and dried *in vacuo* to afford the desired (5*S*)-{4'-[5-(acetylamino-methyl)-2-oxo-oxazolidin-3-yl]-2'-fluoro-biphenyl-4-ylmethyl}-(3-fluoro-propyl)-carbamic acid *tert*-butyl ester **121** (1.3 g, 30%) as an off-white powder.

A solution of BOC-amine **121** (15.65 g, 30.3 mmol) in CH₂Cl₂ (30 mL) was treated with a solution of 4 N hydrogen chloride in 1,4-dioxane (37.5 mL, 150.0 mmol, 5.0 equiv) at room temperature and stirred for 12 h. When TLC and HPLC/MS showed that the reaction was complete, the solvents were removed *in vacuo.* The residue was suspended in a mixture of acetonitrile (CH₃CN, 200 mL) and methanol (MeOH, 50 mL), and the resulting slurry was stirred at room temperature for 1 h. The solids were collected by filtration, washed with 20% MeOH/CH₃CN (2 x 50 mL), and dried *in vacuo* to afford the desired (5*S*)-*N*-(3-{2-fluoro-4'-[(3-fluoro-propylamino)-methyl]-biphenyl-4-yl}-2-oxo-oxazolidin-5-ylmethyl)-acetamide mono hydrochloride salt **11** (13.0 g, 95.3% yield) as white crystals. ¹H NMR (300 MHz, DMSO-*d*₆) δ 1.90 (s, 3H, COC*H*₃), 2.11-2.20 (m, 2H), 3.10 (m, 2H), 3.50 (t, 2H, *J*= 5.4 Hz), 3.87 (dd, 1H, *J* = 6.4, 9.2 Hz), 4.24 (t, 1H, *J=* 9.1 Hz), 4.27 (s, 2H, ArC*H*₂), 4.54 (t, 1H, *J*= 5.8 Hz), 4.70 (t, 1H, *J*= 5.8 Hz), 4.83 (m, 1H), 7.50 (dd, 1H, *J=* 2.2, 8.6 Hz), 7.65-7.74 (m, 6H, aromatic-*H*), 8.37 (t, 1H, *J*= 5.8 Hz, N*H*COCH₃), 9.43 (br. s, 2H, RArN⁺*H*₂). C₂₂H₂₅F₂N₃O₃ HCl, LCMS (EI) *mle* 418 (M⁺+H).

### Example 2

Scheme 6 depicts an alternate synthesis of aryl boronic acid **120**, which is coupled to aryl iodide **108** to yield compound **11**.

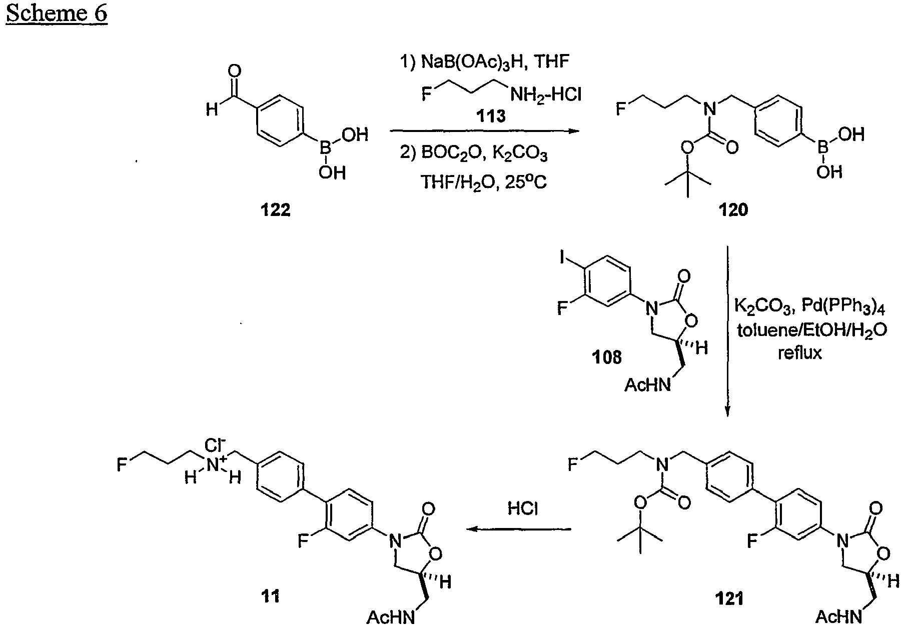

A solution of 4-formylphenyl boronic acid **122** (10.0 g, 66.69 mmol) in anhydrous DMF (150 mL) was treated with 3-fluoropropylamine hydrochloride salt **113** (8.70 g, 76.70 mmol, 1.15 equiv, prepared as described in Example 1, above) at room temperature. The resulting mixture was treated with NaB(OAc)₃H (28.30 g, 133.39 mmol, 2.0 equiv) at room temperature and stirred for 3 h. When TLC and HPLC/MS showed the reaction was complete, the reaction mixture was treated with water (150 mL), solid Na₂CO₃ (14.14 g, 133.39 mmol, 2.0 equiv), and BOC₂O (22.05 g, 100.04 mmol, 1.5 equiv). The resulting reaction mixture was stirred at room temperature for 3 h. When TLC and HPLC/MS showed the reaction was complete, the reaction mixture was poured into water (500 mL) and EtOAc (500 mL). The two layers were separated and the aqueous layer was treated with a 2 N aqueous HCl (130 mL) to pH 4. The aqueous layer was then extracted with EtOAc (160 mL), and the combined organic layers were washed with water (2 x 100 mL) and saturated aqueous NaCl (2 x 100 mL), dried over Na₂SO₄, and concentrated *in vacuo.* The residue was further *dried in vacuo* to afford the desired 4-(*N-tert-*butylcarbonyl-3-fluoropropylaminomethyl)phenyl boronic acid **120** (25.0 g) as a pale-yellow oil. This product was found to be identical with the material obtained from Example 1 above and was directly used in the subsequent reaction without further purification.

A suspension of aryl boronic acid **120** (25.0 g, 64.30 mmol, 1.45 equiv) in a mixture of toluene (120 mL), EtOH (40 mL), and water (40 mL) was treated with (5*S*)-*N*-[3-(3-fluoro-4-iodo-phenyl)-2-oxo-oxazolidin-5-ylmethyl]-acetamide **108** (16.80 g, 44.44 mmol, prepared as described in Example 1, above) and solid K₂CO₃ (18.40 g, 133.4 mmol, 3.0 equiv) at room temperature. The resulting reaction mixture was degassed three times under a steady stream of argon before being treated with Pd(PPh₃)₄ (2.57 g, 2.23 mmol, 0.05 equiv). The resulting reaction mixture was degassed three times under a steady stream of argon before being warmed to reflux for 8 h. When TLC and HPLC/MS showed the reaction was complete, the reaction mixture was cooled to room temperature before being poured into water (300 mL) and ethyl acetate (EtOAc, 300 mL). The two layers were separated, and the organic phase was washed with water (60 mL) and saturated aqueous NaCl (2 x 50 mL), dried over anhydrous Na₂SO₄, and *concentrated in vacuo.* The product was recrystallized from EtOAc/hexanes and dried *in vacuo* to afford the desired (5*S*)-{4'-[5-(acetylamino-methyl)-2-oxo-oxazolidin-3-yl]-2'-fluoro-biphenyl-4-ylmethyl}-(3-fluoro-propyl)-carbamic acid *tert*-butyl ester **121** (21.2 g, 61.5% yield for three steps) as an off-white powder.

BOC-protected amine **121** was subsequently treated with 4 N hydrogen chloride in 1,4-dioxane as in Example 1 to afford compound **11**. The product obtained from this process was identical by NMR and LCMS to the material obtained in Example 1.

### Example 3

Scheme 7 depicts the synthesis of compound **11** from aryl bromide **115** and aryl boronic ester **123**.

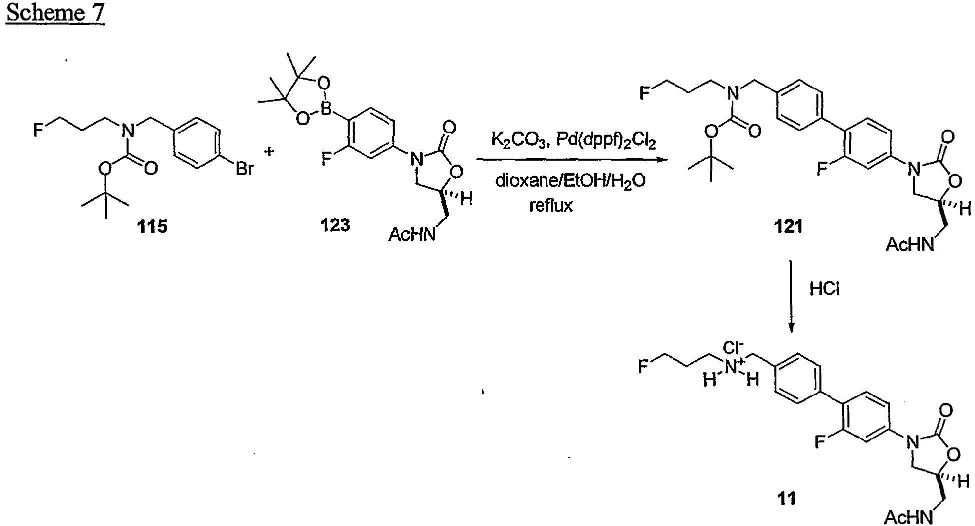

### Synthesis of boronic ester 123

A suspension of (5*S*)-*N*-[3-(3-fluoro-4-iodo-phenyl)-2-oxo-oxazolidin-5-ylmethyl]-acetamide **108** (20.0 g, 52.8 mmol, prepared as described in Example 1, above) in anhydrous 1,4-dioxane (130 mL) was treated with 4,4,5,5-tetramethyl-[1,3,2]dioxaborolane (10.2 g, 11.6 mL, 80.0 mmol, 1.5 equiv) and triethylamine (16.0 g, 22.4 mL, 158.4 mmol, 3.0 equiv) at room temperature. The resulting reaction mixture was degassed three times under a steady stream of argon before being treated with dichloro[1,1'-bis(diphenylphosphino)ferrocene] palladium (II) (Pd(dppf)₂Cl₂, 1.32 g, 1.6 mmol, 0.03 equiv) at room temperature. The resulting reaction mixture was degassed three times under a steady stream of argon before being warmed to reflux for 7 h. When HPLC/MS showed the reaction was complete, the reaction mixture was cooled to room temperature before being treated with water (100 mL) and ethyl acetate (100 mL). The two layers were separated, and the aqueous layer was extracted with ethyl acetate (2 x 50 mL). The combined organic extracts were washed with water (2 x 50 mL) and saturated aqueous NaCl (50 mL), dried over MgSO₄, and concentrated *in vacuo.* The residual brown oil was further dried *in vacuo* to afford the desired (5*S*)-*N*-{3-[3-fluoro-4-(4,4,5,5-tetramethyl-[1,3,2]dioxaborolan-2-yl)-phenyl]-2-oxo-oxazolidin-5-ylmethyl}acetamide **123** (18.8 g, 94%) as brown solids. This product was directly used in subsequent reactions without further purification. C₁₈H₂₄BFN₂O₅, HPLC/MS (ESI) *mle* 379 (M⁺+H).

### Synthesis of compound 11

A solution of boronic ester **123** (1.40 g, 3.7 mmol, 1.3 equiv) and (4-bromo-benzyl)-(3-fluoro-propyl)-carbamic acid *tert*-butyl ester **115** (1.0 g, 2.89 mmol, prepared as described in Example 1, above) in a mixture of 1,4-dioxane (21 mL), EtOH (7.0 mL) and H₂O (7.0 mL) was treated with solid potassium carbonate (1.2 g, 8.7 mmol, 3.0 equiv) at room temperature. The resulting reaction mixture was degassed three times under a steady stream of argon before being treated with Pd(dppf)₂Cl₂ (118 mg, 0.144 mmol, 0.05 equiv) at room temperature. The reaction mixture was degassed three times under a steady stream of argon before being warmed to reflux for 2 h. When TLC and HPLC/MS showed the reaction was complete, the reaction mixture was cooled to room temperature before being treated with water (60 mL). The aqueous solution was then extracted with CH₂Cl₂ (3 x 20 mL), and the combined organic extracts were washed with water (2 x 20 mL) and saturated aqueous NaCl (20 mL), dried over MgSO₄, and concentrated *in vacuo.* The residue was purified by flash column chromatography (0-5 % MeOH-CH₂Cl₂ gradient elution) to afford the desired (5*S*)-{4'-[5-(acetylamino-methyl)-2-oxo-oxazolidin-3-yl]-2'-fluoro-biphenyl-4-ylmethyl}-(3-fluoro-propyl)-carbamic acid *tert*-butyl ester **121** (1.36 g, 91% yield) as a colorless oil, which solidified upon standing at room temperature *in vacuo.*

BOC-protected amine **121** was subsequently treated with 4 N hydrogen chloride in 1,4-dioxane as in Example 1 to afford compound **11**. The product obtained from this process was identical by NMR and LCMS to the material obtained in Example 1.

### Example 4 - Synthesis of Compound 63

Scheme **8** illustrates the synthesis of amide **63.** 2,5-Dibromopyridine **124** is converted to activated pyridyl ester **125** which is then treated with histamine **126** to provide amide **127**. The Suzuki coupling of **127** and boronate **123** gave the final target amide **63.**

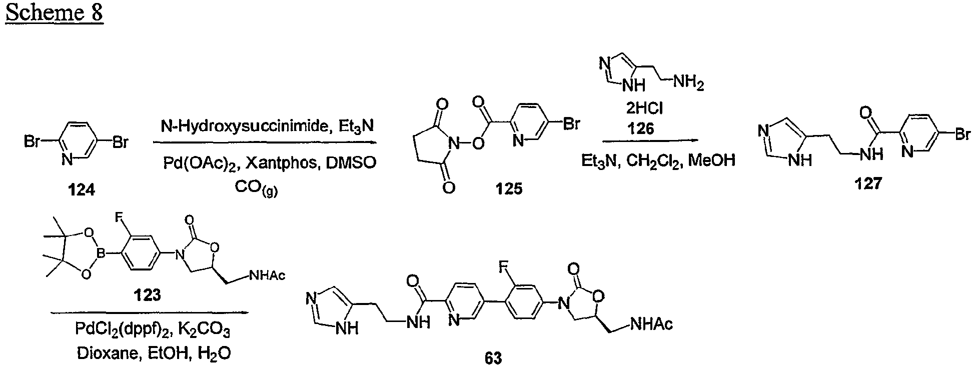

Under an argon atmosphere, triethylamine (0.31 mL, 2.25 mmol) was added to a mixture of 2,5-dibromopyridine **124** (355 mg, 1.5 mmol), palladium acetate (16.8 mg. 0.075 mmol), Xantphos (4,5-bis(diphenylphosphino)-9,9-dimethylxanthene, 43.4 mg, 0.075 mmol) and *N-*hydroxysuccinimide (241.5 mg, 2.1 mmol) in DMSO (2 mL). The solution was purged with carbon monoxide for 15 min and stirred under a carbon monoxide balloon at 80°C for 16 h. The reaction mixture was then cooled to room temperature, diluted with 20 mL of ethyl acetate and washed with saturated sodium bicarbonate solution and water. The organic phase was dried over sodium sulfate and evaporated to give crude product. Chromatography on silica gel using hexane:acetone (3:1) provided ester **125** (75 mg; 17%). ¹HNMR (300 MHz, CDCl₃) δ 8.85 (m, 1H), 8.06 (m, 2H), 2.90 (s, 4H).

A mixture of active ester **125** (350 mg, 1.17 mmol), histamine dihydrochloride **126** (216 mg, 1.17 mmol) and triethylamine (Et₃N, 0.33 mL, 2.34 mmol) in CH₂Cl₂ (5 mL) was stirred at room temperature for 1 h. The reaction was washed with brine and dried under vaccum. The crude product was purified by chromatography (15:1:0.05/CH₂Cl₂:MeOH:NH₃.H₂O) to afford **127** (280 mg; 81%). LCMS (ESI) *mlz* 295 (M + H)⁺.

Under an argon atmosphere, a mixture of amide **127** (230 mg, 0.78 mmol), boronate **123** (295 mg, 0.78 mmol), PdCl₂(dppf)₂ (19 mg, 0.023 mmol) and K₂CO₃ (323 mg, 2.34 mmol) in 5 mL of a mixture of dioxane:EtOH:H₂O (3:1:1) were heated at 100°C for 12 h. The reaction was concentrated and the residue was dissolved in MeOH (2 mL) and CH₂Cl₂ (10 mL). Inorganic salts were removed by filtration. The filtrate was concentrated and purified by chromatography (15:1:0.05/CH₂Cl₂:MeOH:NH₃.H₂O) to afford amide **63** (106 mg; 29%). LCMS (ESI) *m*/*z 467* (M+H)⁺.

### Example 5 - Synthesis of Compounds 64 and 65

Scheme 9 illustrates the synthesis of amides **64** and **65.** Aryl bromides **128** and **129** were coupled to boronate **123** to afford **64** and **65** respectively.

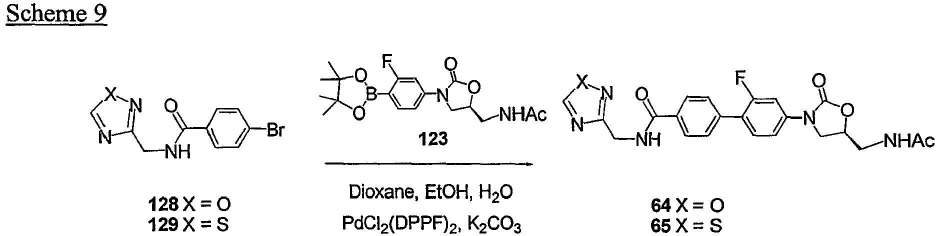

### Synthesis of compound 64

A mixture of 4-bromobenzoyl chloride (110 mg, 0.5 mmol), 1,2,4-oxadiazol-3-yl-methylamine hydrochloride (68 mg, 0.5 mmol), DMF (1 drop) and Et₃N (0.33 mL, 2.34 mmol) in CH₂Cl₂ (5 mL) was stirred at room temperature for 4 h. The reaction was washed with brine and dried under vaccum to afford crude amide **128.** The resultant amide **128** was added to a mixture of boronate **123** (189 mg, 0.5 mmol), Pd(dppf)₂Cl₂ (20 mg, 0.025 mmol) and K₂CO₃ (207 mg, 1.5 mmol) in 5 mL of dioxane:EtOH:H₂O (3:1:1) under an argon atmosphere. After being heated at 100°C for 12 h, the reaction was diluted with water and MeOH, and then filtered through celite. The filtrate was concentrated to remove organic solvent. The crude product was collected by filtration and further purified by chromatography (25:1:0.05/CH₂Cl₂:MeOH:NH₃.H₂O) to afford compound **246** (45 mg; 32% (2 steps)). LCMS (ESI) *mlz* 452 (M - H)⁺.

### Synthesis of compound 65

A mixture of 4-bromobenzoyl chloride (29 mg, 0.132 mmol), 1,2,4-thiadiazol-3-yl-methylamine hydrochloride (20 mg, 0.132 mmol), DMF (1 drop) and Et₃N (27 mg, 0.264 mmol) in THF (4 mL) was stirred at room temperature for 2 h. The reaction was concentrated, dissolved in CH₂Cl₂, washed with brine and dried under vaccum to afford crude amide **129.** The resultant amide **129** was added to a mixture of boronate **123** (50 mg, 0.132 mmol), PdCl₂(dppf)₂ (6 mg, 0.0066 mmol) and K₂CO₃ (55 mg, 0.396 mmol) in 2 mL of dioxane:EtOH:H₂O (3:1:1) under an argon atmosphere. After being heated at 100°C for 12 h, the reaction was concentrated, dissolved in EtOAc, washed with brine and dried under vaccum. The crude product was purified by chromatography on silica gel (25:1:0.05/CH₂Cl₂:MeOH:NH₃.H₂O) to afford compound **65** (30 mg; 48% (2 steps)). LCMS (ESI) *m*/*z* 470 (M + H)⁺.

### Example 6 - Synthesis of Compound 66

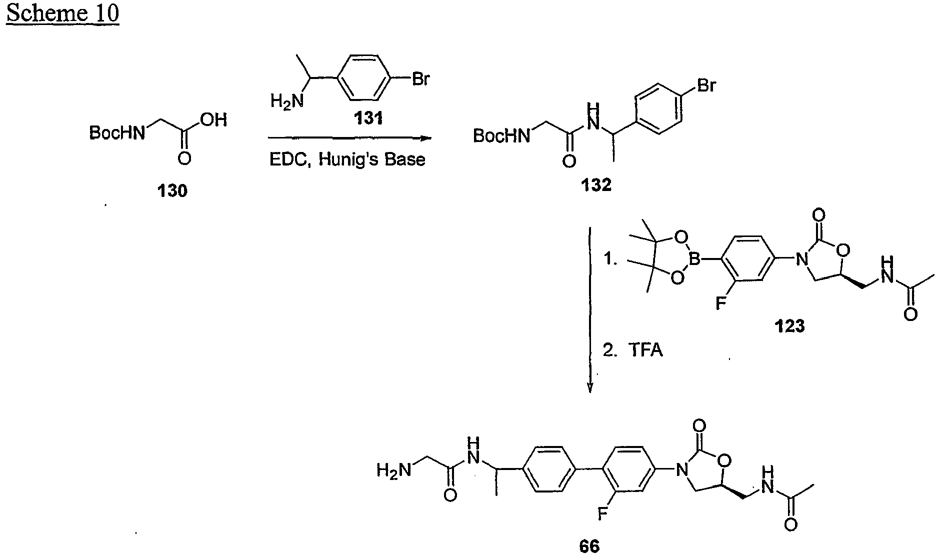

To a solution of Boc-glycine **130** (1.04g, 5.88mmol) and 4-bromobenzylethylamine **131** (1.00g, 4.90mmol) in CH₂Cl₂ (25mL) at room temperature was added Hunig's base (1.30mL, 7.35mmol). The mixture was stirred at room temperature for 16h. The mixture was poured into water (40mL) and sat. NaHCO₃ (3mL), then extracted with CH₂Cl₂ (60mL), washed with water (100mL), and dried with Na₂SO₄. The residue was isolated by column chromatography (50/50/0.1 EtOAc/Hexane/ NH₄OH), to give 1.30g of amide **132** as a white crystalline solid in 69% yield. ¹HNMR (300 MHz, CDCl₃, ppm): δ: 7.37 (d, J=7Hz, 2H), 7.09 (d, J=7Hz, 1H), 6.50 (s br, 1H), 5.15 (s br, 1H), 5.01 -4.95 (m, 1H), 3.69 (d, J=6Hz, 2H), 1.39 (d, J=7Hz, 3H), 1.37 (s, 9H).

A mixture of amide **132** (143mg, 0.40mmol) and boronic ester **123** (168mg, 0.4mmol), Pd(dppf)₂Cl₂ (16mg, 0.02mmol) and K₂CO₃ (221mg, 1.60mmol) in dioxane (3mL), EtOH (1mL) and H₂O (1mL) was degassed with argon. The mixture was stirred at 90-95 °C for 3h, then water (10mL) was added. The mixtue was extracted with CH₂Cl₂ (4 x 30mL) and dried over Na₂SO₄. The residue was purified by column chromatography (5:100:0.1 MeOH/ CH₂Cl₂/NH₄OH) to yield 200 mg Boc-protected product in100% yield. The Boc-protected product (200 mg) taken up in 2 mL CH₂Cl₂ and 2 mL TFA and the mixture was stirred at room temperature for 3 hr. The solvent was removed *in vacuo* and the residue was purified by column chromatography (15:85:0.1 MeOH/CH₂Cl₂/NH₄OH) to give 168mg compound **66** in 99% yield. ¹HNMR (300 MHz, CDCl₃, ppm, partial): δ: 7.64-7.33 (m, 7H), 5.13-5.07 (m, 1H), 4.19 (ddd, J=9, 3, 3Hz, 1H), 3.87 (ddd, J=9, 7, 3Hz, 1H), 3.77-3.50 (m, 5H), 1.99 (s, 3H), 1.54 (d, J=7Hz, 3H). LCMS (ESI) m/e 429 (M+H)⁺.

### Example 7 - Synthesis of Compound 67

Scheme 11 illustrates the synthesis of compound **67**.

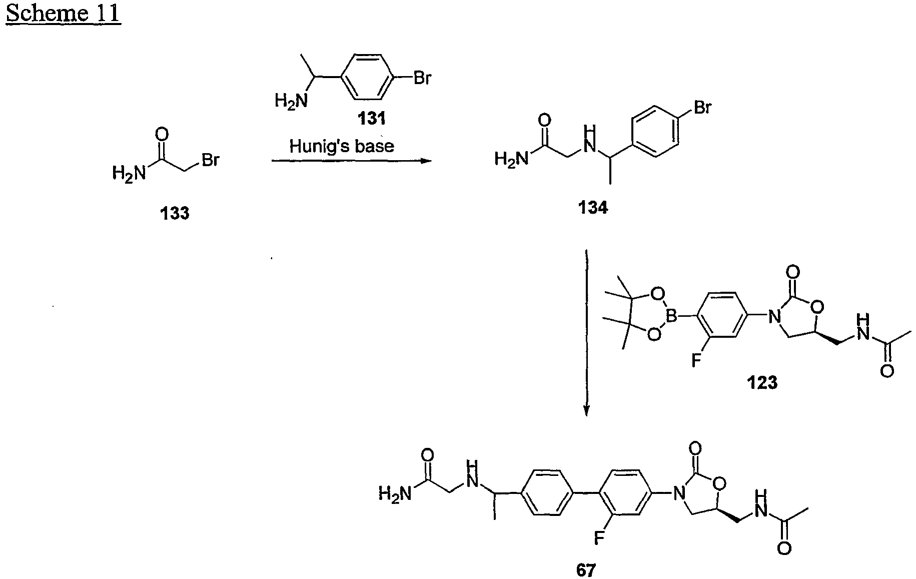

To a solution of 2-bromoacetamide **133** (827mg, 5.88mmol), and 4-bromobenzylethylamine **131** (1.00g, 4.90mmol) in MeOH (5mL) and CH₂Cl₂ (5mL) at room temperature was added Hunig's base (5mL). The mixture was stirred at 50-60 °C for 16 h, then water (30mL) was added. The mixture was extracted with CH₂Cl₂ (4 x 30mL), dried over Na₂SO₄, and the solvent was removed *in vacuo* to provide 1.27 g amide **134** as a white crystalline solid in 100% yield. ¹HNMR (300 MHz, CDCl₃, ppm): δ: 7.38 (d, J=8Hz, 2H), 7.09 (d, J=8Hz, 1H), 6.77 (s br, 1H), 5.69 (s br, 1H), 3.67 (q, J=7Hz, 1H), 3.07 (s, 2H), 1.29 (d, J=7Hz, 3H).

A mixture of amide **34** (103mg, 0.40mmol) and boronic ester **123** (168mg, 0.4mmol), Pd(dppf)₂Cl₂ (16mg, 0.02mmol) and K₂CO₃ (221mg, 1.60mmol) in dioxane (3mL), EtOH (1mL) and H₂O (1mL) was degassed with argon. The mixture was stirred at 90-95 °C for 3 h, then water (10mL) was added. The mixture was extracted with CH₂Cl₂ (4 x 30mL), dried over Na₂SO₄, and the solvent was removed *in vacuo.* The residue was purified by column chromatography (7:100:0.1 MeOH/CH₂Cl₂/NH₄OH) to yield 85 mg compound **67** in 50% yield. ¹HNMR (300 MHz, CDCl₃, ppm, partial): δ: 7.70-7.30 (m, 7H), 7.09 (s br, 1H), 6.31 (s br, 1H), 5.63 (s br, 1H), 4.96-4.92 (m, 1H), 4.22 (t, J=9Hz, 1H), 3.33 (s, 2H), 2.13 (s, 3H), 1.55 (d, J=7Hz, 3H). LCMS (ESI) m/e 451.2 (M+Na)⁺.

### Example 8 - Synthesis of Compound 68

Scheme 12 illustrates the synthesis of compound **68**.

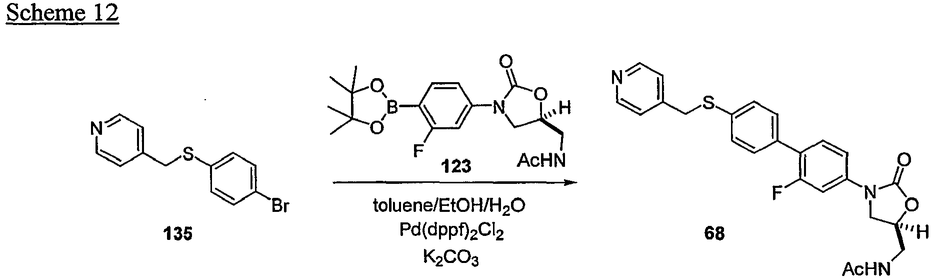

### Synthesis of bromide 135

A suspension of 4-bromomethylpyridine hydrochloride (1.59 g, 6.3 mmol) in THF (10 mL) was treated dropwise with a solution of potassium carbonate (3.33 g, 24.0 mmol) in H₂O (6 mL) at 0-5 °C, and the resulting mixture was stirred at 0-5 °C for 10 min before being treated dropwise with a solution of 4-bromo-benzenethiol (1.14 g, 6.0 mmol) in THF (5.0 mL) at 0-5 °C under N₂. The resulting reaction mixture was subsequently stirred at 0-5 °C for an additional 20 min. When TLC and LCMS showed that the reaction was complete, the reaction mixture was treated with water (15 mL) and ethyl acetate (25 mL). The two layers were separated, and the aqueous layer was extracted with ethyl acetate (2 x 20 mL). The combined organic extracts were washed with water (2 x 15 mL) and saturated aqueous NaCl solution (10 mL), dried over MgSO₄, and concentrated *in vacuo.* The residue was purified by flash column chromatography (5-25% EtOAc-hexane gradient elution) to afford the desired 4-(4-bromo-phenylsulfanylmethyl) pyridine (1.374 g; 82%) as a pale-yellow solid, which was directly used in subsequent reactions.

### Synthesis of compound 68

A solution of boronate **123** (200 mg, 0.53 mmol) and bromide **135** (150 mg, 0.53 mmol) in toluene (9 mL) was treated with solid potassium carbonate (220 mg, 1.6 mmol), ethanol (3.0 mL) and H₂O (3.0 mL) at room temperature, and the resulting reaction mixture was degassed three times under a steady stream of argon before being treated with Pd(dppf)₂Cl₂ (16 mg, 0.013 mmol) at room temperature. The reaction mixture was then degassed three times again under a steady stream of argon before being warmed to reflux for 2 h. When LCMS showed that the reaction was complete, the reaction mixture was cooled to room temperature before being treated with water (10 mL) and ethyl acetate (20 mL). The two layers were separated, and the aqueous layer was extracted with ethyl acetate (2 x 10 mL). The combined organic extracts were washed with water (2 x 10 mL) and saturated aqueous NaCl (10 mL), dried over MgSO₄, and concentrated *in vacuo.* The residue was then purified by flash column chromatography (0-5% MeOH-CH₂Cl₂ gradient elution) to afford compound **68** (177 mg; 74%) as a yellow oil, which solidified upon standing at room temperature *in vacuo.* LCMS (ESI) *m*/*z* 452 (M + H)⁺.

### Example 9 - Synthesis of Compound 69

Scheme 13 illustrates the synthesis of compound **69.**

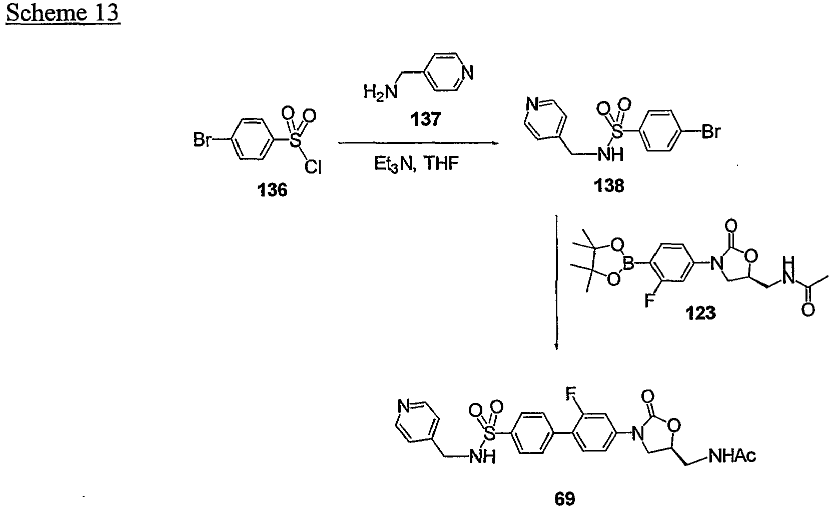

4-bromobenzenesulfonyl chloride **136** (2.56 g, 10 mmol) was added to a solution of 4-aminomethylpyridine **137** (1.08 g, 10 mmol) and triethylamine (2 mL, 14.3 mmol) in THF (20 mL) at 0 °C. After stirring at 0 °C for 1 h, 50 mL of water was added. A white solid was collected by filtration, washing with EtOAc and *dried in vacuo* to give 3.10 g of bromide **138** in a yield of 95%.

Bromide **138** (327 mg, 1 mmol), boronate **123** (378 mg, 1 mmol), Pd(dppf)₂Cl₂ (40 mg, 0.05 mmol) and K₂CO₃ (414 mg, 3 mmol) were dissolved 8 mL of a mixture of dioxane:EtOH: H₂O (3:1:1) under argon atmosphere. After heating at 100 °C for 12 hours, the reaction mixture was added to 20 mL of cool water. The organic solvent was removed *in vacuo* and the crude product was collected by filtration. The crude product was treated with active charcoal and recrystallized in a mixed solvent system (1:2:2 MeOH/CH₂Cl₂/acetone) to give 155 mg of compound **69** in a yield of 31%. MS (ESI): 499.1 (100%, (M+H)⁺).

### Example 10 - Synthesis of Compound 70

Scheme 14 depicts the synthesis of compound **70**.

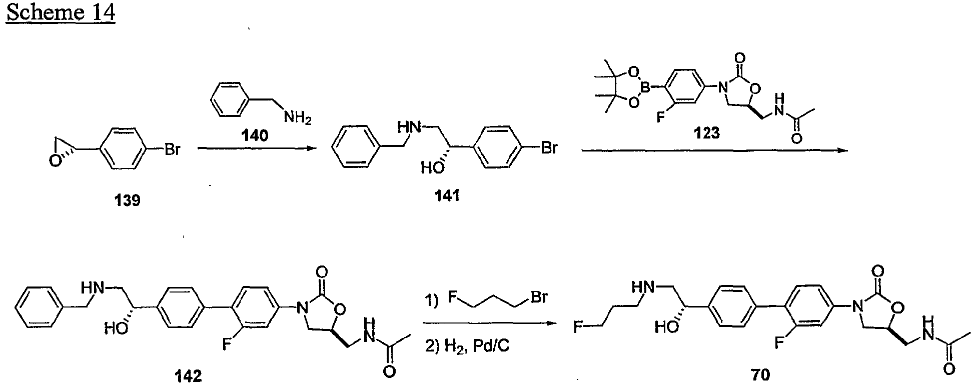

### Synthesis of epoxide 139

To a solution of 4-bromostyrene (5.00 g, 26.8 mmol) in CH₂Cl₂ (130 mL) was added anhydrous 4-methylmorpholine *N*-oxide (NMO, 12.90 g, 107.1 mmol) and (1S, 2S)-(+)-[1,2-(cyclohexanodiamino-N,N'-bis(3,5-di-t-butyl-salicylidene)] manganese(III) chloride (Jacobsen catalyst, 850 mg, 1.34 mmol). The solution was cooled to -78 °C, then 3-chloroperoxybenzoic acid (m-CPBA, 7.40 g, 42.8 mmol) was added in four portions every 10 min. The mixture was stirred at -78 °C for 2 h. The reaction was quenched by addition of aqueous Na₂S₂O₃ (10.0 g in 30 mL water), then the cooling bath was removed and water (70 mL) and 1N NaOH (60 mL) was added. The aqueous phase was extracted with CH₂Cl₂ (3 x 30 mL), dried with Na₂SO₄, and evaporated. The residue was purified by flash chromatography (4:100 Et₂O/hexane) to yield 5.20 g epoxide **139** (98% yield).

### Synthesis of compound 70

To a suspension of epoxide **139** (1 mmol) in acetonitrile (3.0 mL) at room temperature was added lithium perchloriate (LiClO₄, 1.05 mmol). After the formation of clear solution, benzylamine **140** (1.5 mmol) was added. The mixture was stirred at 80 °C for 4.5 h. The solvent was removed *in vacuo* and the residue was purified by flash column chromatography (3.5:100 MeOH/CH₂Cl₂), to afford **141** (460 mg; 50% yield). LCMS (ESI) *m*/*z* 307 (M + H)⁺.

A suspension **of 141** (1 eq), boronate ester **123** (1 eq), Pd(dppf)₂Cl₂ (0.05 eq), and K₂CO₃ (4 eq) in a 3:1:1 mixture of dioxane/EtOH/H₂O was degassed by passing a steady stream of argon through the mixture. The mixture was stirred at 80 °C for 3 h. The solvent was removed *in vacuo* and the residue was purified by flash column chromatography (3:100 MeOH/CH₂Cl₂), to yield amine **142** (690mg; 96% yield). LCMS (ESI) *mlz* 439 (M + H)⁺.

A mixture of amine **142** (80 mg, 0.168 mmol), 3-fluoro-1-bromopropane (47 mg, 0.335 mmol) and Hunig's base (117 µL, 0.670 mmol) in DMF (1.5 mL) was stirred at 55-60 °C for 15 h. The solvent was removed *in vacuo* and residue was purified by flash column chromatography (2:100 MeOH/CH₂Cl₂), to give 87 mg of the alkylation product (96% yield). LCMS (ESI) *m*/*z* 538 (M + H)⁺.

To a solution of the alkylation product (80 mg, 0.149 mmol), in EtOH (1.5 mL) at room temperature was added 3N aqueous HCl (120 µL, 0.360 mmol), followed by 10% Pd-C (15 mg). The mixture was stirred under the atmosphere of H₂ (1 atm.) for 18 h. The mixture was passed through a pad of celite, and the cake was washed with MeOH (3 x 10 mL). The filtrate was evaporated to give the HCl salt of compound **70** (57 mg; 79% yield). LCMS (ESI) *m*/*z* 448 (M + H)⁺.

### Example 11 - Synthesis of Compounds 71 and 72

Scheme 15 illustrates the synthesis of compounds **71** and **72.** Hydroxyamidine **143** was converted to bromide **144,** which was subsequently coupled to boronate **123** to afford compound **71.** Hydroxyamidine **143** was transformed to oxadiazole **145,** which was coupled to boronate **123** to afford compound **72.**

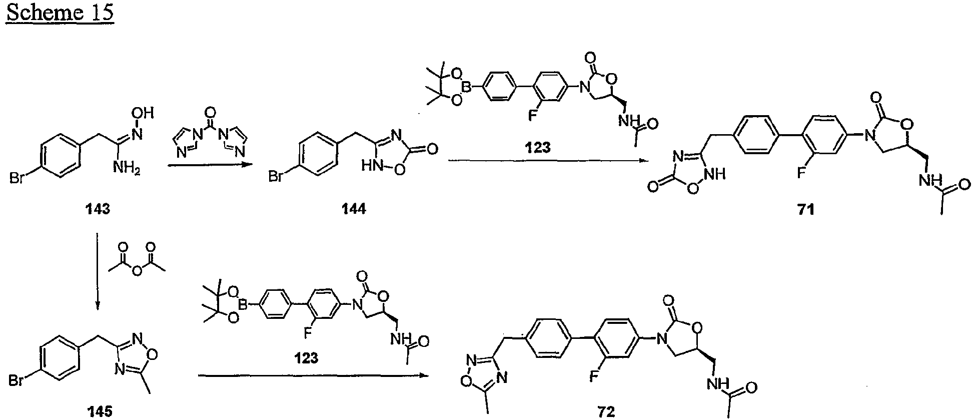

### Synthesis of hydroxamidine 143

A solution of 4-bromophenylacetonitrile (10 g, 54 mmol) in methanol (100 mL) was treated with sodium bicarbonate (2.2 g, 57 mmol) and hydroxylamine hydrochloride (4.0 g, 57 mmol) and refluxed for 1.5 h. Additional sodium bicarbonate (0.21 g, 5.4 mmol) and hydroxylamine hydrochloride (0.38 g, 5.4 mmol) were added, and the reaction mixture was refluxed for 12 h. The reaction mixture was cooled to 23 °C and the solvent removed *in vacuo* to afford hydroxyamidine **143** as a blue powder (4.0 g; 34%).

### Synthesis of compound 71

A solution of hydroxyamidine **143** (0.20 g, 0.91 mmol) in 1,4-dioxane (1 mL) was treated with 1,1'-carbonyldiimidazole (0.18 g, 1.1 mmol) and 1,8-diazabicyclo[5.4.0]undec-7-ene (DBU, 0.15 mL, 0.97 mmol) and stirred at 105 °C for 1 h. The reaction mixture was diluted with water and extracted with ethyl acetate. The water layer was treated with 1.0 M HCl (aqueous) until the pH was 2, and then extracted with ethyl acetate. The organic layer was dried (Na₂SO₄), and the solvent removed *in vacuo* to afford bromide **144** as a yellow powder (0.11 g; 49%).

A solution of boronate ester **123** (0.085 g, 0.220 mmol), bromide **144** (0.055 g, 0.220 mmol), and potassium carbonate (0.12 g, 0.90 mmol) in dioxane (1.4 mL), ethanol (0.46 mL) and water (0.46 mL) was degassed and treated with Pd(dppf)Cl₂ (6.0 mg, 6.7 µmol), degassed again, and heated at 80 °C for 1.5 h. The reaction mixture was diluted with CH₂Cl₂ and water, and the precipitate in the water layer was recovered by vacuum filtration to afford compound **71** as a grey powder (0.034 g; 36%). LCMS (ESI) *m*/*z* 427 (M + H)⁺.

### Synthesis of compound 71

A solution of hydroxyamidine **143** (0.25 g, 1.1 mmol) in pyridine (5 mL) was cooled to 0 °C and treated with a solution of acetic anhydride (0.11 mL, 1.1 mmol) in pyridine (5 mL) and then stirred at 120 °C for 1.5 h. The reaction mixture was diluted with ethyl acetate, washed with 1M aqueous HCl and saturated aqueous sodium bicarbonate, dried over Na₂SO₄, and the solvent was evaporated *in vacuo.* The crude product was purified by flash chromatography to afford bromide **145** as a clear film (0.10 g; 36%).

A solution of boronate ester **123** (0.15 g, 0.40 mmol), bromide **145** (0.10 g, 0.40 mmol), and potassium carbonate (0.22 g, 1.6 mmol) in dioxane (2.5 mL), ethanol (0.83 mL) and water (0.83 mL) was degassed and treated with Pd(dppf)Cl₂ (10.0 mg, 0.012 mmol), degassed again, and stirred at 80 °C for 2 h. The reaction mixture was diluted with CH₂Cl₂ and washed with water. The water layer was extracted with 2 x CH₂Cl₂, dried (Na₂SO₄), and the solvent evaporated *in vacuo.* The crude product was purified by flash chromatography and preparatory TLC to afford compound **72** as a white powder (0.054 g; 32%). LCMS (ESI) *m*/*z* 425 (M + H)⁺.

### Example 12 - Synthesis of Compounds 73 and 74

Scheme 16 illustrates the synthesis of compounds **73** and **74.** Bromoketone **146** was subjected to alkylation with thioureas **147** and **148** to afford thiazoles **149** and **150** respectively. Coupling of **149** and **150** with boronate **123** yielded thiazoles **73** and **74.**

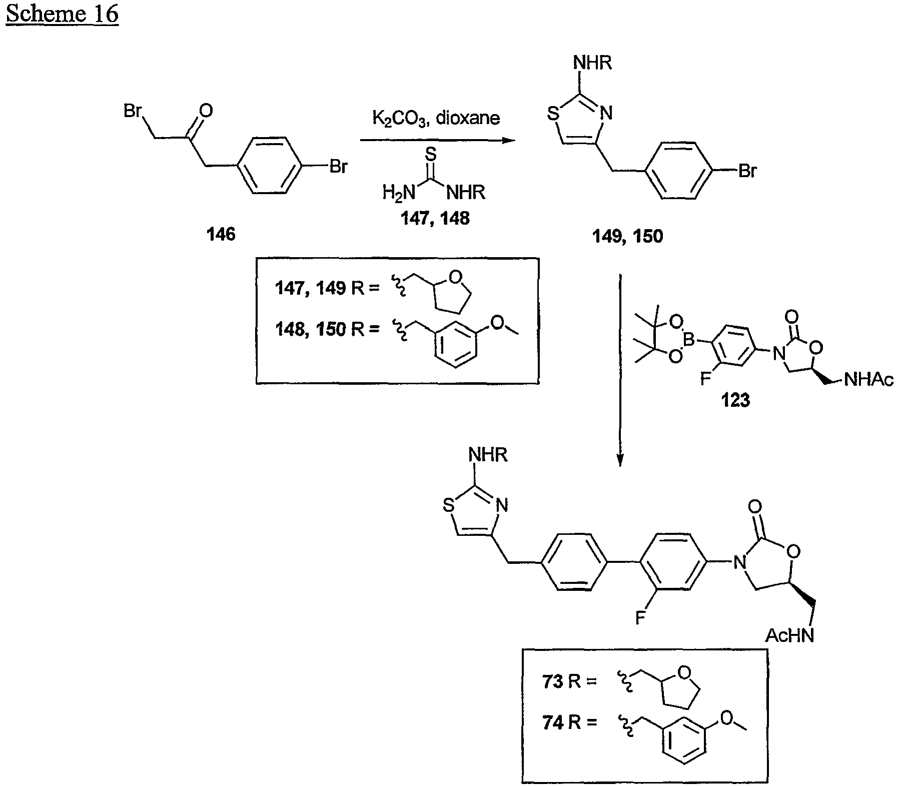

### Synthesis of Compound 73

Bromoketone **146** (0.29 g, 1.0 mmol) was dissolved in dioxane (10 mL). Thiourea **147** (0.19 g, 1.2 mmol) and potassium carbonate (0.28 g, 2 mmol) were added sequentially and the resulting slurry stirred at 50 °C for 4 h. The mixture was cooled to room temperature, diluted with 100 mL CH₂Cl₂, and washed with saturated aqueous NaHCO₃ and brine. The aqueous washes were back-extracted with CH₂Cl₂ (2 x 50 mL). The combined organic extracts were dried over K₂CO₃, filtered, and concentrated *in vacuo* to afford bromide **149** as a yellow solid (0.32 g) which was used without further purification. LCMS (ESI) *m*/*z* 353 (M + H)⁺.

The crude bromide **149** (0.20 g, 0.56 mmol), boronate ester **123** (0.25 g, 0.66 mmol), and K₂CO₃ (0.14 g, 1.0 mmol) were combined with a 1:1:1 mixture of toluene, ethanol and water (2 mL each). The slurry was degassed by alternately applying high vacuum to the reaction mixture and flushing with dry argon. The reaction vessel was then sealed and heated in an 80 °C oil bath for 14 h. The reaction mixture was cooled to room temperature, diluted with 100 mL 9:1 CH₂Cl₂/MeOH, and washed with water and brine (50 mL each). The aqueous washes were back-extracted once with 50 mL 9:1 CH₂Cl₂/MeOH. The combined organic extracts were dried over K₂CO₃, filtered, and concentrated *in vacuo* to afford 0.48 g of a brown solid. The solid was purified by flash column chromatography (7:3 acetone/hexane) to yield compound **73** as an off-white solid (0.17 g, 0.32 mmol). LCMS (ESI) *m*/*z* 525 (M + H)⁺.

### Synthesis of Compound 74

Compound **74** was synthesized according to the procedure described above for compound **73,** using thiourea **148** in place of **147.** The coupling reaction of bromide **150** and boronate **123** yielded compound **74** as a white solid (0.12 g, 0.21 mmol). LCMS (ESI) *m*/*z* 561 (M + H)⁺.

### Example 13 - Synthesis of Compound 75

Scheme 17 depicts the synthesis of compound **75.** D-*p*-Hydroxyphenyl-glycine **151** was converted to triflate **154,** which was subsequently coupled to boronate **123** to afford alcohol **155.** Mesylation of **155,** followed by displacement with the anion of imidazole and deprotection of the BOC group yielded compound **75.**

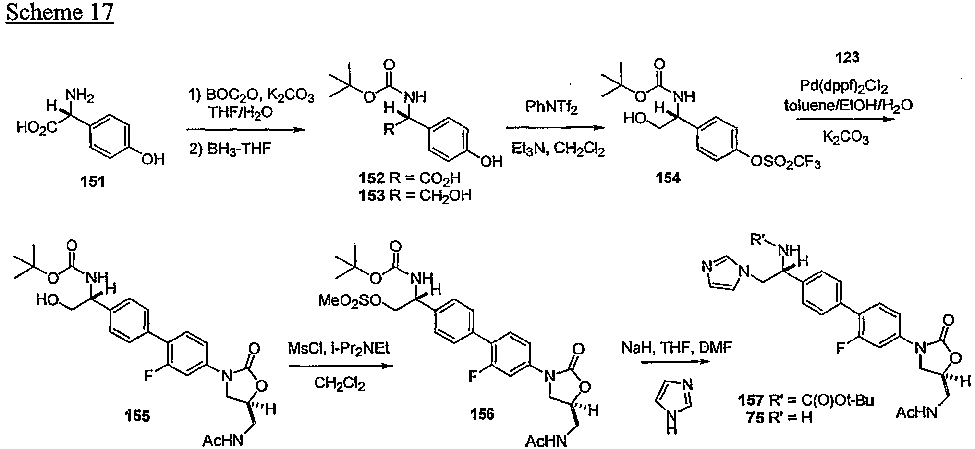

A solution of D-*p*-hydroxyphenylglycine **151** (23.8 g, 142.3 mmol) and potassium carbonate (39.3 g, 284.6 mmol) in THF (200 mL) and H₂O (200 mL) was treated with BOC₂O (34.14 g, 156.6 mmol) at 25 °C, and the resulting reaction mixture was stirred at 25 °C for 2 h. When TLC and LCMS showed that the reaction was complete, the reaction mixture was treated with ethyl acetate (200 mL) and H₂O (200 mL). The two layers were separated, and the aqueous solution was extracted with ethyl acetate (200 mL). The aqueous layer was then acidified with a 2N aqueous HCl to pH 4 before being extracted with ethyl acetate (2 x 200 mL). The combined organic extracts were then washed with water (2 x 100 mL) and saturated aqueous NaCl (100 mL), dried over MgSO₄, and concentrated *in vacuo.* The residual white solids were further dried *in vacuo* to afford the crude desired acid **152** (36.5 g; 96%), which was of suitable purity for use in subsequent reactions.

A solution of acid **152** (4.005 g, 15 mmol) in anhydrous THF (20 mL) was treated dropwise with a 1 M solution of BH₃-THF in THF (30 mL, 30 mmol) at 0-5 °C, and the resulting reaction mixture was stirred at 0- 5 °C for an additional 2 h. When TLC and LCMS showed that the reduction reaction was complete, the reaction mixture was treated with water (50 mL) and ethyl acetate (50 mL). The mixture was then stirred at 25 °C for 30 min before being separated, and the aqueous layer was extracted with ethyl acetate (2 x 50 mL). The combined organic extracts were then washed with water (2 x 20 mL) and saturated aqueous NaCl (20 mL), dried over MgSO₄, and concentrated *in vacuo.* The residue was then directly purified by flash column chromatography (0-5% MeOH-CH₂Cl₂ gradient elution) to afford desired alcohol **153** (2.50 g; 66%) as a white powder which was of suitable purity for use in subsequent reactions.

A suspension alcohol **153** (670 mg, 2.65 mmol) in CH₂Cl₂ (10 mL) was treated with N-phenyltrifluoromethane sulfonamide (947 mg, 2.65 mmol) and triethylamine (535.3 mg, 0.74 mL, 5.3 mmol) at 25 °C, and the resulting reaction mixture was stirred at 25 °C for an additional 2 h. When TLC and LCMS showed that the reaction was complete, the reaction mixture was quenched with water (10 mL) and CH₂Cl₂ (20 mL). The two layers were then separated, and the aqueous layer was extracted with CH₂Cl₂ (2 x 20 mL). The combined organic extracts were then washed with water (2 x 10 mL) and saturated aqueous NaCl (10 mL), dried over MgSO₄, and concentrated *in vacuo.* The residue was then directly purified by flash column chromatography (0-5% MeOH-CH₂Cl₂ gradient elution) to afford triflate **154** (945 mg; 93%) as a white powder which was of suitable purity for use in subsequent reactions.

A solution of boronate **123** (2.162 g, 5.72 mmol) and triflate **154** (1.70 g, 4.4 mmol) in toluene (24 mL) was treated with solid potassium carbonate (1.82 g, 13.2 mmol), ethanol (8.0 mL) and H₂O (8.0 mL) at room temperature, and the resulting reaction mixture was degassed three times under a steady stream of argon before being treated with Pd(dppf)₂Cl₂ (184 mg, 0.22 mmol) at room temperature. The reaction mixture was then degassed three times again under a steady stream of argon before being warmed to reflux for 2 h. When TLC and LCMS showed that the reaction was complete, the reaction mixture was cooled to room temperature before being treated with water (20 mL) and ethyl acetate (20 mL). The two layers were separated, and the aqueous layer was extracted with ethyl acetate (2 x 20 mL). The combined organic extracts were washed with water (2 x 20 mL) and saturated aqueous NaCl (20 mL), dried over MgSO₄, and concentrated *in vacuo.* The residue was then purified by flash column chromatography (0-5% MeOH-CH₂Cl₂ gradient elution) to afford (1-{4'-[5-(acetylamino-methyl)-2-oxo-oxazolidin-3-yl]-2'-fluoro-biphenyl-4-yl}-2-hydroxyethyl)carbamic acid *tert*-butyl ester **155** (1.543 g; 72%) as yellow oil, which solidified upon standing at room temperature *in vacuo.*

A suspension of alcohol **155** (694 mg, 1.43 mmol) in anhydrous CH₂Cl₂ (10 mL) was treated with diisopropylethylamine (388 mg, 0.522 mL, 2.85 mmol) and methanesulfonyl chloride (196 mg, 0.132 mL, 1.71 mmol) at 0-5 °C, and the resulting reaction mixture was stirred at 0-5 °C for an additional 2 h. When TLC and LCMS showed that the reaction was complete, the reaction mixture was quenched with water (10 mL). The two layers were separated, and the aqueous layer was extracted with CH₂Cl₂ (2 x 10 mL). The combined organic extracts were washed with water (2 x 10 mL) and saturated aqueous NaCl (10 mL), dried over MgSO₄, and concentrated *in vacuo.* The residue was then purified by flash column chromatography (0-5% MeOH-CH₂Cl₂ gradient elution) to afford mesylate **156** (647 mg; 80%) as a pale-yellow solid, which was of suitable purity for use in subsequent reactions.

A solution of imidazole (41 mg, 0.6 mmol) in anhydrous THF (3 mL) was treated with sodium hydride (NaH, 60% oil dispersion, 29 mg, 0.72 mmol) at 0 °C, and the resulting mixture was stirred at 0-5 °C for 30 min before a solution of mesylate **156** (170 mg, 0.3 mmol) in anhydrous DMF (3.0 mL) was added. The resulting reaction mixture was then stirred at 0-5 °C for 30 min before being gradually warmed to room temperature for 12 h. When TLC and LCMS showed that the reaction was complete, the solvents were removed *in vacuo,* and the residue was directly purified by flash column chromatography (0-5% MeOH-CH₂Cl₂ gradient elution) to afford imidazole **157** (46 mg; 29%) as a yellow solid.

A solution of imidazole **157** (23 mg, 0.043 mmol) in MeOH (1.0 mL) was treated with 4N HCl in 1,4-dioxane (3.0 mL), and the resulting reaction mixture was stirred at room temperature for 30 min. When TLC and LCMS showed that the reaction was complete, the solvents were removed *in vacuo,* and the desired *N-*{3-[4'-(1-amino-2-imidazol-1-yl-ethyl)-2-fluoro-biphenyl-4-yl]-2-oxo-oxazolidin-5-ylmethyl}acetamide hydrochloride **75** (18.8 mg; 100%) was obtained as a yellow solid. LCMS (ESI) *m*/*z* 438 (M + H)⁺.

### Example 14 - Synthesis of Compound 76

Scheme 18 depicts the synthesis of compound **76.** Iodide **158** was converted to boronate **159**, which was coupled to bromide **160** to afford tetrazole **161.** Deprotection of **161** afforded compound **76**.

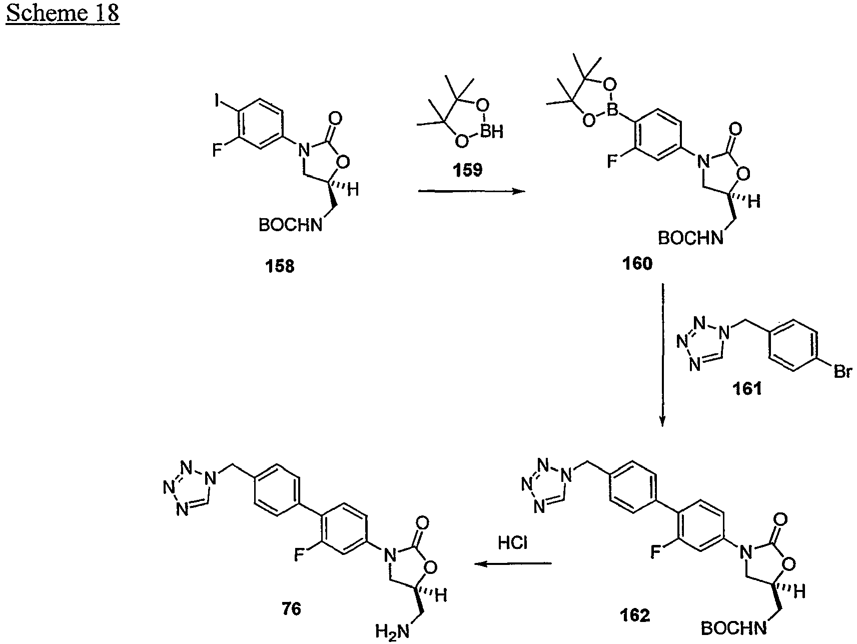

### Synthesis of boronate 160

A solution of known 5-aminomethyl-3-(3-fluoro-4-iodo-phenyl)-oxazolidin-2-one (2.02 g, 6.0 mmol; *see* U.S. Patent Nos. 5,523,403 and 5,565,571) and potassium carbonate (1.66 g, 12.0 mmol) in THF (20 mL) and H₂O (20 mL) was treated with BOC₂O (1.334 g, 6.12 mmol) at 25 °C, and the resulting reaction mixture was stirred at 25 °C for 2 h. When TLC and LCMS showed the reaction was complete, the reaction mixture was treated with ethyl acetate (20 mL) and H₂O (20 mL). The two layers were separated, and the aqueous solution was extracted with ethyl acetate (20 mL), and the combined organic extracts were then washed with water (2 x 10 mL) and saturated aqueous NaCl (10 mL), dried over MgSO₄, and concentrated *in vacuo.* The residual white solids were further dried *in vacuo* to afford the crude, desired iodide **158** (2.40 g; 92%), which was of suitable purity for use in subsequent reactions.

A solution of iodide **158** (1.11 g, 2.55 mmol) in 1,4-dioxane (25 mL) was treated with 4,4,5,5-tetramethyl-[1,3,2]dioxaborolane **159** (489 mg, 0.56 mL, 3.82 mmol) and triethylamine (772 mg, 1.07 mL, 7.65 mmol) at room temperature, and the resulting reaction mixture was degassed three times under a steady stream of argon before being treated with Pd(dppf)₂Cl₂ (107 mg, 0.13 mmol) at room temperature. The reaction mixture was then degassed three times again under a steady stream of argon before being warmed to reflux for 6 h. When TLC and LCMS showed that the reaction was complete, the reaction mixture was cooled to room temperature before being treated with water (20 mL) and ethyl acetate (20 mL). The two layers were separated, and the aqueous layer was extracted with ethyl acetate (2 x 20 mL). The combined organic extracts were washed with water (2 x 20 mL) and saturated aqueous NaCl (20 mL), dried over MgSO₄, and concentrated *in vacuo.* The residual brown oil was then purified by flash column chromatography (10-30% EtOAc-hexanes gradient elution) to afford boronate **160** (646 mg; 58%) as a brown oil that solidified upon standing at room temperature *in vacuo.* The product was of suitable purity for use in subsequent reactions.

### Synthesis of bromide 161

A solution of 4-bromobenzylamine hydrochloride (2.22 g, 10.0 mmol) in acetic acid (30 mL) was treated with triethyl orthoformate (2.964 g, 3.29 mL, 20.0 mmol) and sodium azide (NaN₃, 2.30 g, 20.0 mmol) at room temperature, and the resulting reaction mixture was subsequently stirred at reflux for 12 h. When TLC and LCMS showed that the reaction was complete, the reaction mixture was cooled to room temperature, and the cooled reaction mixture was poured into ice water (100 mL). The precipitate was then collected by filtration, washed with water (2 x 20 mL), and dried *in vacuo* to afford bromide **161** (460 mg; 19%) as a white solid which was of suitable purity for use in subsequent reactions.

### Synthesis of compound 76

A solution of boronate **160** (658 mg, 1.5 mmol) and bromide **161** (300 mg, 1.25 mmol) in toluene (9.0 mL) was treated with solid potassium carbonate (621 mg, 4.5 mmol), ethanol (3.0 mL) and H₂O (3.0 mL) at room temperature, and the resulting reaction mixture was degassed three times under a steady stream of argon before being treated with Pd(dppf)₂Cl₂ (52.3 mg, 0.063 mmol) at room temperature. The reaction mixture was then degassed three times again under a steady stream of argon before being warmed to reflux for 3 h. When TLC and LCMS showed that the reaction was complete, the reaction mixture was cooled to room temperature before being treated with water (10 mL) and ethyl acetate (20 mL). The two layers were separated, and the aqueous layer was extracted with ethyl acetate (2 x 10 mL). The combined organic extracts were washed with water (2 x 5 mL) and saturated aqueous NaCl (5 mL), dried over MgSO₄, and concentrated *in vacuo.* The residue was then purified by flash column chromatography (0-5% MeOH-CH₂Cl₂ gradient elution) to afford tetrazole **162**(357 mg; 61%) as a yellow oil, which solidified upon standing at room temperature *in vacuo.*

A solution of tetrazole **162** (350 mg, 0.748 mmol) in EtOAc (5.0 mL) was treated with 4N HCl in 1,4-dioxane (5.0 mL), and the resulting reaction mixture was stirred at room temperature for 30 min. When TLC and LCMS showed that the reaction was complete, the solvents were removed *in vacuo,* and the residue was treated with aqueous sodium bicarbonate (10 mL) and EtOAc (15 mL). The mixture was stirred at room temperature for 30 min before the two layers were separated. The aqueous layer was extracted with EtOAc (10 mL), and the combined organic extracts were washed with H₂O (10 mL) and saturated aqueous NaCl (10 mL), dried over MgSO₄, and *concentrated in vacuo* to afford compound **76** (266 mg; 97%) as a pale-yellow solid. LCMS (ESI) *mlz* 369 (M+H)⁺.

### Example 15 - Synthesis of Compounds 77-79

Scheme 19 depicts the synthesis of aryl bromides **163-165** required for the synthesis of compounds **77-79.** Epoxide **139** was treated with 1-formyl piperazine to afford a mixture of bromides **163** and **164**. Epoxide ring-opening of **139** with imidazole afforded bromide **165**. These bromides were coupled with boronate **123** to afford the target compounds **77-79**.

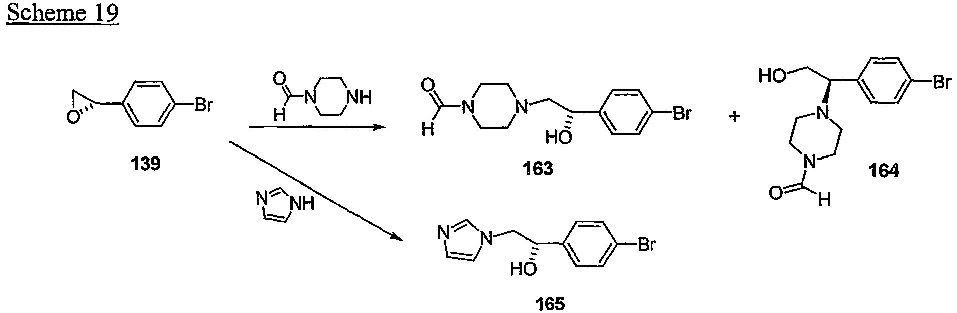

### Synthesis of bromides 163 and 164

To a suspension of epoxide **139** (1mmol, 1eq) in acetonitrile (3.0 mL) at room temperature was added LiClO₄ (1.05 mmol, 1.05 eq). After the formation of a clear solution, 1-formyl piperazine (1.5 mmol, 1.5 eq) was added. The mixture was stirred at room temperature for 16 h. The solvent was removed *in vacuo* and the residue was purified by flash chromatography (3:100 MeOH/CH₂Cl₂) to yield 132 mg of bromide **163** and 42 mg of bromide **164**.

### Synthesis of compound 77

A suspension of bromide **163** (1 eq), boronate **123** (1 eq), PdCl₂(dppf)₂ (0.05 eq), and K₂CO₃ (4 eq) in a 3:1:1mixture of dioxane/EtOH/H₂O was degassed by a stream of argon. The mixture was stirred at 80 °C for 3.5 h. The solvent was *removed in vacuo* and the residue was purified by flash chromatography (4:100 MeOH/CH₂Cl₂) to afford 150 mg compound **77**. LCMS (ESI) *m*/*z* 485 (M + H)⁺.

### Synthesis of compound 78

A suspension of bromide **164** (1 eq), boronate **123** (1 eq), PdCl₂(dppf)₂ (0.05 eq), and K₂CO₃ (4 eq) in a 3:1:1 mixture of dioxane/EtOH/H₂O was degassed by a stream of argon. The mixture was stirred at 80 °C for 3.5 h. The solvent was removed *in vacuo* and the residue was purified by flash chromatography (5:100 MeOH/CH₂Cl₂) to afford 52 mg compound **78**. LCMS (ESI) *mlz* 485 (M + H)⁺.

### Synthesis of bromide 165

To a suspension of epoxide **139** (1mmol, 1eq) in acetonitrile (3.0 mL) at room temperature was added LiClO₄(1.05 mmol, 1.05 eq). After the formation of a clear solution, imidazole (1.5 mmol, 1.5 eq) was added. The mixture was stirred at 60°C for 4 h. The solvent was removed *in vacuo* and the residue was purified by flash chromatography (3:100 MeOH/CH₂Cl₂) to yield 103 mg of romide **165**.

### Synthesis of compound 79

A suspension of bromide **165** (1 eq), boronate **123** (1 eq), PdCl₂(dppf)₂ (0.05 eq), and K₂CO₃ (4 eq) in a 3:1:1mixtures of dioxane/EtOH/H₂O was degassed by a stream of argon. The mixture was stirred at 80 °C for 2.5 h. The solvent was *removed in vacuo* and the residue was purified by flash chromatography (10:100 MeOH/CH₂Cl₂) to afford 155 mg compound **79**. LCMS (ESI) *m*/*z* 439 (M + H)⁺.

## Claims

1. A process for preparing a compound having the formula: 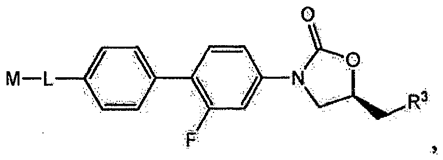 the process comprising the steps of:
combining a compound (I) of formula: 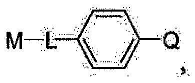
with a compound (II) of the formula: 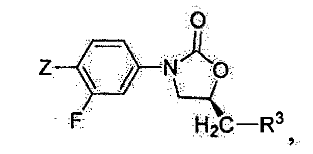 in a solvent comprising a mixture of water, toluene, and ethanol,
in the presence of a potassium carbonate and a palladium catalyst,
wherein the palladium catalyst is tetrakis(triphenylphosphine) palladium (0) and the ratio of the equivalents of tetrakis(triphenylphosphine) palladium (0) to the equivalents of compound (I) is 1:20;
further wherein,
R³ is -NHC(O)R₄ and R⁴ is -CH₃;
M-L is M-CH₂-X-CH₂, wherein X is NR⁴ and R⁴ is H or an amine protecting group selected from the group consisting of:
a) benzyl, b) *t*-butyldimethylsilyl, c) *t*-butdyldiphenylsilyl, d) *t*-butyloxycarbonyl, e) *p*-methoxybenzyl, f) methoxymethyl, g) tosyl, h) trifluoroacetyl; i) trimethylsilyl, j) fluorenyl-methyloxycarbonyl, k) 2-trimethylsilyl-ethyoxycarbonyl, 1) 1-methyl-1-(4-biphenylyl)ethoxycarbonyl, m) allyloxycarbonyl, and n) benzyloxycarbonyl;
M is triazole, tetrazole, oxazole, isoxazole;
Z is I; and
Q is -B(OH)₂.

2. The process according to claim 1, wherein X is -NR⁴- and R⁴ is H.

3. The process according to claim 1, wherein X is -NR⁴ and R⁴ is an amine protecting group.

4. The process according to claim 3, further comprising the step of removing the amine protecting group.

5. The process according to claim 1, wherein M is isoxazol-4-yl.

6. The process according to claim 1, wherein M is [1,2,3]triazol-1-yl.

7. The process according to claim 1, wherein M is [1,2,3]triazol-4-yl.

8. The process according to any one of claims 1-7, wherein the ratio of equivalents of base to equivalents of compound (I) is 3:1.

9. The process according to any one of claims 1-8, wherein the mixture of water, toluene, and ethanol is in a ratio of 1:3:1 by volume.

10. The process according to any one of claims 1-9, wherein the process is carried out at the reflux temperature of the solvent.

## Patentansprüche

1. Verfahren zur Herstellung einer Verbindung der Formel: 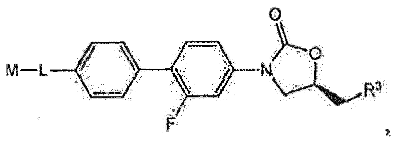 wobei das Verfahren die Schritte umfasst:
Zusammenbringen einer Verbindung (I) der Formel: 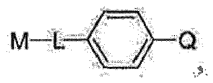
mit einer Verbindung (II) der Formel: 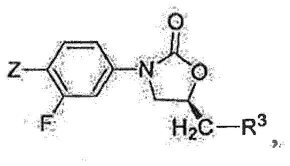 in einem Lösemittel, das eine Mischung aus Wasser, Toluol und Ethanol umfasst,
in Gegenwart eines Kaliumcarbonats und eines Palladiumkatalysators,
wobei der Palladiumkatalysator Terakis(triphenylphosphin)palladium(0) ist und das Verhältnis der Äquivalente von Terakis(triphenylphosphin)palladium(0) zu den Äquivalenten von Verbindung (I) 1:20 ist;
wobei weiterhin
R³ -NHC(O)R₄ ist und R⁴ -CH₃ ist;
M-L M-CH₂-X-CH₂ ist, wobei X NR⁴ ist und R⁴ H oder eine Aminschutzgruppe ist, die ausgewählt ist aus der Gruppe bestehend aus:
a) Benzyl, b) t-Butyldimethylsilyl, c) t-Butyldiphenylsilyl, d) t-Butyloxycarbonyl, e) *p*-Methoxybenzyl, f) Methoxymethyl, g) Tosyl, h) Trifluoracetyl, i) Trimethylsilyl, j) Fluorenylmethyloxycarbonyl, k) 2-Trimethylsilylethoxycarbonyl, 1) 1-Methyl-1-(4-biphenylyl)ethoxycarbonyl, m) Allyloxycarbonyl und n) Benzyloxycarbonyl;
M Triazol, Tetrazol, Oxazol, Isoxazol ist;
Z I ist; und
Q -B(OH)₂ ist.

2. Verfahren nach Anspruch 1, wobei X -NR⁴ ist und R⁴ H ist.

3. Verfahren nach Anspruch 1, wobei X -NR⁴ ist und R⁴ eine Aminschutzgruppe ist.

4. Verfahren nach Anspruch 3, weiterhin umfassend den Schritt des Abspaltens der Aminschutzgruppe.

5. Verfahren nach Anspruch 1, wobei M Isoxazol-4-yl ist.

6. Verfahren nach Anspruch 1, wobei M [1,2,3]Triazol-1-yl ist.

7. Verfahren nach Anspruch 1, wobei M [1,2,3]Triazol-4-yl ist.

8. Verfahren nach einem der Ansprüche 1-7, wobei das Verhältnis von Baseäquivalenten zu Äquivalenten von Verbindung (I) 3:1 ist.

9. Verfahren nach einem der Ansprüche 1-8, wobei die Mischung aus Wasser, Toluol und Ethanol in einem Volumenverhältnis von 1:3:1 vorliegt.

10. Verfahren nach einem der Ansprüche 1-9, wobei das Verfahren bei der Rückflusstemperatur des Lösemittels durchgeführt wird.

## Revendications

1. Procédé de préparation d'un composé de formule : 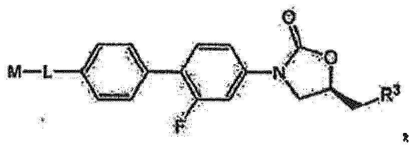 le procédé comprenant les étapes :
de combinaison d'un composé (I) de formule : 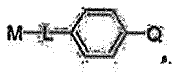
avec un composé (II) de formule : 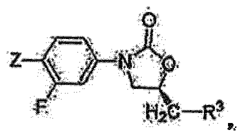 dans un solvant comprenant un mélange d'eau, de toluène, et d'éthanol,
en présence d'un carbonate de potassium et d'un catalyseur au palladium,
dans lequel le catalyseur au palladium est le tétrakis(triphénylphosphine) palladium (0) et le rapport des équivalents du tétrakis(triphénylphosphine) palladium (0) sur les équivalents du composé (I) est de 1/20 ;
dans lequel en outre
R³ est -NHC (O) R₄ et R⁴ est -CH₃ ;
M-L est M-CH₂-X-CH₂, dans lequel X est -NR⁴ et R⁴ est H ou un groupe de protection amine sélectionné dans le groupe constitué des groupes :
a) benzyle, b) t-butyldiméthylsilyle, c) *t-*butyldiphénylsilyle, d) *t*-butyloxycarbonyle, e) *p-*méthoxybenzyle, f) méthoxyméthyle, g) tosyle, h) trifluoroacétyle, i) triméthylsilyle, j) fluorényl-méthyloxycarbonyle, k) 2-triméthylsilyl-éthyloxycarbonyle, 1) 1-méthyl-1-(4-biphénylyl)éthoxycarbonyle, m) allyloxycarbonyle, et n) benzyloxycarbonyle ;
M est un groupe triazole, tétrazole, oxazole, isoxazole ;
Z est I ; et
Q est -B(OH)₂.

2. Procédé selon la revendication 1, dans lequel X est -NR⁴ et R⁴ est H.

3. Procédé selon la revendication 1, dans lequel X est -NR⁴ et R⁴ est un groupe de protection amine.

4. Procédé selon la revendication 3, comprenant en outre l'étape d'élimination du groupe de protection amine.

5. Procédé selon la revendication 1, dans lequel M est un groupe isoxazol-4-yl.

6. Procédé selon la revendication 1, dans lequel M est un groupe [1,2,3]triazol-1-yl.

7. Procédé selon la revendication 1, dans lequel M est un groupe [1,2,3]triazol-4-yl.

8. Procédé selon l'une quelconque des revendications 1 à 7, dans lequel le rapport des équivalents de la base sur les équivalents du composé (I) est de 3/1.

9. Procédé selon l'une quelconque des revendications 1 à 8, dans lequel le mélange d'eau, de toluène et d'éthanol est en un rapport de 1/3/1 en volume.

10. Procédé selon l'une quelconque des revendications 1 à 9, dans lequel le procédé est réalisé à la température de reflux du solvant.
